# EUROPEAN PATENT APPLICATION

(11) **EP 4 368 621 A1**
(43) Date of publication of application: **15.05.2024**
(21) Application number: 22836824.7
(22) Date of filing: 01.07.2022
(51) Int. Cl.: C07D 471/14, C07D 471/04, C07D 519/00, C07F 5/02

(54) **METHOD FOR PREPARING NITROGEN-CONTAINING POLYCYCLIC FUSED RING COMPOUND, AND INTERMEDIATE AND USE OF COMPOUND**

(30) Priority: 09.07.2021 CN 202110780873
(71) Applicant: Applied Pharmaceutical Science, Inc., Beijing 100176 (CN)
(72) Inventor: ZHONG, Jun, Beijing 100176 (CN); GAO, Qin, Beijing 100176 (CN); CHEN, Minchun, Beijing 100176 (CN); LIU, Yongbo, Beijing 100176 (CN); WANG, Hao, Beijing 100176 (CN)
(74) Representative: Gille Hrabal Partnerschaftsgesellschaft mbB Patentanwälte
(86) International application number: PCT/CN2022/103336
(87) International publication number: WO 2023/280073

(57) **Abstract**

A method for preparing a nitrogen-containing polycyclic fused ring compound, and an intermediate of the compound. By means of the preparation method, a compound of formula I and an intermediate thereof can be synthesized in a high-yield, low-cost and environmentally friendly manner, such that the compound of formula I can be produced on an industrial scale. In the preparation method, column chromatography separation and purification steps such as silica gel column chromatography are not used, and hydrogen is also not used as a hydrogenation or reduction agent, thereby significantly improving the production efficiency and safety. In addition, the preparation method achieves highly efficient utilization of position isomers IIIA and IIIB, and prevents the yield of a target product from being adversely influenced by discarding position isomer IIIA. The total yield of the method, in which the position isomer IIIA is used, can reach 9.4%, and is significantly higher than a yield of less than 4%, which is generated where the position isomer IIIB is used. Where the two position isomers are applied to the preparation of compound I at the same time, the yield is increased by 3 times relative to that generated where position isomer IIIB is used alone.

## Description

The present disclosure claims priority to the prior application with the patent application No. 202110780873.9 and entitled "METHOD FOR PREPARING NITROGEN-CONTAINING POLYCYCLIC FUSED RING COMPOUND, AND INTERMEDIATE AND USE OF COMPOUND" filed to China National Intellectual Property Administration on July 9, 2021, which is incorporated herein by reference in its entirety.

### TECHNICAL FIELD

The present disclosure relates to the field of preparation methods for pharmaceutical compounds, and particularly to a preparation method for a nitrogen-containing polycyclic fused ring compound, and an intermediate and use of the compound.

### BACKGROUND

RET (rearranged during transfection) proto-oncogene was first demonstrated in 1985 by transfection of NIH3T3 (mouse embryonic fibroblast cell line) cells with human lymphoma DNA (Cell, 1985, 42(2): 581-588). RET proto-oncogene, located on chromosome 10q11.2, has 60 kb of DNA, contains 21 exons, and encodes an RET protein consisting of 1100 amino acids. The RET protein is a tyrosine kinase receptor containing an extracellular domain composed of cysteine, a transmembrane domain, and an intracellular domain capable of catalyzing tyrosine kinase (Mol Cell Endocrinol, 2010, 322(1-2): 2-7). RET is involved in cell proliferation, nerve conduction, cell migration and cell differentiation, and activates various downstream pathways, such as RAS/RAF/MEK/ERK, PI3K/AKT and STAT through the signal from ligand/receptor complex/RET multiprotein complex pathway to induce cell proliferation (J Clin Oncol, 2012, 30(2): 200-202).

In many human cancers such as thyroid cancer, the RET kinase signal transduction plays an important role. The mutation in gatekeeper residue RET 804V of RET is an important cause of tumor resistance to currently approved non-selective RET inhibitors (such as cabozantinib and vandetanib). One of the important mutations in the extracellular or intracellular domain of RET in isolated familial medullary thyroid carcinoma, i.e., the mutation in gatekeeper residue V804M in the kinase ATP binding site, leads to a decrease in the affinity of existing drugs for the ATP binding site. It has been reported in the document (RET Solvent Front Mutations Mediate Acquired Resistance to Selective RET Inhibition in RET-Driven Malignancies, Journal of Thoracic Oncology, 2020, Vol. 15, No. 4, 541-549) that upon the use of the selective RET inhibitor LOXO-292 (selpercatinib), the aforementioned RET 804V mutation can be avoided, but there are still other mutations that lead to drug resistance. For example, mutations in residue G810 such as G810R, G810S, and G801C, which can result in a solvent-front of the kinase ATP binding site in non-small cell lung cancer, lead to a decrease in the binding of LOXO-292 to the ATP binding site, resulting in drug resistance and cancer progression.

Patent document WO2021023209A1 discloses compounds with excellent activity or selectivity as RET inhibitors, which have strong inhibitory effects on the RET gatekeeper residue mutant RET V804M, RET solvent-front residue mutant G810R and other clinically relevant RET mutants, as well as wt-RET. Such compounds can also significantly inhibit the growth of TT cell line derived from thyroid cancer and Ba/F3 cells transformed with various RET mutants, and have good inhibitory effects. In addition, such compounds can greatly block cellular RET autophosphorylation and downstream pathways, can significantly induce TT cell death, and have excellent pharmacokinetic properties. Therefore, there is a need to develop more efficient, more amenable to scale-up and/or more cost-effective methods for preparing such compounds, as well as intermediate compounds and preparation methods therefor that achieve the stated objectives.

### SUMMARY

To ameliorate the problems described above, the present disclosure provides a preparation method M1A for a compound of formula I, wherein the preparation method comprises reacting a compound of formula XX with R^{k}-C(O)-H to give the compound of formula I: 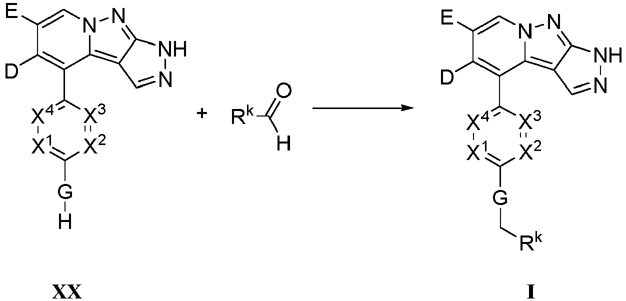 wherein,
X¹, X², X³, and X⁴ are identical or different, and are each independently selected from CR¹ or N;
each R¹ is identical or different, and is independently selected from H, halogen, CN, OH, and the following groups unsubstituted or optionally substituted with one, two or more R^{a}: C₁₋₄₀ alkyl, C₃₋₄₀ cycloalkyl, C₁₋₄₀ alkyloxy, C₃₋₄₀ cycloalkyloxy, NR²R³, -C(O)R⁴, -OCR⁵, -S(O)₂R⁶, and OS(O)₂R⁷;
D and E are identical or different, and are each independently selected from H, halogen, CN, OH, -O-R²¹, and the following groups unsubstituted or optionally substituted with one, two or more R^{c}: C₁₋₄₀ alkyl, C₃₋₄₀ cycloalkyl, C₆₋₂₀ aryl, 5- to 20-membered heteroaryl, 3- to 20-membered heterocyclyl, and NR²R³, provided that at least one of D and E is selected from -O-R²¹;
R²¹ is selected from the following groups unsubstituted or optionally substituted with one, two or more R^{d}: C₁₋₄₀ alkyl, C₃₋₄₀ cycloalkyl, C₆₋₂₀ aryl, 5- to 20-membered heteroaryl, and 3- to 20-membered heterocyclyl;
G is selected from the following groups unsubstituted or optionally substituted with one, two or more R^{e}: C₃₋₄₀ cycloalkyl, C₃₋₄₀ cycloalkenyl, C₆₋₂₀ aryl, 5- to 20-membered heteroaryl, 3- to 20-membered heterocyclyl, C₃₋₄₀ cycloalkyloxy, C₃₋₄₀ cycloalkenyloxy, C₆₋₂₀ aryloxy, 5- to 20-membered heteroaryloxy, and 3- to 20-membered heterocyclyloxy;
R^{k} is selected from the following groups unsubstituted or optionally substituted with one, two or more R^{g}: C₁₋₄₀ alkyl, C₃₋₄₀ cycloalkyl, C₃₋₄₀ cycloalkenyl, C₆₋₂₀ aryl, 5- to 20-membered heteroaryl, and 3- to 20-membered heterocyclyl;
each R² is identical or different, and is independently selected from H, C₁₋₄₀ alkyl, C₃₋₄₀ cycloalkyl, C₆₋₂₀ aryl, 5- to 20-membered heteroaryl, 3- to 20-membered heterocyclyl, -C(O)R⁴, and -S(O)₂R⁶;
each R³ is identical or different, and is independently selected from H, C₁₋₄₀ alkyl, C₃₋₄₀ cycloalkyl, C₆₋₂₀ aryl, 5- to 20-membered heteroaryl, 3- to 20-membered heterocyclyl, -C(O)R⁴, and -S(O)₂R⁶;
or, R² and R³, together with the N atom connected thereto, form 5- to 20-membered heteroaryl or 3- to 20-membered heterocyclyl;
each R⁴ is identical or different, and is independently selected from H, C₁₋₄₀ alkyl, C₃₋₄₀ cycloalkyl, C₆₋₂₀ aryl, 5- to 20-membered heteroaryl, 3- to 20-membered heterocyclyl, C₁₋₄₀ alkyloxy, C₃₋₄₀ cycloalkyloxy, C₆₋₂₀ aryloxy, 5- to 20-membered heteroaryloxy, 3- to 20-membered heterocyclyloxy, and NR²R³;
each R⁵ is identical or different, and is independently selected from H, C₁₋₄₀ alkyl, C₃₋₄₀ cycloalkyl, C₆₋₂₀ aryl, 5- to 20-membered heteroaryl, 3- to 20-membered heterocyclyl, C₁₋₄₀ alkylcarbonyl, C₃₋₄₀ cycloalkylcarbonyl, C₆₋₂₀ arylcarbonyl, 5- to 20-membered heteroarylcarbonyl, and 3- to 20-membered heterocyclylcarbonyl;
each R⁶ is identical or different, and is independently selected from H, C₁₋₄₀ alkyl, C₂₋₄₀ alkenyl, C₂₋₄₀ alkynyl, C₃₋₄₀ cycloalkyl, C₆₋₂₀ aryl, 5- to 20-membered heteroaryl, 3- to 20-membered heterocyclyl, C₁₋₄₀ alkyloxy, C₃₋₄₀ cycloalkyloxy, C₆₋₂₀ aryloxy, 5- to 20-membered heteroaryloxy, 3- to 20-membered heterocyclyloxy, and NR²R³;
each R⁷ is identical or different, and is independently selected from H, C₁₋₄₀ alkyl, C₃₋₄₀ cycloalkyl, C₆₋₂₀ aryl, 5- to 20-membered heteroaryl, and 3- to 20-membered heterocyclyl;
each R^{a}, R^{c}, R^{d} and R^{e} are identical or different, and are independently selected from halogen, CN, OH, SH, oxo (=O), NO₂, and the following groups unsubstituted or optionally substituted with one, two or more R^{g}: C₁₋₄₀ alkyl, C₂₋₄₀ alkenyl, C₂₋₄₀ alkynyl, C₃₋₄₀ cycloalkyl, C₆₋₂₀ aryl, 5-to 20-membered heteroaryl, 3- to 20-membered heterocyclyl, C₁₋₄₀ alkyloxy, C₃₋₄₀ cycloalkyloxy, C₆₋₂₀ aryloxy, 5- to 20-membered heteroaryloxy, and 3- to 20-membered heterocyclyloxy;
each R^{g} is identical or different, and is independently selected from halogen, CN, OH, SH, oxo (=O), NO₂, and the following groups unsubstituted or optionally substituted with one, two or more R^{h}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, C₁₋₄₀ alkyloxy, C₂₋₄₀ alkenyloxy, C₂₋₄₀ alkynyloxy, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, and 3- to 20-membered heterocyclyloxy;
each R^{h} is identical or different, and is independently selected from halogen, CN, OH, SH, oxo (=O), NO₂, and the following groups unsubstituted or optionally substituted with one, two or more R^{g}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, C₁₋₄₀ alkyloxy, C₂₋₄₀ alkenyloxy, C₂₋₄₀ alkynyloxy, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, and 3- to 20-membered heterocyclyloxy;
or, where the C₃₋₄₀ cycloalkyl, C₃₋₄₀ cycloalkenyl, C₃₋₄₀ cycloalkynyl or 3- to 20-membered heterocyclyl described above is substituted with two or more substituents at different positions, any two of the substituents may also, together with the atom connected thereto, form a bridged ring, wherein the bridge atoms other than the bridgehead atoms in the bridged ring may comprise 1, 2, 3, 4, or 5 divalent groups selected from CH₂, O, and NH;
or, where one atom (such as carbon atom) is substituted with two or more substituents, two of the substituents may also, together with the shared atom connected thereto, form a cyclic group such as C₃₋₄₀ cycloalkyl, C₃₋₄₀ cycloalkenyl, C₃₋₄₀ cycloalkynyl, 3- to 20-membered heterocyclyl, or the like.

According to an embodiment of the present disclosure, X¹, X², X³, and X⁴ are identical or different, and are each independently selected from CR¹ or N; for example, at least one of X¹, X², X³, and X⁴ is N, e.g., one, two or three of X¹, X², X³, and X⁴ are N.

According to an embodiment of the present disclosure, each R¹ is identical or different, and is independently selected from H, halogen, CN, OH, and the following groups unsubstituted or optionally substituted with one, two or more R^{a}: C₁₋₆ alkyl, C₃₋₁₀ cycloalkyl, C₁₋₆ alkyloxy, and C₃₋₁₀ cycloalkyloxy, for example, selected from the following group unsubstituted or optionally substituted with 1, 2 or 3 R^{a}: C₁₋₆ alkyl, C₃₋₆ cycloalkyl, C₁₋₆ alkoxy, or C₃₋₆ cycloalkyloxy. According to an embodiment of the present disclosure, D and E are identical or different, and are each independently selected from H, halogen, CN, NH₂, C₁₋₆ alkyl, or -O-R²¹, provided that at least one of D and E is selected from -O-R²¹.

According to an embodiment of the present disclosure, at least one of D and E is selected from the following group:

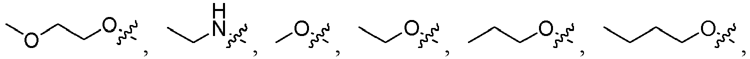

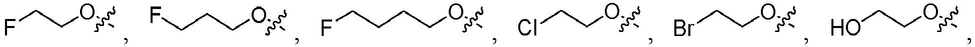

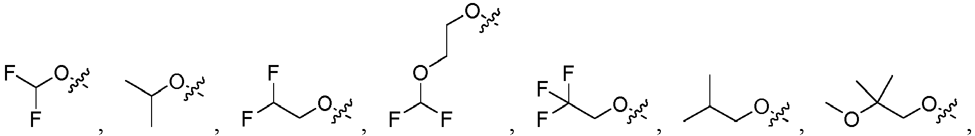

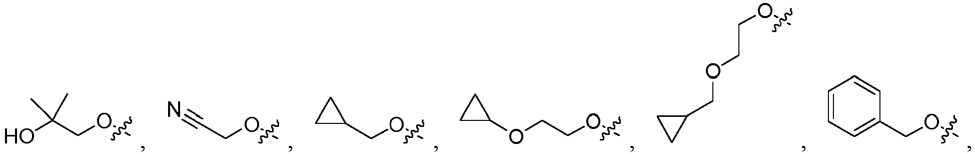

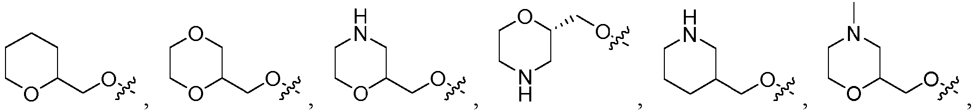

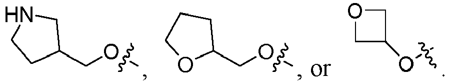

According to an embodiment of the present disclosure, R² is selected from C₁₋₆ alkyl unsubstituted or optionally substituted with one, two or more R^{d}.

According to an embodiment of the present disclosure, each R^{a}, R^{c} and R^{d} are identical or different, and are independently selected from halogen, CN, OH, and the following groups unsubstituted or optionally substituted with one, two or more R^{g}: C₁₋₆ alkyl, C₁₋₆ alkyloxy, C₃₋₁₀ cycloalkyl, and C₃₋₁₀ cycloalkyloxy.

According to an embodiment of the present disclosure, each R^{g} is identical or different, and is independently selected from halogen or C₃₋₁₀ cycloalkyl.

According to an embodiment of the present disclosure, G is selected from C₃₋₁₀ cycloalkyl, C₆₋₁₄ aryl, 5- to 14-membered heteroaryl, and 3- to 10-membered heterocyclyl, for example, 6- to 7-membered heterocyclyl having a monocyclic, bicyclic or bridged ring structure containing 1, 2 or 3 heteroatoms independently selected from N, O, and S.

According to an embodiment of the present disclosure, R^{k} is selected from the following groups unsubstituted or optionally substituted with one, two or more R^{g}: C₃₋₁₀ cycloalkyl, C₆₋₁₄ aryl, 5-to 14-membered heteroaryl, and 3- to 10-membered heterocyclyl, wherein the heteroaryl may be pyridinyl, for example, selected from pyridin-2-yl, pyridin-3-yl, pyridin-4-yl, pyridin-5-yl, and pyridin-6-yl; the aryl may be phenyl.

According to an exemplary embodiment of the present disclosure, X¹, X², X³, and X⁴ are identical or different, and are each independently selected from CH or N; for example, at least one of X¹, X², X³, and X⁴ is N, e.g., one, two or three of X¹, X², X³, and X⁴ are N.

According to an exemplary embodiment of the present disclosure, R¹ is H.

According to an exemplary embodiment of the present disclosure, E is H.

According to an exemplary embodiment of the present disclosure, D is selected from the following groups: halogen, BnO-, H, -CN, -NH₂, -OCH₃, and the following groups:

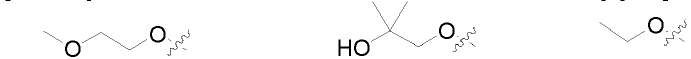

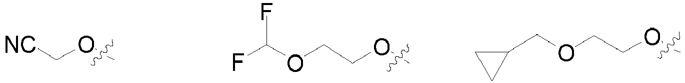

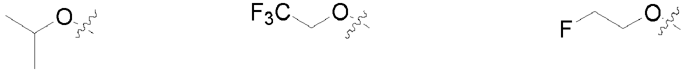

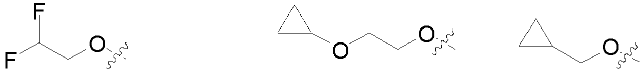

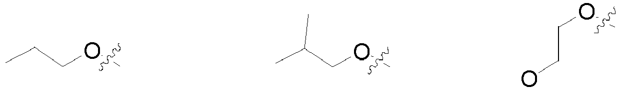

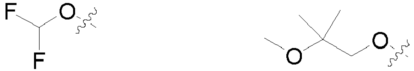

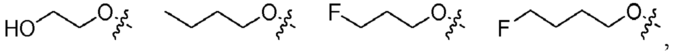

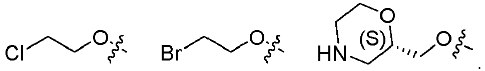

According to an exemplary embodiment of the present disclosure, G is selected from

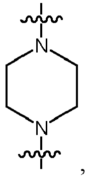

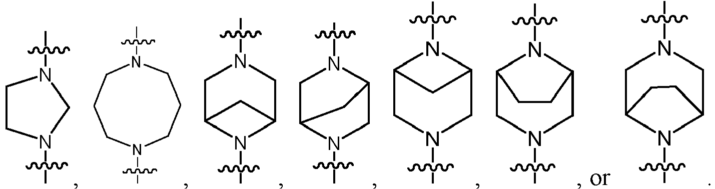

According to an exemplary embodiment of the present disclosure, R^{k} is selected from pyridinyl or phenyl unsubstituted or optionally substituted with one, two or more R^{g}, wherein where one, two or more R^{g} substituents are present, the R^{g} may be a substituent at position 1, 2, 3, 4, 5 or 6 of the pyridinyl or phenyl, as long as it does not affect the connection of R^{k} to group G via methylene.

As an example, R^{k} in the compound of formula I may, together with methylene, form a group selected from the following: 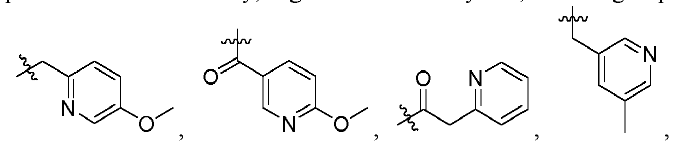 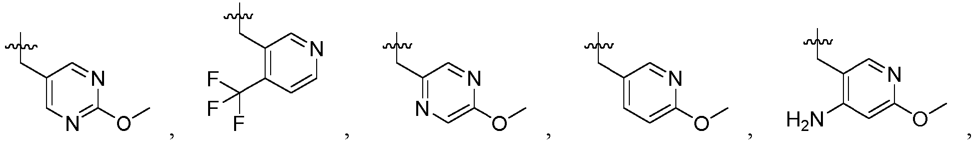 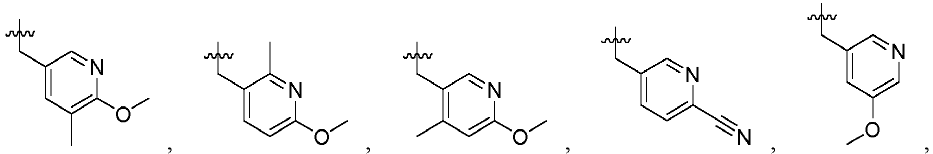 or

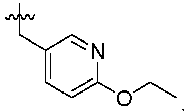

Alternatively, R^{k} in the compound of formula I may be selected from group 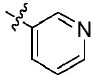 substituted with 1, 2, 3 or 4 groups selected from C₁₋₆ alkyl and C₁₋₆ alkyloxy.

According to an exemplary embodiment of the present disclosure, the compound of formula I may be selected from the following compounds:

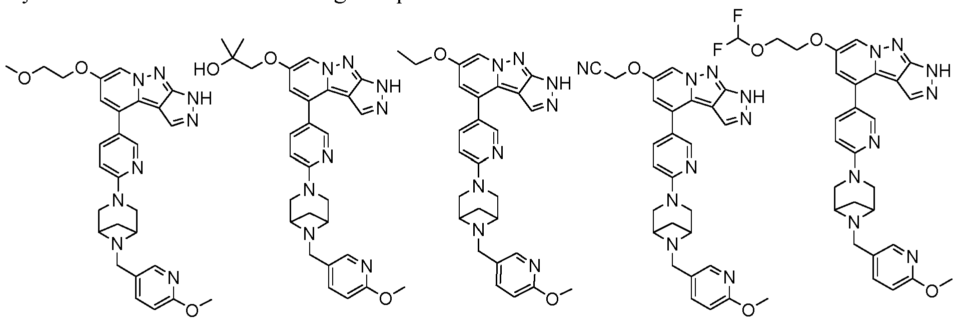

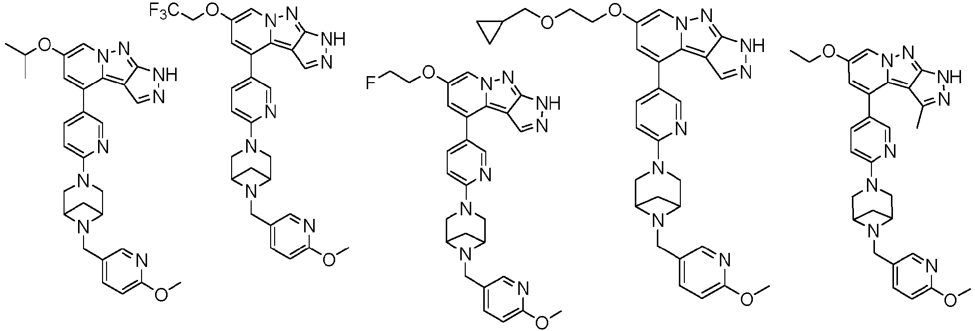

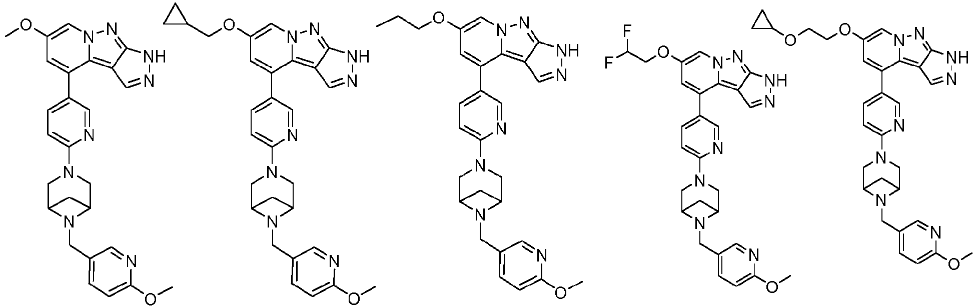

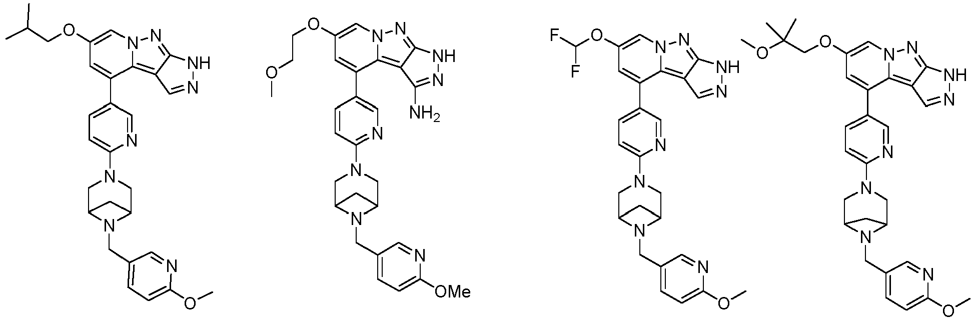

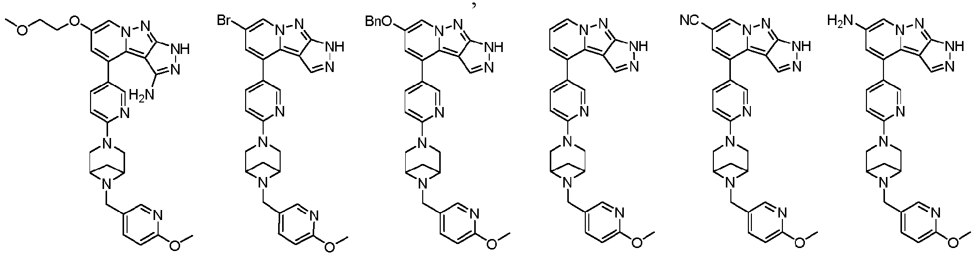

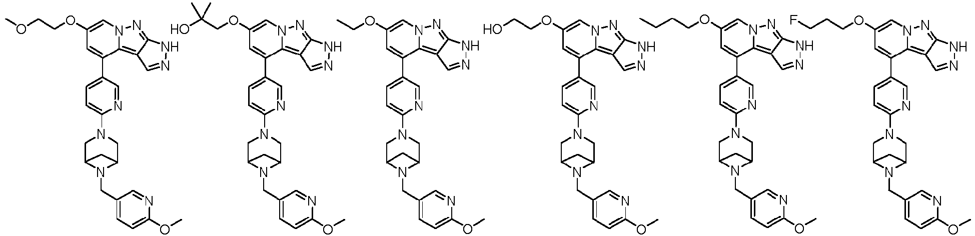

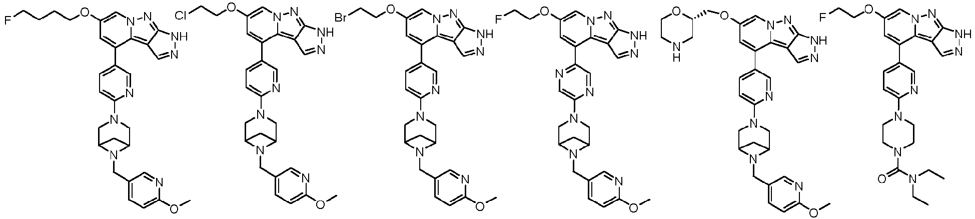

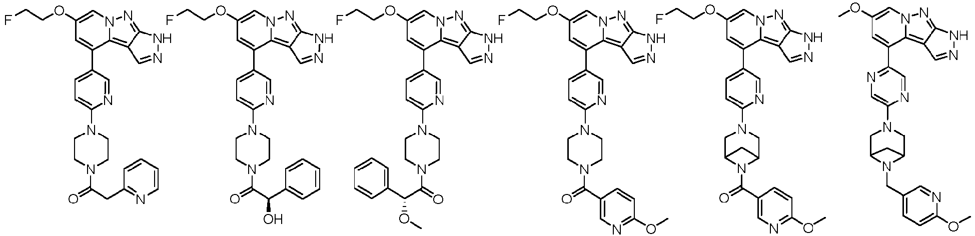

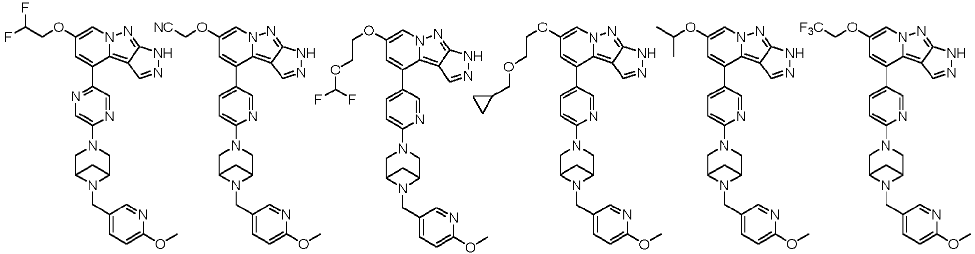

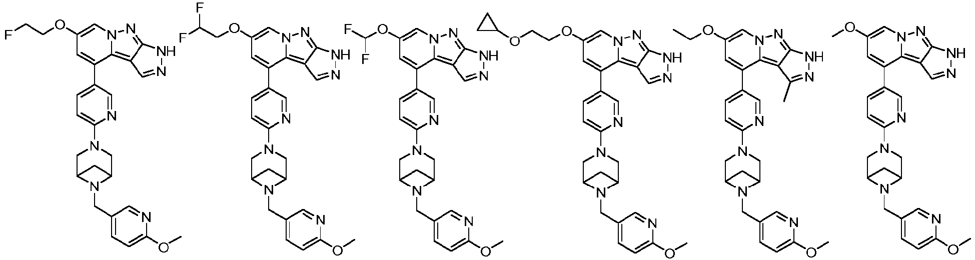

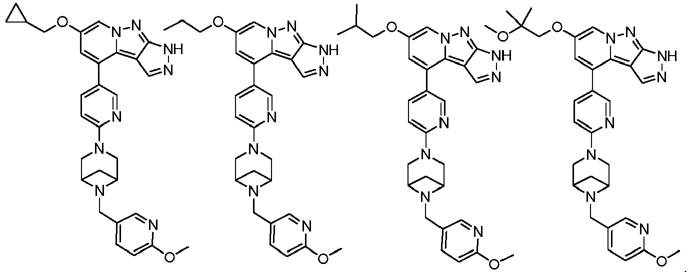

As an example, the compound of formula I is selected from the following compounds:

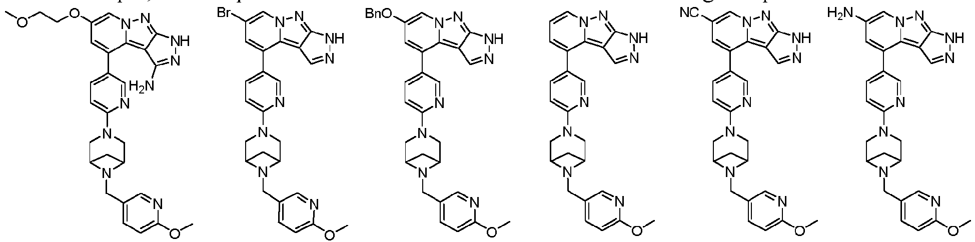

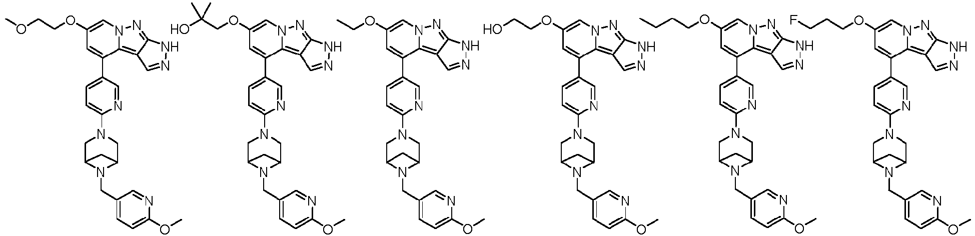

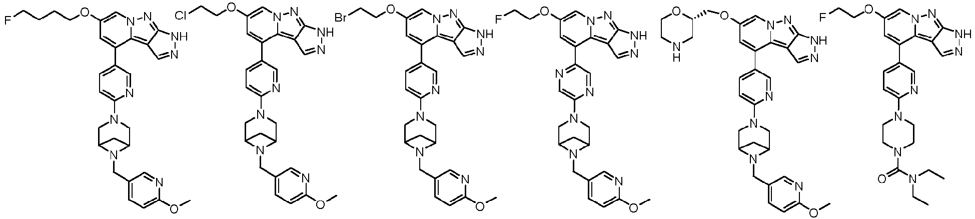

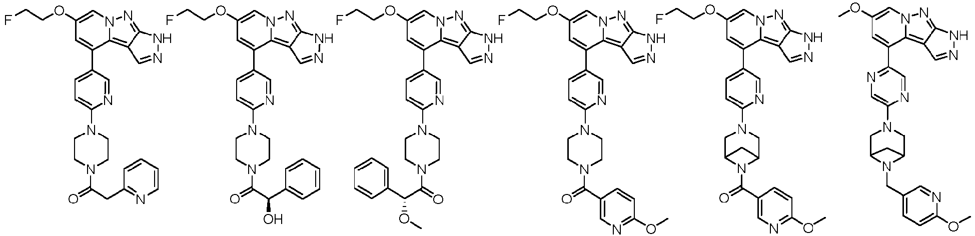

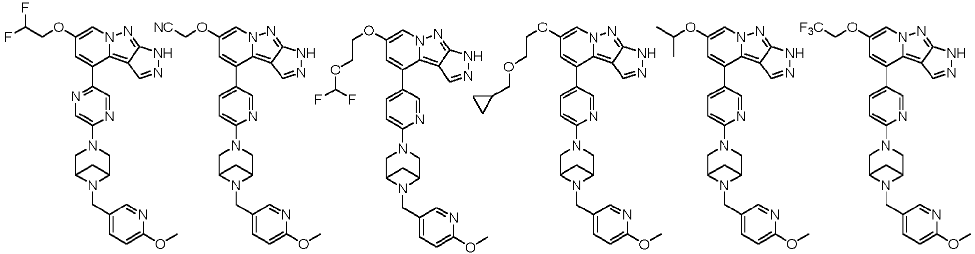

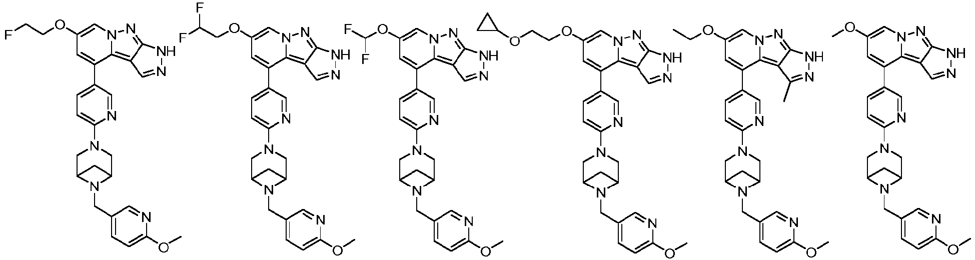

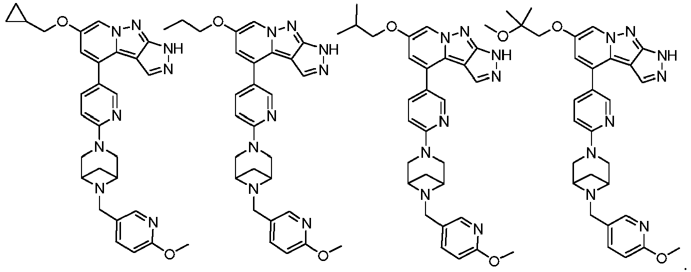

According to an embodiment of the present disclosure, the reaction of the compound of formula XX with R^{k}-C(O)-H may be carried out in the presence of borane, pyridine borane, 2-picoline borane (Pic-BH₃), sodium borohydride, sodium triacetoxyborohydride, or sodium cyanoborohydride, preferably in the presence of 2-picoline borane.

According to an embodiment of the present disclosure, the reaction of the compound of formula XX with R^{k}-C(O)-H may be carried out in the presence of an organic solvent. The organic solvent may be selected from one or a mixture of two or more of the following: ethers (e.g., ethyl propyl ether, methyl *tert*-butyl ether, *n*-butyl ether, anisole, phenetole, cyclohexyl methyl ether, dimethyl ether, diethyl ether, diphenyl ether, dipropyl ether, diisopropyl ether, di-*n*-butyl ether, diisobutyl ether, diisoamyl ether, ethylene glycol dimethyl ether, isopropyl ethyl ether, diethylene glycol dimethyl ether, triethylene glycol dimethyl ether, tetrahydrofuran, 2-methyltetrahydrofuran, 1,4-dioxane and ethylene oxide, and/or propylene oxide polyethers); sulfoxides (e.g., tetrahydrodioxythiophene and dimethyl sulfoxide, tetramethyl sulfoxide, dipropyl sulfoxide, benzyl methyl sulfoxide, diisobutyl sulfoxide, dibutyl sulfoxide, or diisoamyl sulfoxide); sulfones (e.g., dimethyl sulfone, diethyl sulfone, dipropyl sulfone, dibutyl sulfone, diphenyl sulfone, dihexyl sulfone, methyl ethyl sulfone, ethyl propyl sulfone, ethyl isobutyl sulfone, and cyclopentyl sulfone); aliphatic, cycloaliphatic or aromatic hydrocarbons (e.g., pentane, hexane, heptane, octane, nonane, cyclohexane, methylcyclohexane, petroleum ether, naphtha, octane, benzene, toluene, or xylene); haloalkanes (e.g., dichloromethane, chloroform, carbon tetrachloride, dichloroethane, or trichloroethane); halogenated aromatic compounds (e.g., chlorobenzene or dichlorobenzene); amides (e.g., hexamethylphosphoramide, formamide, *N,N-*dimethylacetamide, *N*-methylformamide, *N,N-*dimethylformamide, *N,N-*dipropylformamide, *N,N-*dibutylformamide, *N*-methylpyrrolidine, *N-methylcaprolactam,* 1,3-dimethyl-3,4,5,6-tetrahydro-2(1*H*)-pyrimidine, octylpyrrolidine, octylcaprolactam, 1,3-dimethyl-2-imidazolinedione, *N-*formylpiperidine, or *N,N*'-1,4-diformylpiperazine); nitriles (e.g., acetonitrile, propionitrile, *n*-butyronitrile, isobutyronitrile, or benzonitrile); alcohols (e.g., methanol, ethanol, *n*-propanol, isopropanol, *n*-butanol, *sec*-butanol, or *tert*-butanol); and ketones (e.g., acetone or *N*-methylpyrrolidone). Preferably, the organic solvent is *n*-propanol.

According to an embodiment of the present disclosure, in the preparation method for the compound of formula I, the molar ratio of the compound of formula XX to R^{k}-C(O)-H may be from 1:1 to 1:5, preferably 1:1.1 to 1:1.3.

According to an embodiment of the present disclosure, in the preparation method for the compound of formula I, the molar ratio of the compound of formula XX to borane or 2-picoline borane may be 1:1 to 1:5, preferably 1: 1.1 to 1:1.3.

According to an embodiment of the present disclosure, in the preparation method for the compound of formula I, the molar ratio of the compound of formula XX to a base may be 1:1 to 1:5, preferably 1:1.5 to 1:3.5, such as 1:1.8 to 1:2.2.

According to an embodiment of the present disclosure, the reaction in the preparation method for the compound of formula I may be carried out at a temperature of 15-50 °C, for example, 20-30 °C, such as room temperature.

According to an embodiment of the present disclosure, the compound of formula XX may also be reacted in a salt form (such as an acid addition salt) thereof as a starting material. Preferably, where the reaction is carried out using a salt form of the compound of formula XX, a base may be used for freeing the salt form to the compound of formula XX. The freeing can be carried out alone or by means of a "one-pot process" with the reaction in the preparation method M1A described above.

According to an embodiment of the present disclosure, the base may be an organic base or inorganic base, for example,
an organic base selected from the following: tertiary amines, substituted or unsubstituted pyridines, and substituted or unsubstituted triethylamine, trimethylamine, *N,N-*diisopropylethylamine, tri-*n*-propylamine, tri-*n*-butylamine, tri-*n*-hexylamine, tricyclohexylamine, *N-*methylcyclohexylamine, *N-*methylpyrrolidine, *N*-methylpyrrolidone, *N*-methylpiperidine, *N*-ethylpiperidine, *N,N*-dimethylaniline, *N*-methylmorpholine, pyridine, 2,3-or 4-methylpyridine, 2-methyl-5-ethylpyridine, 2,6-dimethylpyridine, 2,4,6-trimethylpyridine, 4-dimethylaminopyridine, quinoline, methylquinoline, *N,N,N,N*-tetramethylethylenediamine, *N,N-*dimethyl-1,4-diazacyclohexane, *N,N*-diethyl-1,4-diazacyclohexane, 1,8-bis(dimethylamino)naphthalene, diazabicyclooctane (DABCO), diazabicyclononane (DBN), diazabicycloundecane (DBU), butylimidazole, methylimidazole, sodium *tert*-butoxide and potassium *tert*-butoxide; or
an inorganic base selected from the following: hydrides, hydroxides, amides, alkoxides, acetates, fluorides, phosphates, carbonates and bicarbonates of alkali metals or alkaline earth metals, such as sodium amide, sodium hydride, lithium diisopropylamide, sodium methoxide, potassium *tert*-butoxide, sodium hydroxide, potassium hydroxide, sodium acetate, sodium phosphate, potassium phosphate, potassium fluoride, cesium fluoride, sodium carbonate, potassium carbonate, potassium bicarbonate, sodium bicarbonate, and cesium carbonate.

According to an exemplary embodiment of the present disclosure, the preparation method M1A comprises subjecting compound 20 to the following reaction to prepare compound 1:

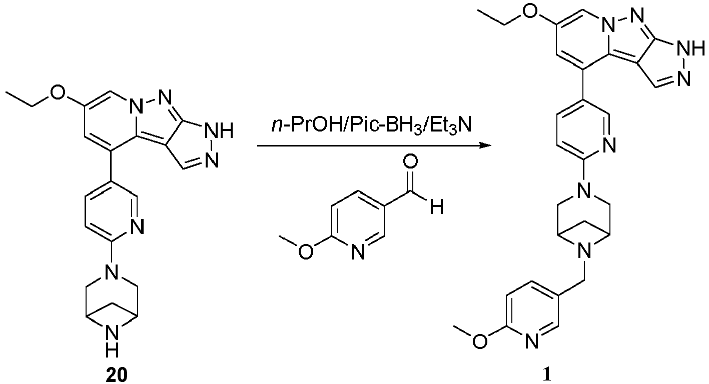

The present disclosure further provides the compound of formula XX described above, such as compound 20.

The present disclosure further provides use of the compound of formula XX described above (e.g., compound 20) in the preparation of the compound of formula I (e.g., compound 1).

The present disclosure further provides a compound of formula XIX shown below: 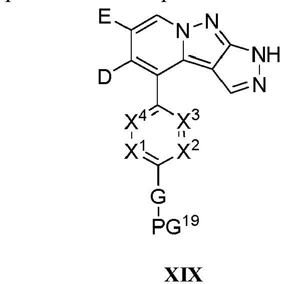 wherein,
G is selected from the following groups unsubstituted or optionally substituted with one, two or more R^{e}: 5- to 20-membered heteroaryl, 3- to 20-membered heterocyclyl, 5- to 20-membered heteroaryloxy, or 3- to 20-membered heterocyclyloxy, wherein G has at least 1 N atom, and the N atom is connected to PG¹⁹,
X¹, X², X³, X⁴, D, E, and R^{e} are independently defined as described above;
PG¹⁹ is an amino protecting group.

According to an embodiment of the present disclosure, PG¹⁹ may be selected from one of amino protecting groups known to those skilled in the art, such as *tert*-butoxycarbonyl (Boc), cyclobutoxycarbonyl, benzyloxycarbonyl (CBz), *p*-methoxybenzylcarbonyl (Moz), 2-biphenyl-2-propoxycarbonyl (BPoc), 2,2,2-trichloroethoxycarbonyl (Troc), phthalimidyl, *p*-toluenesulfonyl, trifluoroacetyl, (9*H*-fluoren-9-ylmethoxy)carbonyl (Fmoc), benzyl, 4-methoxybenzyl, diphenylmethyl, 2-(trimethylsilyl)ethoxycarbonyl (Teoc), adamantyloxycarbonyl (Adoc), formyl, acetyl, and the like.

The present disclosure further provides a preparation method M20 for the compound of formula XX, comprising reacting a compound of formula XIX under a condition where PG¹⁹ is removed to give the compound of formula XX.

The condition where PG¹⁹ of the compound of formula XIX is removed is known to those skilled in the art for removing an amino protecting group. For example, PG¹⁹ group of the compound of formula XIX may be removed in the presence of an acid, wherein the acid may be selected from one, two or more of inorganic acids or organic acids such as hydrochloric acid, sulfuric acid, formic acid, acetic acid, and the like.

According to an embodiment of the present disclosure, in the preparation method for the compound of formula XX, the molar ratio of the compound of formula XIX to the acid may be 1:1 to 1:5, such as 1:3 to 1:4.

According to an embodiment of the present disclosure, the reaction of the compound of formula XIX for removing PG¹⁹ may be carried out in the presence of an organic solvent. The organic solvent is defined as described above, for example, the organic solvent is an alcohol solvent, such as one, two or more selected from methanol, ethanol, *n*-propanol, isopropanol, *n*-butanol, *sec-*butanol, and *tert-*butanol.

According to an embodiment of the present disclosure, the reaction in the preparation method for the compound of formula I may be carried out at a temperature of 40-70 °C, for example, 50-60 °C.

According to an embodiment of the present disclosure, the preparation method M20 comprises subjecting compound 19 to the following reaction to prepare compound 20:

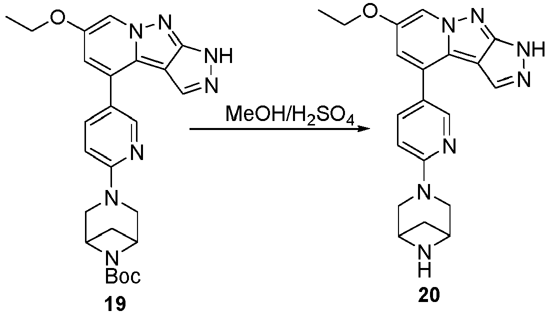

Accordingly, the present disclosure further provides compound 19.

The present disclosure further provides use of the compound of formula XIX described above (e.g., compound 19) in the preparation of the compound of formula XX (e.g., compound 20).

The present disclosure further provides a compound of formula XI shown below: 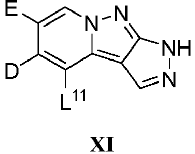 wherein,
D and E are independently defined as described above;
L¹¹ is selected from leaving groups, such as the following groups unsubstituted or substituted with 1, 2, 3, 4, 5 or 6 halogens: C₁₋₆ alkylsulfonyloxy and C₁₋₆ alkylbenzenesulfonyloxy (e.g., methanesulfonyloxy (MsO-), trifluoromethanesulfonyloxy (TfO-), and *p*-toluenesulfonyloxy (TsO-)), and halogen (e.g., F, Cl, Br, or I).

The present disclosure further provides a compound of formula XVIII shown below: 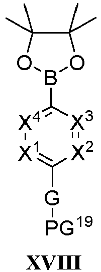 wherein X¹, X², X³, X⁴, G, and PG¹⁹ are independently defined as described above.

The present disclosure further provides a preparation method M19A for the compound of formula XIX, comprising reacting a compound of formula XI with a compound of formula XVIII to give the compound of formula XIX: 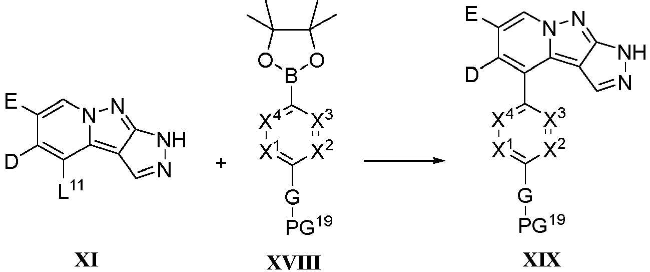 wherein X¹, X², X³, X⁴, D, E, G, L¹¹, and PG¹⁹ are independently defined as described above. According to an embodiment of the present disclosure, in the preparation method for the compound of formula XIX described above, the reaction may be carried out in the presence of a base.

The base may be an organic base or an inorganic base, for example,
an organic base selected from the following: tertiary amines, substituted or unsubstituted pyridines, and substituted or unsubstituted triethylamine, trimethylamine, *N,N-*diisopropylethylamine, tri-*n*-propylamine, tri-*n*-butylamine, tri-*n*-hexylamine, tricyclohexylamine, *N*-methylcyclohexylamine, *N*-methylpyrrolidine, *N*-methylpyrrolidone, *N*-methylpiperidine, *N*-ethylpiperidine, *N,N-*dimethylaniline, *N*-methylmorpholine, pyridine, 2,3- or 4-methylpyridine, 2-methyl-5-ethylpyridine, 2,6-dimethylpyridine, 2,4,6-trimethylpyridine, 4-dimethylaminopyridine, quinoline, methylquinoline, *N,N,N,N*-tetramethylethylenediamine, *N,N-*dimethyl-1,4-diazacyclohexane, *N,N-*diethyl-1,4-diazacyclohexane, 1,8-bis(dimethylamino)naphthalene, diazabicyclooctane (DABCO), diazabicyclononane (DBN), diazabicycloundecane (DBU), butylimidazole, methylimidazole, sodium *tert*-butoxide and potassium *tert*-butoxide; or
an inorganic base selected from the following: hydrides, hydroxides, amides, alkoxides, acetates, fluorides, phosphates, carbonates and bicarbonates of alkali metals or alkaline earth metals, such as sodium amide, sodium hydride, lithium diisopropylamide, sodium methoxide, potassium tert-butoxide, sodium hydroxide, potassium hydroxide, sodium acetate, sodium phosphate, potassium phosphate, potassium fluoride, cesium fluoride, sodium carbonate, potassium carbonate, potassium bicarbonate, sodium bicarbonate, and cesium carbonate.

According to an embodiment of the present disclosure, the preparation method for the compound of formula XIX described above may be carried out in the presence of an organic solvent or a mixed solvent of an organic solvent and water. The organic solvent may be selected from one or a mixture of two or more of the following: ethers (e.g., ethyl propyl ether, methyl *tert*-butyl ether, *n*-butyl ether, anisole, phenetole, cyclohexyl methyl ether, dimethyl ether, diethyl ether, diphenyl ether, dipropyl ether, diisopropyl ether, di-*n*-butyl ether, diisobutyl ether, diisoamyl ether, ethylene glycol dimethyl ether, isopropyl ethyl ether, diethylene glycol dimethyl ether, triethylene glycol dimethyl ether, tetrahydrofuran, 2-methyltetrahydrofuran, 1,4-dioxane and ethylene oxide, and/or propylene oxide polyethers); sulfoxides (e.g., tetrahydrodioxythiophene and dimethyl sulfoxide, tetramethyl sulfoxide, dipropyl sulfoxide, benzyl methyl sulfoxide, diisobutyl sulfoxide, dibutyl sulfoxide, or diisoamyl sulfoxide); sulfones (e.g., dimethyl sulfone, diethyl sulfone, dipropyl sulfone, dibutyl sulfone, diphenyl sulfone, dihexyl sulfone, methyl ethyl sulfone, ethyl propyl sulfone, ethyl isobutyl sulfone, and cyclopentyl sulfone); aliphatic, cycloaliphatic or aromatic hydrocarbons (e.g., pentane, hexane, heptane, octane, nonane, cyclohexane, methylcyclohexane, petroleum ether, naphtha, octane, benzene, toluene, or xylene); haloalkanes (e.g., dichloromethane, chloroform, carbon tetrachloride, dichloroethane, or trichloroethane); halogenated aromatic compounds (e.g., chlorobenzene or dichlorobenzene); amides (e.g., hexamethylphosphoramide, formamide, *N,N-*dimethylacetamide, *N*-methylformamide, *N,N-*dimethylformamide, *N,N*-dipropylformamide, *N,N-*dibutylformamide, *N*-methylpyrrolidine, *N*-methylcaprolactam, 1,3-dimethyl-3,4,5,6-tetrahydro-2(1*H*)-pyrimidine, octylpyrrolidine, octylcaprolactam, 1,3-dimethyl-2-imidazolinedione, *N*-formylpiperidine, or *N,N'*-1,4-diformylpiperazine); nitriles (e.g., acetonitrile, propionitrile, *n*-butyronitrile, isobutyronitrile, or benzonitrile); alcohols (e.g., methanol, ethanol, *n*-propanol, isopropanol, *n*-butanol, *sec-*butanol, or *tert*-butanol); and ketones (e.g., acetone or *N*-methylpyrrolidone). Preferably, the organic solvent is dioxane.

According to an embodiment of the present disclosure, the preparation method for the compound of formula XIX described above may be carried out in the presence of a catalyst, wherein the catalyst may be a palladium catalyst, such as Pd(dba)₂, PdCl₂, Pd(OAc)₂, Pd(dppf)Cl₂, Pd₂(dba)₃, PdCl₂(CH₃CN)₂, Pd(PPh₃)₄, PdCl₂(PPh₃)₂, Pd(t-Bu)₃, PdCl₂(PPh₃)₂•CH₂Cl₂, Pd(OAc)/PPh₃, PdCl₂[(Pet₃)]₂, Pd(DIPHOS)₂, PdCl₂(Bipy), [PdCl(Ph₂PCH₂PPh₂)]₂, PdCl₂[P(o-Tol)₃]₂, Pd₂(dba)₃/P(o-Tol)₃, Pd₂(dba)/P(furanyl)₃, PdCl₂[P(furanyl)₃]₂, PdCl₂(PMePh₂)₂, PdCl₂[P(4-F-Ph)₃]₂, PdCl₂[P(C₆F₆)₃]₂, PdCl₂[P(2-COOH-Ph)(Ph)₂]₂, and PdCl₂[P(4-COOH-Ph)(Ph)₂]₂.

According to an embodiment of the present disclosure, the reaction in the preparation method for the compound of formula XIX described above may be carried out at a temperature of not less than 50 °C, for example, not less than 80 °C, such as 85 °C.

According to an embodiment of the present disclosure, in the preparation method for the compound of formula XIX described above, the molar ratio of the compound of formula XI to the compound of formula XVIII may be 1:1 to 1:2, for example, 1:1.1 to 1:1.3, such as 1:1.2 to 1:1.3.

According to an embodiment of the present disclosure, in the preparation method for the compound of formula XIX described above, the molar ratio of the compound of formula XI to the base may be 1:1 to 1:5, for example, 1:1.5 to 1:3.5, such as 1:1.8 to 1:2.2.

According to an embodiment of the present disclosure, in the preparation method for the compound of formula XIX described above, the molar ratio of the compound of formula XI to the catalyst may be 1:0.001 to 1:0.05, such as 1:0.02 to 1:0.03.

According to an embodiment of the present disclosure, the preparation method M19A comprises subjecting compound 11 and compound 18 to the following reaction to prepare compound 19:

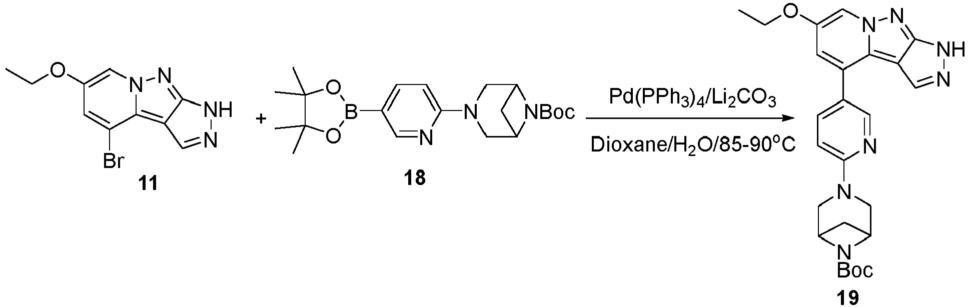

Accordingly, the present disclosure further provides the compound 11 or compound 18 described above.

The present disclosure further provides use of the compound of formula XI (e.g., compound 11) and/or the compound of formula XVIII (e.g., compound 18) described above in the preparation of the compound of formula XIX (e.g., compound 19).

The present disclosure further provides a preparation method M11 for the compound of formula XI, comprising reacting a compound of formula X with hydrazine (e.g., hydrazine hydrate) to give the compound of formula XI: 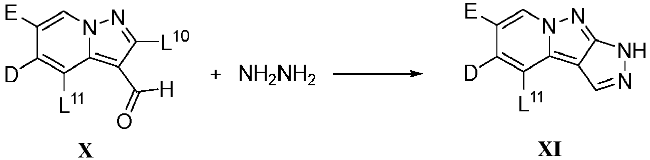 wherein,
D, E, and L¹¹ are independently defined as described above;
L¹⁰ is selected from a leaving group, for example, halogen, such as F, Cl, Br, or I.

According to an embodiment of the present disclosure, the preparation method for the compound of formula XI described above may be carried out in the presence of an organic solvent or a mixed solvent of an organic solvent and water. The organic solvent may be selected from one or a mixture of two or more of the following: ethers (e.g., ethyl propyl ether, methyl *tert*-butyl ether, *n*-butyl ether, anisole, phenetole, cyclohexyl methyl ether, dimethyl ether, diethyl ether, diphenyl ether, dipropyl ether, diisopropyl ether, di-*n*-butyl ether, diisobutyl ether, diisoamyl ether, ethylene glycol dimethyl ether, isopropyl ethyl ether, diethylene glycol dimethyl ether, triethylene glycol dimethyl ether, tetrahydrofuran, 2-methyltetrahydrofuran, 1,4-dioxane and ethylene oxide, and/or propylene oxide polyethers); sulfoxides (e.g., tetrahydrodioxythiophene and dimethyl sulfoxide, tetramethyl sulfoxide, dipropyl sulfoxide, benzyl methyl sulfoxide, diisobutyl sulfoxide, dibutyl sulfoxide, or diisoamyl sulfoxide); sulfones (e.g., dimethyl sulfone, diethyl sulfone, dipropyl sulfone, dibutyl sulfone, diphenyl sulfone, dihexyl sulfone, methyl ethyl sulfone, ethyl propyl sulfone, ethyl isobutyl sulfone, and cyclopentyl sulfone); aliphatic, cycloaliphatic or aromatic hydrocarbons (e.g., pentane, hexane, heptane, octane, nonane, cyclohexane, methylcyclohexane, petroleum ether, naphtha, octane, benzene, toluene, or xylene); haloalkanes (e.g., dichloromethane, chloroform, carbon tetrachloride, dichloroethane, or trichloroethane); halogenated aromatic compounds (e.g., chlorobenzene or dichlorobenzene); amides (e.g., hexamethylphosphoramide, formamide, *N,N-*dimethylacetamide, *N*-methylformamide, *N,N-*dimethylformamide, *N,N-*dipropylformamide, *N,N*-dibutylformamide, *N*-methylpyrrolidine, *N*-methylcaprolactam, 1,3-dimethyl-3,4,5,6-tetrahydro-2(1*H*)-pyrimidine, octylpyrrolidine, octylcaprolactam, 1,3-dimethyl-2-imidazolinedione, *N-*formylpiperidine, or *N,N'*-1,4-diformylpiperazine); nitriles (e.g., acetonitrile, propionitrile, *n*-butyronitrile, isobutyronitrile, or benzonitrile); alcohols (e.g., methanol, ethanol, *n*-propanol, isopropanol, *n*-butanol, *sec-*butanol, or *tert*-butanol); and ketones (e.g., acetone or *N*-methylpyrrolidone). Preferably, the organic solvent is dimethyl sulfoxide.

According to an embodiment of the present disclosure, in the preparation method for the compound of formula XI, the reaction may be carried out at a temperature of not less than 100 °C, such as 110-115 °C.

According to an embodiment of the present disclosure, in the preparation method for the compound of formula XI, the molar ratio of the compound of formula X to hydrazine may be 1:1 to 1:15, for example, 1:2 to 1:10, such as 1:6 to 1:8.

According to an embodiment of the present disclosure, the preparation method M11 comprises subjecting compound 10 to the following reaction to prepare compound 11:

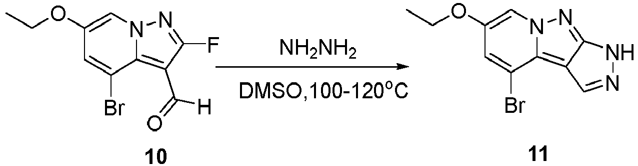

The present disclosure further provides the compound of formula X described above, such as compound 10.

The present disclosure further provides use of the compound of formula X described above (e.g., compound 10) in the preparation of the compound of formula XI (e.g., compound 11).

The present disclosure further provides a preparation method M10A for the compound of formula X, comprising reacting a compound of formula XIII with a compound of R²¹-L¹³ to give the compound of formula X: 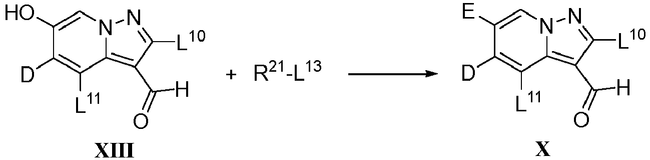 wherein,
D, L¹⁰, L¹¹, and R²¹ are independently defined as described above;
E is selected from -O-R²¹,
L¹³ is selected from leaving groups, for example, halogens, such as the following groups unsubstituted or substituted with 1, 2, 3, 4, 5 or 6 halogens: C₁₋₆ alkylsulfonyloxy and C₁₋₆ alkylbenzenesulfonyloxy (e.g., methanesulfonyloxy (MsO-), trifluoromethanesulfonyloxy (TfO-), and *p*-toluenesulfonyloxy (TsO-)), and F, Cl, Br or I.

According to an embodiment of the present disclosure, the preparation method for the compound of formula X may be carried out in the presence of a base.

According to an embodiment of the present disclosure, the base may be an organic base or inorganic base, for example,
an organic base selected from the following: tertiary amines, substituted or unsubstituted pyridines, and substituted or unsubstituted triethylamine, trimethylamine, *N,N-*diisopropylethylamine, tri-*n*-propylamine, tri-*n*-butylamine, tri-*n*-hexylamine, tricyclohexylamine, *N-*methylcyclohexylamine, *N*-methylpyrrolidine, *N*-methylpyrrolidone, *N*-methylpiperidine, *N*-ethylpiperidine, *N,N*-dimethylaniline, *N*-methylmorpholine, pyridine, 2,3-or 4-methylpyridine, 2-methyl-5-ethylpyridine, 2,6-dimethylpyridine, 2,4,6-trimethylpyridine, 4-dimethylaminopyridine, quinoline, methylquinoline, *N,N,N,N-*tetramethylethylenediamine, *N,N-*dimethyl-1,4-diazacyclohexane, *N,N-*diethyl-1,4-diazacyclohexane, 1,8-bis(dimethylamino) naphthalene, diazabicyclooctane (DABCO), diazabicyclononane (DBN), diazabicycloundecane (DBU), butylimidazole, methylimidazole, sodium *tert-*butoxide and potassium *tert*-butoxide; or
an inorganic base selected from the following: hydrides, hydroxides, amides, alkoxides, acetates, fluorides, phosphates, carbonates and bicarbonates of alkali metals or alkaline earth metals, such as sodium amide, sodium hydride, lithium diisopropylamide, sodium methoxide, potassium *tert*-butoxide, sodium hydroxide, potassium hydroxide, sodium acetate, sodium phosphate, potassium phosphate, potassium fluoride, cesium fluoride, sodium carbonate, potassium carbonate, potassium bicarbonate, sodium bicarbonate, and cesium carbonate.

According to an embodiment of the present disclosure, the preparation method for the compound of formula X may be carried out in the presence of an organic solvent. The organic solvent may be selected from one or a mixture of two or more of the following: ethers (e.g., ethyl propyl ether, methyl *tert*-butyl ether, *n*-butyl ether, anisole, phenetole, cyclohexyl methyl ether, dimethyl ether, diethyl ether, diphenyl ether, dipropyl ether, diisopropyl ether, di-*n*-butyl ether, diisobutyl ether, diisoamyl ether, ethylene glycol dimethyl ether, isopropyl ethyl ether, diethylene glycol dimethyl ether, triethylene glycol dimethyl ether, tetrahydrofuran, 2-methyltetrahydrofuran, 1,4-dioxane and ethylene oxide, and/or propylene oxide polyethers); sulfoxides (e.g., tetrahydrodioxythiophene and dimethyl sulfoxide, tetramethyl sulfoxide, dipropyl sulfoxide, benzyl methyl sulfoxide, diisobutyl sulfoxide, dibutyl sulfoxide, or diisoamyl sulfoxide); sulfones (e.g., dimethyl sulfone, diethyl sulfone, dipropyl sulfone, dibutyl sulfone, diphenyl sulfone, dihexyl sulfone, methyl ethyl sulfone, ethyl propyl sulfone, ethyl isobutyl sulfone, and cyclopentyl sulfone); aliphatic, cycloaliphatic or aromatic hydrocarbons (e.g., pentane, hexane, heptane, octane, nonane, cyclohexane, methylcyclohexane, petroleum ether, naphtha, octane, benzene, toluene, or xylene); haloalkanes (e.g., dichloromethane, chloroform, carbon tetrachloride, dichloroethane, or trichloroethane); halogenated aromatic compounds (e.g., chlorobenzene or dichlorobenzene); amides (e.g., hexamethylphosphoramide, formamide, *N,N-*dimethylacetamide, *N*-methylformamide, *N,N-*dimethylformamide, *N,N-*dipropylformamide, *N,N-*dibutylformamide, *N* methylpyrrolidine, *N*-methylcaprolactam, 1,3-dimethyl-3,4,5,6-tetrahydro-2(1*H*)-pyrimidine, octylpyrrolidine, octylcaprolactam, 1,3-dimethyl-2-imidazolinedione, *N*-formylpiperidine, or *N,N'*-1,4-diformylpiperazine); nitriles (e.g., acetonitrile, propionitrile, *n*-butyronitrile, isobutyronitrile, or benzonitrile); alcohols (e.g., methanol, ethanol, *n*-propanol, isopropanol, *n*-butanol, *sec-*butanol, or *tert*-butanol); and ketones (e.g., acetone or *N*-methylpyrrolidone). Preferably, the organic solvent is selected from dimethyl sulfoxide or *N,N-*dimethylformamide.

According to an embodiment of the present disclosure, in the preparation method for the compound of formula X, the molar ratio of the compound of formula XIII to the compound of R²¹-L¹³ may be 1:1 to 1:2, for example, 1:1 to 1:1.2, such as 1:1 to 1:1.1.

According to an embodiment of the present disclosure, in the preparation method for the compound of formula X, the molar ratio of the compound of formula XIII to the base is 1:1 to 1:2, for example, 1: 1.1 to 1:1.3, such as 1: 1.1 to 1:1.2.

According to an embodiment of the present disclosure, the reaction in the preparation method for the compound of formula X may be carried out at a temperature of not less than 50 °C, for example, 60-70 °C, such as 65 °C.

According to an embodiment of the present disclosure, the preparation method M10A comprises subjecting compound 13 to the following reaction to prepare compound 10:

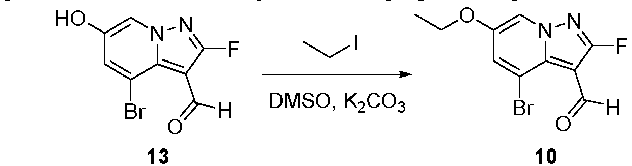

The present disclosure further provides the compound of formula XIII described above, such as compound 13.

The present disclosure further provides use of the compound of formula XIII described above (e.g., compound 13) in the preparation of the compound of formula X (e.g., compound 10).

The present disclosure further provides a preparation method M13 for the compound of formula XIII, comprising reacting a compound of formula IIIB in the presence of phosphorus oxychloride (POCl₃), wherein the compound of formula IIIB has the following structure: 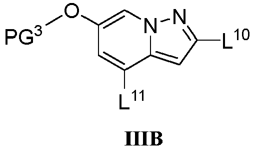 wherein,
L¹⁰ and L¹¹ are independently defined as described above;
PG³ is selected from a hydroxyl protecting group.

According to an embodiment of the present disclosure, PG³ may be selected from hydroxyl protecting groups known to those skilled in the art, such as the following groups unsubstituted or optionally substituted with 1, 2, 3, 4 or 5 substituents selected from C₁₋₆ alkyl, C₁₋₆ alkyloxy and halogen: benzyl, C₁₋₆ alkyl, tri(C₁₋₆ alkyl)silyl, *tert*-butyldimethylsilyl, *tert*-butyldiphenylsilyl, allyl, triphenylmethyl, C₁₋₆ alkyloxymethyl, benzyloxymethyl, C₁₋₆ alkylcarbonyl, C₁₋₆ haloalkylcarbonyl, and benzoyl, and an example thereof may be selected from benzyl, 4-methoxybenzyl, 4-methylbenzyl, 4-chlorobenzyl, 4-bromobenzyl, or 2,3,4-trimethoxybenzyl. According to an embodiment of the present disclosure, the preparation method M13 comprises reacting compound 3b in the presence of phosphorus oxychloride (POCl₃), wherein compound 3b has the following structure:

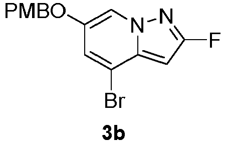

According to an embodiment of the present disclosure, in the preparation method for the compound of formula XIII, the compound of formula IIIB (e.g., compound 3b) may be first reacted with phosphorus oxychloride to give a compound of formula XII, and then the compound of formula XII is further reacted to give the compound of formula XIII, wherein the compound of formula XII has the following structure: 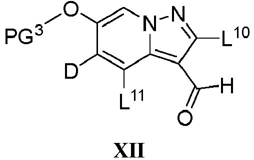 wherein L¹⁰, L¹¹, and PG³ are independently defined as described above.

According to an embodiment of the present disclosure, in the preparation method for the compound of formula XIII, the molar ratio of the compound of formula IIIB to phosphorus oxychloride may be 1:2 to 1:4, for example, 1:2 to 1:3, such as 1:2.4 to 1:2.5.

According to an embodiment of the present disclosure, the preparation method for the compound of formula XIII may be carried out by a one-pot process.

According to an embodiment of the present disclosure, a reaction solvent for the reaction of the compound of formula IIIB with phosphorus oxychloride to give the compound of formula XII may be the organic solvent described above, for example, *N,N-*dimethylformamide.

According to an embodiment of the present disclosure, the reaction of the compound of formula IIIB with phosphorus oxychloride to give the compound of formula XII may be carried out at a temperature of -5 to 80 °C, for example, 0-70 °C.

According to an embodiment of the present disclosure, a reaction solvent for further reacting the compound of formula XII to give the compound of formula XIII may be a mixed solvent of the organic solvent described above and water, for example, a mixed solvent of *N,N-*dimethylformamide and water.

According to an embodiment of the present disclosure, the reaction for further reacting the compound of formula XII to give the compound of formula XIII may be carried out at a temperature of 15 to 40 °C, for example, 20 to 35 °C, such as 25 to 30 °C.

According to an embodiment of the present disclosure, the preparation method M13 comprises preparing compound 12 from compound 3b and preparing compound 13 from compound 12 by the following reaction:

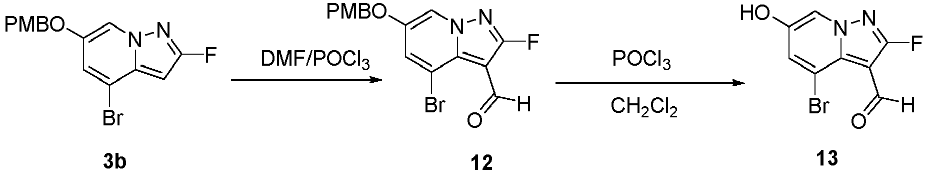

The present disclosure further provides the compound of formula IIIB described above, such as compound 3b.

The present disclosure further provides the compound of formula XII described above, such as compound 12.

The present disclosure further provides use of the compound of formula IIIB (e.g., compound 3b) and/or the compound of formula XII (e.g., compound 12) described above in the preparation of the compound of formula XIII (e.g., compound 13).

The present disclosure further provides use of the compound of formula IIIB described above (e.g., compound 3b) in the preparation of the compound of formula XII (e.g., compound 12).

The present disclosure further provides a preparation method M10B for the compound of formula X, comprising reacting a compound of formula XVII with phosphorus oxychloride to give the compound of formula X: 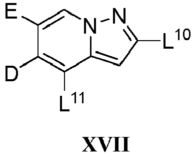 wherein D, E, L¹⁰, and L¹¹ are independently defined as described above.

According to an embodiment of the present disclosure, the preparation method for the compound of formula X may be carried out in the presence of an organic solvent. The organic solvent may be selected from one or a mixture of two or more of the following: ethers (e.g., ethyl propyl ether, methyl *tert*-butyl ether, *n*-butyl ether, anisole, phenetole, cyclohexyl methyl ether, dimethyl ether, diethyl ether, diphenyl ether, dipropyl ether, diisopropyl ether, di-*n*-butyl ether, diisobutyl ether, diisoamyl ether, ethylene glycol dimethyl ether, isopropyl ethyl ether, diethylene glycol dimethyl ether, triethylene glycol dimethyl ether, tetrahydrofuran, 2-methyltetrahydrofuran, 1,4-dioxane and ethylene oxide, and/or propylene oxide polyethers); sulfoxides (e.g., tetrahydrodioxythiophene and dimethyl sulfoxide, tetramethyl sulfoxide, dipropyl sulfoxide, benzyl methyl sulfoxide, diisobutyl sulfoxide, dibutyl sulfoxide, or diisoamyl sulfoxide); sulfones (e.g., dimethyl sulfone, diethyl sulfone, dipropyl sulfone, dibutyl sulfone, diphenyl sulfone, dihexyl sulfone, methyl ethyl sulfone, ethyl propyl sulfone, ethyl isobutyl sulfone, and cyclopentyl sulfone); aliphatic, cycloaliphatic or aromatic hydrocarbons (e.g., pentane, hexane, heptane, octane, nonane, cyclohexane, methylcyclohexane, petroleum ether, naphtha, octane, benzene, toluene, or xylene); haloalkanes (e.g., dichloromethane, chloroform, carbon tetrachloride, dichloroethane, or trichloroethane); halogenated aromatic compounds (e.g., chlorobenzene or dichlorobenzene); amides (e.g., hexamethylphosphoramide, formamide, *N,N-*dimethylacetamide, *N*-methylformamide, *N,N-*dimethylformamide, *N,N-*dipropylformamide, *N,N-*dibutylformamide, *N-*methylpyrrolidine, *N*-methylcaprolactam, 1,3-dimethyl-3,4,5,6-tetrahydro-2(1*H*)-pyrimidine, octylpyrrolidine, octylcaprolactam, 1,3-dimethyl-2-imidazolinedione, *N-*formylpiperidine, or *N,N'*-1,4-diformylpiperazine); nitriles (e.g., acetonitrile, propionitrile, *n*-butyronitrile, isobutyronitrile, or benzonitrile); alcohols (e.g., methanol, ethanol, *n*-propanol, isopropanol, *n*-butanol, *sec-*butanol, or *tert*-butanol); and ketones (e.g., acetone or *N*-methylpyrrolidone). Preferably, the organic solvent is selected from dimethyl sulfoxide or *N,N-*dimethylformamide.

According to an embodiment of the present disclosure, in the preparation method for the compound of formula X, the molar ratio of the compound of formula XVII to phosphorus oxychloride may be 1:2 to 1:8, for example, 1:3 to 1:6, such as 1:4 to 1:5:2 to 1:4. for example, 1:2 to 1:3, such as 1:2.4 to 1:2.5.

According to an embodiment of the present disclosure, the preparation method M10B comprises subjecting compound 17 to the following reaction to prepare compound 10:

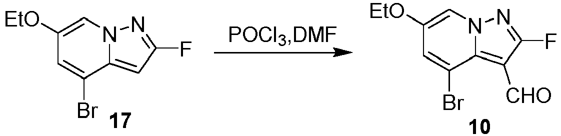

The present disclosure further provides the compound of formula XVII described above, such as compound 17.

The present disclosure further provides use of the compound of formula XVII described above (e.g., compound 17) in the preparation of the compound of formula X (e.g., compound 10).

The present disclosure further provides a preparation method M17 for the compound of formula XVII, comprising reacting a compound of formula XVI with an alkali metal halide or an organic halide salt to give the compound of formula XVII: 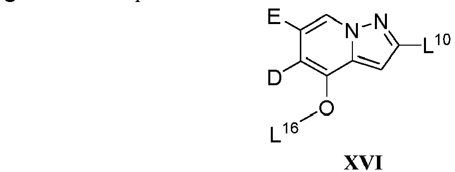 wherein D, E, and L¹⁰ are independently defined as described above;
L¹⁶ is the following group unsubstituted or substituted with 1, 2, 3, 4, 5 or 6 halogens: C₁₋₆ alkylsulfonyl or C₁₋₆ alkylbenzenesulfonyl, such as methanesulfonyl (Ms-), trifluoromethanesulfonyl (Tf-) orp-toluenesulfonyl (Ts-).

According to an embodiment of the present disclosure, in the preparation method for the compound of formula XVII, the alkali metal halide or the organic halide salt may be selected from, for example, at least one of LiBr, NaBr, KBr, CsBr, and Bu₄NBr.

According to an embodiment of the present disclosure, the preparation method for the compound of formula XVII may be carried out in the presence of an organic solvent. The organic solvent may be selected from one or a mixture of two or more of the following: ethers (e.g., ethyl propyl ether, methyl *tert*-butyl ether, *n*-butyl ether, anisole, phenetole, cyclohexyl methyl ether, dimethyl ether, diethyl ether, diphenyl ether, dipropyl ether, diisopropyl ether, di-*n*-butyl ether, diisobutyl ether, diisoamyl ether, ethylene glycol dimethyl ether, isopropyl ethyl ether, diethylene glycol dimethyl ether, triethylene glycol dimethyl ether, tetrahydrofuran, 2-methyltetrahydrofuran, dioxane and ethylene oxide, and/or propylene oxide polyethers); sulfoxides (e.g., tetrahydrodioxythiophene and dimethyl sulfoxide, tetramethyl sulfoxide, dipropyl sulfoxide, benzyl methyl sulfoxide, diisobutyl sulfoxide, dibutyl sulfoxide, or diisoamyl sulfoxide); sulfones (e.g., dimethyl sulfone, diethyl sulfone, dipropyl sulfone, dibutyl sulfone, diphenyl sulfone, dihexyl sulfone, methyl ethyl sulfone, ethyl propyl sulfone, ethyl isobutyl sulfone, and cyclopentyl sulfone); aliphatic, cycloaliphatic or aromatic hydrocarbons (e.g., pentane, hexane, heptane, octane, nonane, cyclohexane, methylcyclohexane, petroleum ether, naphtha, octane, benzene, toluene, or xylene); haloalkanes (e.g., dichloromethane, chloroform, carbon tetrachloride, dichloroethane, or trichloroethane); halogenated aromatic compounds (e.g., chlorobenzene or dichlorobenzene); amides (e.g., hexamethylphosphoramide, formamide, *N,N-*dimethylacetamide, *N*-methylformamide, *N,N-*dimethylformamide, *N,N-*dipropylformamide, *N,N-*dibutylformamide, *N-*methylpyrrolidine, *N*-methylcaprolactam, 1,3-dimethyl-3,4,5,6-tetrahydro-2(1*H*)-pyrimidine, octylpyrrolidine, octylcaprolactam, 1,3-dimethyl-2-imidazolinedione, *N-*formylpiperidine, or *N,N'*-1,4-diformylpiperazine); nitriles (e.g., acetonitrile, propionitrile, *n*-butyronitrile, isobutyronitrile, or benzonitrile); alcohols (e.g., methanol, ethanol, *n*-propanol, isopropanol, *n*-butanol, *sec-*butanol, or *tert*-butanol); and ketones (e.g., acetone or *N*-methylpyrrolidone). Preferably, the organic solvent is n-propanol. According to an embodiment of the present disclosure, in the preparation method for the compound of formula XVII, the molar ratio of the compound of formula XVI to the alkali metal halide or the organic halide salt may be 1:1 to 1:5, for example, 1:1 to 1:2, such as 1:1.5.

According to an embodiment of the present disclosure, the preparation method for the compound of formula XVII may be carried out in the presence of a catalyst, wherein the catalyst is, for example, chloro(pentamethylcyclopentadienyl)bis(triphenylphosphine)ruthenium(II), tris(acetonitrile) pentamethylcyclopentadienylruthenium(II) trifluoromethanesulfonate, pentamethylcyclopentadienyltris (acetonitrile)ruthenium(II) hexafluorophosphate, chloro(pentamethylcyclopentadienyl)ruthenium(II) (dimer), chloro(pentamethylcyclopentadienyl)ruthenium(I) (tetramer), tris(acetonitrile) tetramethylcyclopentadienylruthenium(II) trifluoromethanesulfonate, or dichloro (1,5-cyclooctadien)ruthenium(II).

According to an embodiment of the present disclosure, in the preparation method for the compound of formula XVII, the molar ratio of the compound of formula XVI to the catalyst may be 1:0.01 to 1:0.1, such as 1:0.05.

According to an embodiment of the present disclosure, the reaction in the preparation method for the compound of formula XVII may be carried out at a temperature of not less than 50 °C, for example, not less than 80 °C, such as 90-100 °C.

According to an embodiment of the present disclosure, the preparation method M17 comprises subjecting compound 16 to the following reaction to prepare compound 17:

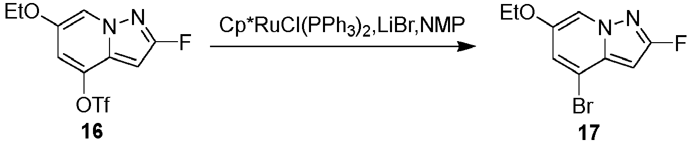

The present disclosure further provides the compound of formula XVI described above, such as compound 16.

The present disclosure further provides use of the compound of formula XVI described above (e.g., compound 16) in the preparation of the compound of formula XVII (e.g., compound 17).

The present disclosure further provides a preparation method M16 for the compound of formula XVI, comprising reacting a compound of formula XV with a sulfonylation reagent to give the compound of formula XVI: 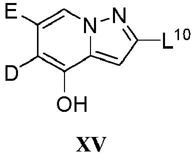 wherein D, E, and L¹⁰ are independently defined as described above.

According to an embodiment of the present disclosure, the preparation method for the compound of formula XVI may be carried out in the presence of an organic solvent. The organic solvent may be selected from one or a mixture of two or more of the following: ethers (e.g., ethyl propyl ether, methyl *tert*-butyl ether, *n*-butyl ether, anisole, phenetole, cyclohexyl methyl ether, dimethyl ether, diethyl ether, diphenyl ether, dipropyl ether, diisopropyl ether, di-*n*-butyl ether, diisobutyl ether, diisoamyl ether, ethylene glycol dimethyl ether, isopropyl ethyl ether, diethylene glycol dimethyl ether, triethylene glycol dimethyl ether, tetrahydrofuran, 2-methyltetrahydrofuran, 1,4-dioxane and ethylene oxide, and/or propylene oxide polyethers); sulfoxides (e.g., tetrahydrodioxythiophene and dimethyl sulfoxide, tetramethyl sulfoxide, dipropyl sulfoxide, benzyl methyl sulfoxide, diisobutyl sulfoxide, dibutyl sulfoxide, or diisoamyl sulfoxide); sulfones (e.g., dimethyl sulfone, diethyl sulfone, dipropyl sulfone, dibutyl sulfone, diphenyl sulfone, dihexyl sulfone, methyl ethyl sulfone, ethyl propyl sulfone, ethyl isobutyl sulfone, and cyclopentyl sulfone); aliphatic, cycloaliphatic or aromatic hydrocarbons (e.g., pentane, hexane, heptane, octane, nonane, cyclohexane, methylcyclohexane, petroleum ether, naphtha, octane, benzene, toluene, or xylene); haloalkanes (e.g., dichloromethane, chloroform, carbon tetrachloride, dichloroethane, or trichloroethane); halogenated aromatic compounds (e.g., chlorobenzene or dichlorobenzene); amides (e.g., hexamethylphosphoramide, formamide, *N,N-*dimethylacetamide, *N*-methylformamide, *N,N-*dimethylformamide, *N,N*-dipropylformamide, *N,N-*dibutylformamide, *N-*methylpyrrolidine, *N*-methylcaprolactam, 1,3-dimethyl-3,4,5,6-tetrahydro-2(1*H*)-pyrimidine, octylpyrrolidine, octylcaprolactam, 1,3-dimethyl-2-imidazolinedione, *N-*formylpiperidine, or *N,N'*-1,4-diformylpiperazine); nitriles (e.g., acetonitrile, propionitrile, *n*-butyronitrile, isobutyronitrile, or benzonitrile); alcohols (e.g., methanol, ethanol, *n*-propanol, isopropanol, *n*-butanol, *sec*-butanol, or *tert*-butanol); and ketones (e.g., acetone or *N*-methylpyrrolidone). Preferably, the organic solvent is selected from dichloromethane.

According to an embodiment of the present disclosure, in the preparation method for the compound of formula XVI, the sulfonylation reagent may be selected from one of C₁₋₆ alkylsulfonyl chloride unsubstituted or substituted with 1, 2, 3, 4, 5 or 6 halogens, C₁₋₆ alkylsulfonic anhydride unsubstituted or substituted with 1, 2, 3, 4, 5 or 6 halogens, and C₁₋₆ alkylbenzenesulfonyl chloride unsubstituted or substituted with 1, 2, 3, 4, 5 or 6 halogens, such as methanesulfonyl chloride, methanesulfonic anhydride, trifluoromethanesulfonyl chloride, trifluoromethanesulfonic anhydride, *p*-toluenesulfonyl chloride, *p*-toluenesulfonic anhydride, and 1,1,1-trifluoro-*N*-phenyl-*N*-((trifluoromethyl)sulfonyl)methanesulfonamide (PhN(Tf)₂). According to an embodiment of the present disclosure, in the preparation method for the compound of formula XVI, the molar ratio of the compound of formula XV to the sulfonylation reagent may be 1:1 to 1:5, for example, 1:1 to 1:1.2, such as 1: 1.1 to 1:1.15.

According to an embodiment of the present disclosure, the preparation method M16 comprises subjecting compound 15 to the following reaction to prepare compound 16:

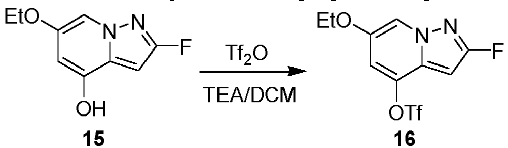

The present disclosure further provides the compound of formula XV described above, such as compound 15.

The present disclosure further provides use of the compound of formula XV described above (e.g., compound 15) in the preparation of the compound of formula XVI (e.g., compound 16).

The present disclosure further provides a preparation method M15 for the compound of formula XV, comprising reacting a compound of formula XIV with an acid to give the compound of formula XV: 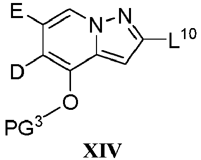 wherein D, E, L¹⁰, and PG³ are independently defined as described above.

According to an embodiment of the present disclosure, in the preparation method for the compound of formula XV, the acid may be selected from at least one of organic acids or inorganic acids such as hydrochloric acid, hydrobromic acid, hydroiodic acid, phosphoric acid, sulfuric acid, methanesulfonic acid, trifluoromethanesulfonic acid, trimethylsilyl trifluoromethanesulfonate, trifluoroacetic acid, phosphorus oxychloride, succinic acid, and ascorbic acid.

According to an embodiment of the present disclosure, the preparation method for the compound of formula XV may be carried out in the presence of an organic solvent. The organic solvent may be selected from one or a mixture of two or more of the following: ethers (e.g., ethyl propyl ether, methyl *tert*-butyl ether, *n*-butyl ether, anisole, phenetole, cyclohexyl methyl ether, dimethyl ether, diethyl ether, diphenyl ether, dipropyl ether, diisopropyl ether, di-*n*-butyl ether, diisobutyl ether, diisoamyl ether, ethylene glycol dimethyl ether, isopropyl ethyl ether, diethylene glycol dimethyl ether, triethylene glycol dimethyl ether, tetrahydrofuran, 2-methyltetrahydrofuran, 1,4-dioxane and ethylene oxide, and/or propylene oxide polyethers); sulfoxides (e.g., tetrahydrodioxythiophene and dimethyl sulfoxide, tetramethyl sulfoxide, dipropyl sulfoxide, benzyl methyl sulfoxide, diisobutyl sulfoxide, dibutyl sulfoxide, or diisoamyl sulfoxide); sulfones (e.g., dimethyl sulfone, diethyl sulfone, dipropyl sulfone, dibutyl sulfone, diphenyl sulfone, dihexyl sulfone, methyl ethyl sulfone, ethyl propyl sulfone, ethyl isobutyl sulfone, and cyclopentyl sulfone); aliphatic, cycloaliphatic or aromatic hydrocarbons (e.g., pentane, hexane, heptane, octane, nonane, cyclohexane, methylcyclohexane, petroleum ether, naphtha, octane, benzene, toluene, or xylene); haloalkanes (e.g., dichloromethane, chloroform, carbon tetrachloride, dichloroethane, or trichloroethane); halogenated aromatic compounds (e.g., chlorobenzene or dichlorobenzene); amides (e.g., hexamethylphosphoramide, formamide, *N,N-*dimethylacetamide, *N*-methylformamide, *N,N-*dimethylformamide, *N,N-*dipropylformamide, *N,N-*dibutylformamide, *N-*methylpyrrolidine, *N*-methylcaprolactam, 1,3-dimethyl-3,4,5,6-tetrahydro-2(1*H*)-pyrimidine, octylpyrrolidine, octylcaprolactam, 1,3-dimethyl-2-imidazolinedione, *N-*formylpiperidine, or *N,N'*-1,4-diformylpiperazine); nitriles (e.g., acetonitrile, propionitrile, *n*-butyronitrile, isobutyronitrile, or benzonitrile); alcohols (e.g., methanol, ethanol, *n*-propanol, isopropanol, *n*-butanol, *sec-*butanol, or *tert*-butanol); and ketones (e.g., acetone or *N*-methylpyrrolidone). Preferably, the organic solvent is selected from dichloromethane.

According to an embodiment of the present disclosure, in the preparation method for the compound of formula XV, the molar ratio of the compound of formula XIV to trifluoroacetic acid may be 1:1 to 1:10, for example, 1:4 to 1:6, such as 1:5 to 1:5.5.

According to an embodiment of the present disclosure, in the preparation method for the compound of formula XV, trifluoroacetic acid may participate in the reaction by means of dropwise addition.

According to an embodiment of the present disclosure, the preparation method M15 comprises subjecting compound 14 to the following reaction to prepare compound 15:

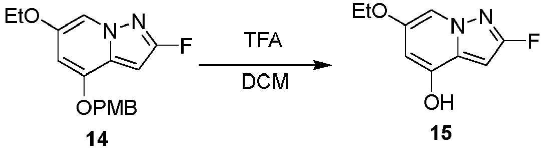

The present disclosure further provides the compound of formula XIV described above, such as compound 14.

The present disclosure further provides use of the compound of formula XIV described above (e.g., compound 14) in the preparation of the compound of formula XV (e.g., compound 15).

The present disclosure further provides a preparation method M14 for the compound of formula XIV, comprising reacting a compound of formula IIIA with ethanol to give the compound of formula XIV: 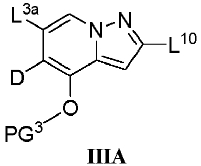 wherein D, L¹⁰, and PG³ are independently defined as described above;
L^{3a} is selected from a leaving group, for example, halogen, such as chlorine, bromine, or iodine.

According to an embodiment of the present disclosure, the preparation method for the compound of formula XIV may be carried out in the presence of a copper catalyst and a ligand.

According to an embodiment of the present disclosure, the copper catalyst may be selected from at least one of copper(I) iodide (CuI), copper(I) bromide (CuBr), and copper(II) acetylacetonate (Cu(acac)₂).

According to an embodiment of the present disclosure, the ligand may be selected from at least one of *N*1,*N*2-bis(4-hydroxy-2,6-dimethylphenyl)oxamide, 8-hydroxyquinoline, *N,N*'-bis(2,4,6-trimethoxyphenyl)oxamide, *N*-benzyl-*N*-(2-methylnaphthalen-1-yl)oxamide, and *N*1,*N*2-bis(4-hydroxy-2,6-dimethylphenyl)oxamide.

According to an embodiment of the present disclosure, the preparation method for the compound of formula XIV may further be carried out in the presence of a base. The base may be an organic base or an inorganic base, for example,
an organic base selected from the following: tertiary amines, substituted or unsubstituted pyridines, and substituted or unsubstituted triethylamine, trimethylamine, *N,N*-diisopropylethylamine, tri-*n*-propylamine, tri-*n*-butylamine, tri-*n*-hexylamine, tricyclohexylamine, *N-*methylcyclohexylamine, *N-*methylpyrrolidine, *N-*methylpiperidine, *N*-ethylpiperidine, *N,N-*dimethylaniline, *N*-methylmorpholine, pyridine, 2,3- or 4-methylpyridine, 2-methyl-5-ethylpyridine, 2,6-dimethylpyridine, 2,4,6-trimethylpyridine, 4-dimethylaminopyridine, quinoline, methylquinoline, *N,N,N,N*-tetramethylethylenediamine, *N,N-*dimethyl-1,4-diazacyclohexane, *N,N*-diethyl-1,4-diazacyclohexane, 1,8-bis(dimethylamino)naphthalene, diazabicyclooctane (DABCO), diazabicyclononane (DBN), diazabicycloundecane (DBU), butylimidazole, methylimidazole, sodium *tert*-butoxide and potassium *tert*-butoxide; or
an inorganic base selected from the following: hydrides, hydroxides, amides, alkoxides, acetates, fluorides, phosphates, carbonates and bicarbonates of alkali metals or alkaline earth metals, such as sodium amide, sodium hydride, lithium diisopropylamide, sodium methoxide, potassium *tert*-butoxide, sodium *tert*-butoxide, sodium hydroxide, potassium hydroxide, sodium acetate, sodium phosphate, potassium phosphate, potassium fluoride, cesium fluoride, sodium carbonate, potassium carbonate, potassium bicarbonate, sodium bicarbonate, and cesium carbonate.

According to an embodiment of the present disclosure, in the preparation method for the compound of formula XIV, the molar volume ratio of the compound of formula IIIA to ethanol may be 1 mol:(2-5) L, such as 1 mol:(3-4) L.

According to an embodiment of the present disclosure, in the preparation method for the compound of formula XIV, the molar ratio of the compound of formula IIIA to the copper(I) catalyst may be 1:0.01 to 1:0.2, such as 1:0.02.

According to an embodiment of the present disclosure, in the preparation method for the compound of formula XIV, the molar ratio of the compound of formula IIIA to the base may be 1:1 to 1:10, for example, 1:2 to 1:4, such as 1:3.

According to an embodiment of the present disclosure, in the preparation method for the compound of formula XIV, the molar ratio of the compound of formula IIIA to the ligand may be 1:0.01 to 1:0.2, such as 1:0.02.

According to an embodiment of the present disclosure, in the preparation method for the compound of formula XIV, the reaction may be carried out at a temperature of not less than 50 °C, for example, not less than 70 °C, such as 80 to 100 °C.

According to an embodiment of the present disclosure, the preparation method M14 comprises subjecting compound 3a to the following reaction to prepare compound 14:

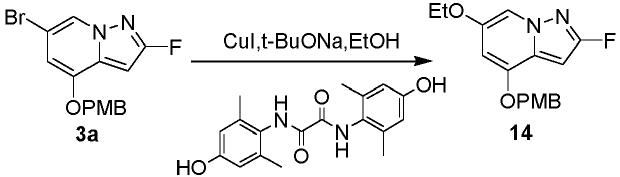

The present disclosure further provides the compound of formula IIIA described above, such as compound 3a.

The present disclosure further provides use of the compound of formula IIIA described above (e.g., compound 3a) in the preparation of the compound of formula XIV (e.g., compound 14).

The present disclosure further provides a preparation method M10C for the compound of formula X, comprising reacting a compound of formula IX with an alkali metal halide or an organic halide salt to give the compound of formula X: 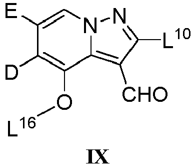 wherein D, E, L¹⁰, and L¹⁶ are independently defined as described above.

According to an embodiment of the present disclosure, in the preparation method for the compound of formula X, the alkali metal halide or the organic halide salt may be selected from, for example, at least one of LiBr, NaBr, KBr, CsBr, and Bu₄NBr.

According to an embodiment of the present disclosure, the preparation method for the compound of formula X may be carried out in the presence of a base. The base may be an organic base or an inorganic base, for example,
an organic base selected from the following: tertiary amines, substituted or unsubstituted pyridines, and substituted or unsubstituted triethylamine, trimethylamine, *N,N-*diisopropylethylamine, tri-*n*-propylamine, tri-*n*-butylamine, tri-*n*-hexylamine, tricyclohexylamine, *N-*methylcyclohexylamine, *N*-methylpyrrolidine, *N*-methylpyrrolidone, *N*-methylpiperidine, *N*-ethylpiperidine, *N,N-*dimethylaniline, *N*-methylmorpholine, pyridine, 2,3-or 4-methylpyridine, 2-methyl-5-ethylpyridine, 2,6-dimethylpyridine, 2,4,6-trimethylpyridine, 4-dimethylaminopyridine, quinoline, methylquinoline, *N,N,N,N*-tetramethylethylenediamine, *N,N-*dimethyl-1,4-diazacyclohexane, *N,N*-diethyl-1,4-diazacyclohexane, 1,8-bis(dimethylamino)naphthalene, diazabicyclooctane (DABCO), diazabicyclononane (DBN), diazabicycloundecane (DBU), butylimidazole, methylimidazole, sodium *tert*-butoxide and potassium *tert*-butoxide; or
an inorganic base selected from the following: hydrides, hydroxides, amides, alkoxides, acetates, fluorides, phosphates, carbonates and bicarbonates of alkali metals or alkaline earth metals, such as sodium amide, sodium hydride, lithium diisopropylamide, sodium methoxide, potassium *tert*-butoxide, sodium hydroxide, potassium hydroxide, sodium acetate, sodium phosphate, potassium phosphate, potassium fluoride, cesium fluoride, sodium carbonate, potassium carbonate, potassium bicarbonate, sodium bicarbonate, and cesium carbonate.

According to an embodiment of the present disclosure, in the preparation method for the compound of formula X, the molar ratio of the compound of formula IX to the alkali metal halide or the organic halide salt may be 1:1 to 1:5, for example, 1:1 to 1:2, such as 1:1.5.

According to an embodiment of the present disclosure, the preparation method for the compound of formula X may be carried out in the presence of a catalyst, wherein the catalyst is, for example, chloro(pentamethylcyclopentadienyl)bis(triphenylphosphine)ruthenium(II), tris(acetonitrile)pentamethylcyclopentadienylruthenium(II) trifluoromethanesulfonate, pentamethylcyclopentadienyltris(acetonitrile)ruthenium(II) hexafluorophosphate, chloro(pentamethylcyclopentadienyl)ruthenium(II) (dimer), chloro(pentamethylcyclopentadienyl)ruthenium(I) (tetramer), tris(acetonitrile)tetramethylcyclopentadienylruthenium(II) trifluoromethanesulfonate, or dichloro(1,5-cyclooctadien)ruthenium(II).

According to an embodiment of the present disclosure, in the preparation method for the compound of formula X, the molar ratio of the compound of formula IX to the catalyst may be 1:0.01 to 1:0.1, such as 0.05.

According to an embodiment of the present disclosure, the reaction in the preparation method for the compound of formula X may be carried out at a temperature of not less than 50 °C, for example, not less than 80 °C, such as 90-100 °C.

According to the preparation method of the present disclosure, the preparation method M10C comprises subjecting compound 9 to the following reaction to prepare compound 10:

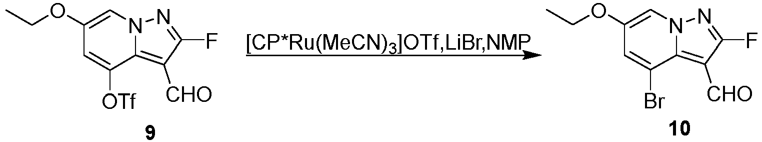

The present disclosure further provides the compound of formula IX described above, such as compound 9.

The present disclosure further provides use of the compound of formula IX described above (e.g., compound 9) in the preparation of the compound of formula X (e.g., compound 10).

According to an embodiment of the present disclosure, the compound of formula IX may also be prepared from the compound of formula IIIA described above as a starting material. For example, the compound of formula IIIA may be reacted with phosphorus oxychloride to give a compound of formula IV shown below: 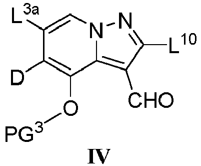 wherein D, L¹⁰, PG³, and L^{3a} are independently defined as described above.

According to an embodiment of the present disclosure, the reaction of the compound of formula IIIA with phosphorus oxychloride may be carried out in the presence of an organic solvent. The organic solvent may be selected from one or a mixture of two or more of the following: ethers (e.g., ethyl propyl ether, methyl *tert-*butyl ether, *n*-butyl ether, anisole, phenetole, cyclohexyl methyl ether, dimethyl ether, diethyl ether, diphenyl ether, dipropyl ether, diisopropyl ether, di-*n*-butyl ether, diisobutyl ether, diisoamyl ether, ethylene glycol dimethyl ether, isopropyl ethyl ether, diethylene glycol dimethyl ether, triethylene glycol dimethyl ether, tetrahydrofuran, 2-methyltetrahydrofuran, 1,4-dioxane and ethylene oxide, and/or propylene oxide polyethers); sulfoxides (e.g., tetrahydrodioxythiophene and dimethyl sulfoxide, tetramethyl sulfoxide, dipropyl sulfoxide, benzyl methyl sulfoxide, diisobutyl sulfoxide, dibutyl sulfoxide, or diisoamyl sulfoxide); sulfones (e.g., dimethyl sulfone, diethyl sulfone, dipropyl sulfone, dibutyl sulfone, diphenyl sulfone, dihexyl sulfone, methyl ethyl sulfone, ethyl propyl sulfone, ethyl isobutyl sulfone, and cyclopentyl sulfone); aliphatic, cycloaliphatic or aromatic hydrocarbons (e.g., pentane, hexane, heptane, octane, nonane, cyclohexane, methylcyclohexane, petroleum ether, naphtha, octane, benzene, toluene, or xylene); haloalkanes (e.g., dichloromethane, chloroform, carbon tetrachloride, dichloroethane, or trichloroethane); halogenated aromatic compounds (e.g., chlorobenzene or dichlorobenzene); amides (e.g., hexamethylphosphoramide, formamide, *N,N-*dimethylacetamide, *N*-methylformamide, *N,N-*dimethylformamide, *N,N-*dipropylformamide, *N,N-*dibutylformamide, *N-*methylpyrrolidine, *N*-methylcaprolactam, 1,3-dimethyl-3,4,5,6-tetrahydro-2(1*H*)-pyrimidine, octylpyrrolidine, octylcaprolactam, 1,3-dimethyl-2-imidazolinedione, *N-*formylpiperidine, or *N,N'*-1,4-diformylpiperazine); nitriles (e.g., acetonitrile, propionitrile, *n*-butyronitrile, isobutyronitrile, or benzonitrile); alcohols (e.g., methanol, ethanol, *n*-propanol, isopropanol, *n*-butanol, *sec-*butanol, or *tert*-butanol); and ketones (e.g., acetone or *N*-methylpyrrolidone). Preferably, the organic solvent is selected from dimethyl sulfoxide or *N,N-*dimethylformamide.

According to an embodiment of the present disclosure, in the preparation method described above, the molar ratio of the compound of formula IIIA to phosphorus oxychloride may be 1:2 to 1:8, for example, 1:3 to 1:6, such as 1:4 to 1:5.

According to an embodiment of the present disclosure, group L^{3a} of the compound of formula IV may be derivatized into group E as defined above according to known organic synthesis methods, for example, L^{3a} may be first converted into a hydroxyl-derived intermediate, and then the hydroxyl-derived intermediate may be further derivatized into group E.

Alternatively, with reference to the preparation method M14 described above, group L^{3a} of the compound of formula IV may be derivatized into group E.

Alternatively, according to an embodiment of the present disclosure, with reference to the preparation method M15 described above, the compound of formula IV or the derivative thereof described above may be reacted with trifluoroacetic acid to derivatize group PG³ into hydroxyl; and/or with reference to the preparation method M16 described above, the derivative described above may be reacted with the sulfonylation reagent described above to give a derivative in which the hydrogen atom on hydroxyl is substituted with C₁₋₆ alkylsulfonyl unsubstituted or substituted with 1, 2, 3, 4, 5 or 6 halogens (such as methanesulfonyl (Ms) or trifluoromethanesulfonyl).

As an example, the compound of formula IV may be reacted with trifluoroacetic acid to give a compound of formula XXIII shown below: 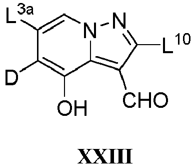 wherein D, L¹⁰, and L^{3a} are independently defined as described above.

According to an embodiment of the present disclosure, with reference to the preparation method M11 described above, the compound of formula XXIII may be reacted with hydrazine (e.g., hydrazine hydrate) to give a compound of formula XXXI shown below: 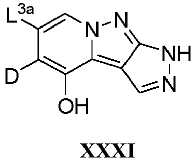 wherein D and L^{3a} are independently defined as described above.

According to an embodiment of the present disclosure, group L^{3a} of the compound of formula XXXI may be derivatized into group E as defined above according to known organic synthesis methods, for example, L^{3a} may be first converted into a hydroxyl-derived intermediate, and then the hydroxyl-derived intermediate may be further derivatized into group E, so as to give a compound of formula XXIX:

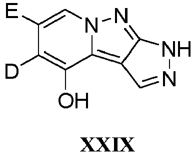

Alternatively, with reference to the preparation method M14 described above, group L^{3a} of the compound of formula XXXI may be derivatized into group E, so as to give the compound of formula XXIX.

According to an embodiment of the present disclosure, with reference also to the preparation method M16 described above, the compound of formula XXIX described above may be reacted with the sulfonylation reagent described above to give a compound of formula XXX in which the hydrogen atom on hydroxyl is substituted with the following group unsubstituted or substituted with 1, 2, 3, 4, 5 or 6 halogens: C₁₋₆ alkylsulfonyl or C₁₋₆ alkylbenzenesulfonyl, such as methanesulfonyl (Ms-), trifluoromethanesulfonyl (Tf-) or *p*-toluenesulfonyl (Ts-): 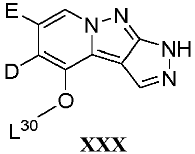 wherein D and E are independently defined as described above;
L³⁰ is the following group unsubstituted or substituted with 1, 2, 3, 4, 5 or 6 halogens: C₁₋₆ alkylsulfonyl or C₁₋₆ alkylbenzenesulfonyl, such as methanesulfonyl (Ms-), trifluoromethanesulfonyl (Tf-) or *p*-toluenesulfonyl (Ts-).

According to an embodiment of the present disclosure, compound 3a may be subjected to the following reaction to prepare compound 4, compound 5, compound 6, compound 7, compound 8, and/or compound 9:

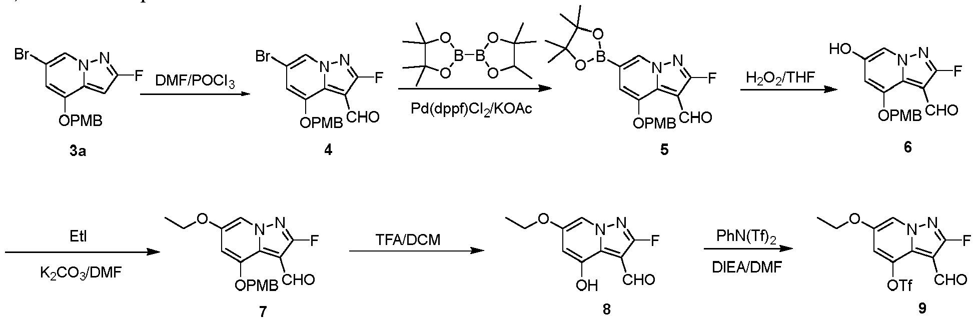

According to an embodiment of the present disclosure, compound 3a may also be subjected to the following reaction to prepare compound 4, compound 23, compound 31, compound 29, and/or compound 30:

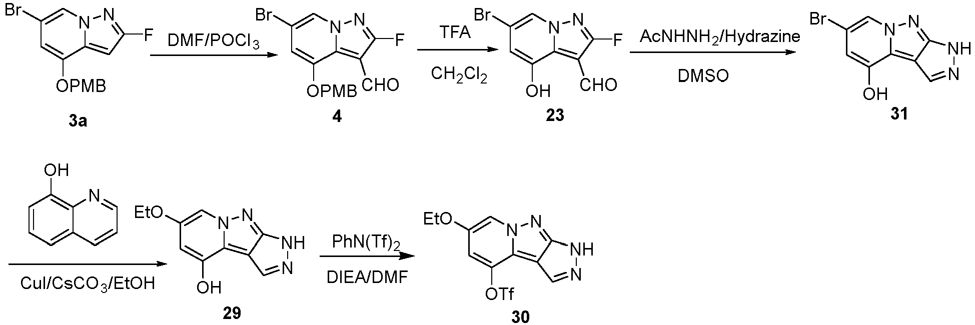

According to an embodiment of the present disclosure, compound 4 may also be subjected to the following reaction to prepare compound 23, compound 31, compound 25, compound 26, compound 27, compound 28, compound 29, and/or compound 30:

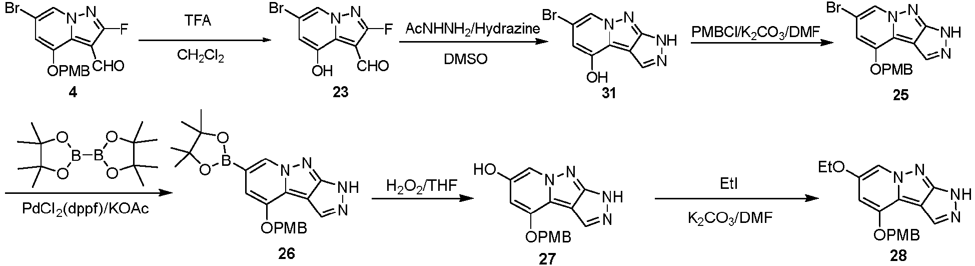

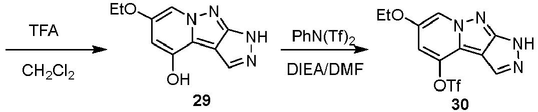

The present disclosure further provides the compound of formula IV (e.g., compound 4), the compound of formula XXIII (e.g., compound 23), the compound of formula XXXI (e.g., compound 31), the compound of formula XXIX (e.g., compound 29) and the compound of formula XXX (e.g., compound 30) described above, and use of any one of the compounds described above in the preparation of the compound of formula I.

According to an embodiment of the present disclosure, further provided is a preparation method M19B for the compound of formula XIX, comprising reacting the compound of formula XXX with the compound of formula XVIII to give the compound of formula XIX, wherein the compound of formula XVIII and the compound of formula XIX are defined as described above. According to an embodiment of the present disclosure, the preparation method for the compound of formula XIX described above may be carried out in the presence of a base.

The base may be an organic base or an inorganic base, for example,
an organic base selected from the following: tertiary amines, substituted or unsubstituted pyridines, and substituted or unsubstituted triethylamine, trimethylamine, *N,N-*diisopropylethylamine, tri-*n*-propylamine, tri-*n*-butylamine, tri-*n*-hexylamine, tricyclohexylamine, *N*-methylcyclohexylamine, *N*-methylpyrrolidine, *N*-methylpyrrolidone, *N*-methylpiperidine, *N*-ethylpiperidine, *N,N-*dimethylaniline, *N*-methylmorpholine, pyridine, 2,3-or 4-methylpyridine, 2-methyl-5-ethylpyridine, 2,6-dimethylpyridine, 2,4,6-trimethylpyridine, 4-dimethylaminopyridine, quinoline, methylquinoline, *N,N,N,N*-tetramethylethylenediamine, *N,N*-dimethyl-1,4-diazacyclohexane, *N,N-*diethyl-1,4-diazacyclohexane, 1,8-bis(dimethylamino)naphthalene, diazabicyclooctane (DABCO), diazabicyclononane (DBN), diazabicycloundecane (DBU), butylimidazole, methylimidazole, sodium *tert*-butoxide and potassium *tert*-butoxide; or
an inorganic base selected from the following: hydrides, hydroxides, amides, alkoxides, acetates, fluorides, phosphates, carbonates and bicarbonates of alkali metals or alkaline earth metals, such as sodium amide, sodium hydride, lithium diisopropylamide, sodium methoxide, potassium *tert*-butoxide, sodium hydroxide, potassium hydroxide, sodium acetate, sodium phosphate, potassium phosphate, potassium fluoride, cesium fluoride, sodium carbonate, potassium carbonate, potassium bicarbonate, sodium bicarbonate, and cesium carbonate.

According to an embodiment of the present disclosure, the preparation method for the compound of formula XIX described above may be carried out in the presence of an organic solvent or a mixed solvent of an organic solvent and water. The organic solvent may be selected from one or a mixture of two or more of the following: ethers (e.g., ethyl propyl ether, methyl *tert*-butyl ether, *n*-butyl ether, anisole, phenetole, cyclohexyl methyl ether, dimethyl ether, diethyl ether, diphenyl ether, dipropyl ether, diisopropyl ether, di-*n*-butyl ether, diisobutyl ether, diisoamyl ether, ethylene glycol dimethyl ether, isopropyl ethyl ether, diethylene glycol dimethyl ether, triethylene glycol dimethyl ether, tetrahydrofuran, 2-methyltetrahydrofuran, 1,4-dioxane and ethylene oxide, and/or propylene oxide polyethers); sulfoxides (e.g., tetrahydrodioxythiophene and dimethyl sulfoxide, tetramethyl sulfoxide, dipropyl sulfoxide, benzyl methyl sulfoxide, diisobutyl sulfoxide, dibutyl sulfoxide, or diisoamyl sulfoxide); sulfones (e.g., dimethyl sulfone, diethyl sulfone, dipropyl sulfone, dibutyl sulfone, diphenyl sulfone, dihexyl sulfone, methyl ethyl sulfone, ethyl propyl sulfone, ethyl isobutyl sulfone, and cyclopentyl sulfone); aliphatic, cycloaliphatic or aromatic hydrocarbons (e.g., pentane, hexane, heptane, octane, nonane, cyclohexane, methylcyclohexane, petroleum ether, naphtha, octane, benzene, toluene, or xylene); haloalkanes (e.g., dichloromethane, chloroform, carbon tetrachloride, dichloroethane, or trichloroethane); halogenated aromatic compounds (e.g., chlorobenzene or dichlorobenzene); amides (e.g., hexamethylphosphoramide, formamide, *N,N-*dimethylacetamide, *N*-methylformamide, *N,N-*dimethylformamide, *N,N-*dipropylformamide, *N,N-*dibutylformamide, *N*-methylpyrrolidine, *N*-methylcaprolactam, 1,3-dimethyl-3,4,5,6-tetrahydro-2(1*H*)-pyrimidine, octylpyrrolidine, octylcaprolactam, 1,3-dimethyl-2-imidazolinedione, *N-*formylpiperidine, or *N,N'*-1,4-diformylpiperazine); nitriles (e.g., acetonitrile, propionitrile, *n*-butyronitrile, isobutyronitrile, or benzonitrile); alcohols (e.g., methanol, ethanol, *n*-propanol, isopropanol, *n*-butanol, *sec*-butanol, or *tert*-butanol); and ketones (e.g., acetone or *N*-methylpyrrolidone). Preferably, the organic solvent is dioxane.

According to an embodiment of the present disclosure, the preparation method for the compound of formula XIX described above may be carried out in the presence of a catalyst, wherein the catalyst may be a palladium catalyst, such as Pd(dba)₂, PdCl₂, Pd(OAc)₂, Pd(dppf)Cl₂, Pd₂(dba)₃, PdCl₂(CH₃CN)₂, Pd(PPh₃)₄, PdCl₂(PPh₃)₂, Pd(t-Bu)₃, PdCl₂(PPh₃)₂•CH₂Cl₂, Pd(OAc)/PPh₃, PdCl₂[(Pet₃)]₂, Pd(DIPHOS)₂, PdCl₂(Bipy), [PdCl(Ph₂PCH₂PPh₂)]₂, PdCl₂[P(o-Tol)₃]₂, Pd₂(dba)₃/P(o-Tol)₃, Pd₂(dba)/P(furanyl)₃, PdCl₂[P(furanyl)₃]₂, PdCl₂(PMePh₂)₂, PdCl₂[P(4-F-Ph)₃]₂, PdCl₂[P(C₆F₆)₃]₂, PdCl₂[P(2-COOH-Ph)(Ph)₂]₂, and PdCl₂[P(4-COOH-Ph)(Ph)₂]₂.

According to an embodiment of the present disclosure, the reaction in the preparation method for the compound of formula XIX described above may be carried out at a temperature of not less than 80 °C, for example, not less than 100 °C, such as 120 °C.

According to an embodiment of the present disclosure, in the preparation method for the compound of formula XIX described above, the molar ratio of the compound of formula XXX to the compound of formula XVIII may be 1:1 to 1:5, for example, 1:2 to 1:4, such as 1:3. According to an embodiment of the present disclosure, in the preparation method for the compound of formula XIX described above, the molar ratio of the compound of formula XXX to the base may be 1:1 to 1:10, for example, 1:4 to 1:8, such as 1:5 to 1:6.

According to an embodiment of the present disclosure, in the preparation method for the compound of formula XIX described above, the molar ratio of the compound of formula XXX to the catalyst may be 1:0.01 to 1:0.2, such as 1:0.05 to 1:0.1.

The present disclosure further provides a preparation method M1B for the compound of formula I, wherein the preparation method comprises reacting the compound of formula XXX with a compound of formula XXXX to give the compound of formula I: 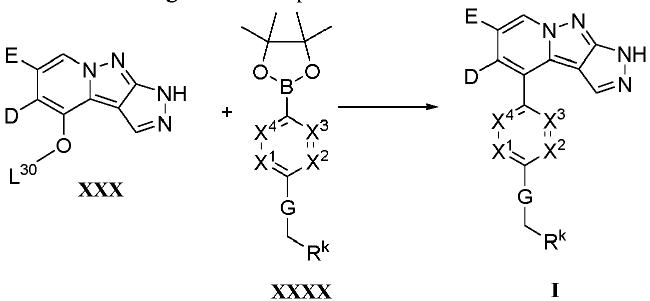 wherein D, E, L³⁰, X¹, X², X³, X⁴, G, and R^{k} are each independently defined as described above.

According to an embodiment of the present disclosure, the reaction in the preparation method M1B described above may be carried out in the presence of a catalyst, wherein the catalyst may be a palladium catalyst, such as Pd(dba)₂, PdCl₂, Pd(OAc)₂, Pd(dppf)Cl₂, Pd₂(dba)₃, PdCl₂(CH₃CN)₂, Pd(PPh₃)₄, PdCl₂(PPh₃)₂, Pd(t-Bu)₃, PdCl₂(PPh₃)₂•CH₂Cl₂, Pd(OAc)/PPh₃, PdCl₂[(Pet₃)]₂, Pd(DIPHOS)₂, PdCl₂(Bipy), [PdCl(Ph₂PCH₂PPh₂)]₂, PdCl₂[P(o-Tol)₃]₂, Pd₂(dba)₃/P(o-Tol)₃, Pd₂(dba)/P(furanyl)₃, PdCl₂[P(furanyl)₃]₂, PdCl₂(PMePh₂)₂, PdCl₂[P(4-F-Ph)₃]₂, PdCl₂[P(C₆F₆)₃]₂, PdCl₂[P(2-COOH-Ph)(Ph)₂]₂, and PdCl₂[P(4-COOH-Ph)(Ph)₂]₂.

According to an embodiment of the present disclosure, the reaction in the preparation method M1B described above may be carried out in the presence of a base.

The base may be an organic base or an inorganic base, for example,
an organic base selected from the following: tertiary amines, substituted or unsubstituted pyridines, and substituted or unsubstituted triethylamine, trimethylamine, *N,N*-diisopropylethylamine, tri-*n*-propylamine, tri-*n*-butylamine, tri-*n*-hexylamine, tricyclohexylamine, *N*-methylcyclohexylamine, *N*-methylpyrrolidine, *N*-methylpyrrolidone, *N*-methylpiperidine, *N*-ethylpiperidine, *N,N-*dimethylaniline, *N*-methylmorpholine, pyridine, 2,3- or 4-methylpyridine, 2-methyl-5-ethylpyridine, 2,6-dimethylpyridine, 2,4,6-trimethylpyridine, 4-dimethylaminopyridine, quinoline, methylquinoline, *N,N,N,N*-tetramethylethylenediamine, *N,N-*dimethyl-1,4-diazacyclohexane, *N,N-*diethyl-1,4-diazacyclohexane, 1,8-bis(dimethylamino)naphthalene, diazabicyclooctane (DABCO), diazabicyclononane (DBN), diazabicycloundecane (DBU), butylimidazole, methylimidazole, sodium *tert-*butoxide and potassium *tert-*butoxide; or
an inorganic base selected from the following: hydrides, hydroxides, amides, alkoxides, acetates, fluorides, phosphates, carbonates and bicarbonates of alkali metals or alkaline earth metals, such as sodium amide, sodium hydride, lithium diisopropylamide, sodium methoxide, potassium *tert-*butoxide, sodium hydroxide, potassium hydroxide, sodium acetate, sodium phosphate, potassium phosphate, potassium fluoride, cesium fluoride, sodium carbonate, potassium carbonate, potassium bicarbonate, sodium bicarbonate, and cesium carbonate.

According to an embodiment of the present disclosure, the reaction in the preparation method M1B described above may be carried out in the presence of an organic solvent. The organic solvent may be selected from one or a mixture of two or more of the following: ethers (e.g., ethyl propyl ether, methyl *tert*-butyl ether, *n*-butyl ether, anisole, phenetole, cyclohexyl methyl ether, dimethyl ether, diethyl ether, diphenyl ether, dipropyl ether, diisopropyl ether, di-*n*-butyl ether, diisobutyl ether, diisoamyl ether, ethylene glycol dimethyl ether, isopropyl ethyl ether, diethylene glycol dimethyl ether, triethylene glycol dimethyl ether, tetrahydrofuran, 2-methyltetrahydrofuran, 1,4-dioxane and ethylene oxide, and/or propylene oxide polyethers); sulfoxides (e.g., tetrahydrodioxythiophene and dimethyl sulfoxide, tetramethyl sulfoxide, dipropyl sulfoxide, benzyl methyl sulfoxide, diisobutyl sulfoxide, dibutyl sulfoxide, or diisoamyl sulfoxide); sulfones (e.g., dimethyl sulfone, diethyl sulfone, dipropyl sulfone, dibutyl sulfone, diphenyl sulfone, dihexyl sulfone, methyl ethyl sulfone, ethyl propyl sulfone, ethyl isobutyl sulfone, and cyclopentyl sulfone); aliphatic, cycloaliphatic or aromatic hydrocarbons (e.g., pentane, hexane, heptane, octane, nonane, cyclohexane, methylcyclohexane, petroleum ether, naphtha, octane, benzene, toluene, or xylene); haloalkanes (e.g., dichloromethane, chloroform, carbon tetrachloride, dichloroethane, or trichloroethane); halogenated aromatic compounds (e.g., chlorobenzene or dichlorobenzene); amides (e.g., hexamethylphosphoramide, formamide, *N,N-*dimethylacetamide, *N*-methylformamide, *N,N-*dimethylformamide, *N,N-*dipropylformamide, *N,N-*dibutylformamide, *N*-methylpyrrolidine, *N*-methylcaprolactam, 1,3-dimethyl-3,4,5,6-tetrahydro-2(1*H*)-pyrimidine, octylpyrrolidine, octylcaprolactam, 1,3-dimethyl-2-imidazolinedione, *N*-formylpiperidine, or *N,N*'*-*1,4-diformylpiperazine); nitriles (e.g., acetonitrile, propionitrile, *n*-butyronitrile, isobutyronitrile, or benzonitrile); alcohols (e.g., methanol, ethanol, *n*-propanol, isopropanol, *n*-butanol, *sec-*butanol, or *tert*-butanol); and ketones (e.g., acetone or *N*-methylpyrrolidone). Preferably, the organic solvent is dioxane.

According to an embodiment of the present disclosure, the reaction in the preparation method M1B may be carried out at 100 °C to 150 °C, for example, 115 °C to 125 °C.

According to an embodiment of the present disclosure, the preparation method M1B comprises subjecting compound 30 and compound 40 to the following reaction to prepare compound 1:

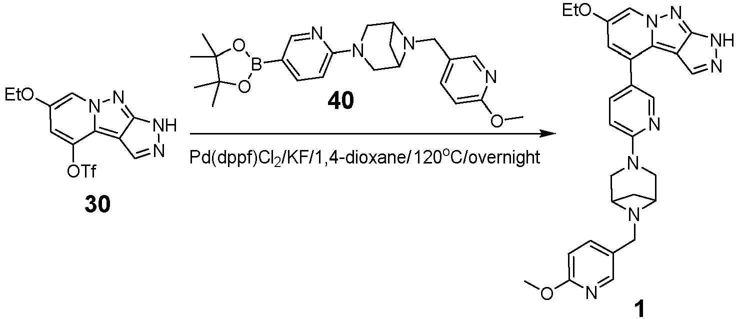

The present disclosure further provides use of the compound of formula XXX described above in the preparation of the compound of formula XIX.

The present disclosure further provides a method for preparing the compound of formula I, a pharmaceutically acceptable salt thereof, or the intermediate described above, comprising the preparation methods taking the compound of formula IIIA and/or the compound of formula IIIB as starting materials or the reactions carried out therein described above.

The present disclosure further provides use of the compound of formula IIIA in the preparation of the compound of formula I, the pharmaceutically acceptable salt thereof, or the intermediate described above.

According to a preferred embodiment of the present disclosure, any one of the preparation methods or uses described above may be carried out in an inert atmosphere (e.g., nitrogen atmosphere).

According to a preferred embodiment of the present disclosure, in any one of the preparation methods or uses described above, the compound as the starting material may be provided and participate in the reaction in the form of an acid addition salt thereof, as required.

According to a preferred embodiment of the present disclosure, in any one of the preparation methods or uses described above, where the compound as the starting material is provided and participates in the reaction in the form of an acid addition salt, the acid addition salt may be freed, if desired, with the base (organic base or inorganic base) as defined above before participating in the reaction, or the freeing step may be carried out *in situ* with the subsequent reaction by means of a "one-pot process".

The present disclosure further provides a preparation method, comprising combining one, two, three or more of the preparation methods described above as a sequential process to produce a target compound. It should be understood by those skilled in the art that where one, two, three or more of the preparation methods described above are combined as a sequential process, it is preferred to react the product resulting from one preparation method as a starting material for the next preparation method.

The present disclosure further provides a preparation method M-IIIB-I, comprising the preparation methods M13, M10A, M11, M19A, M20 and M1A described above, or being selected from a combination of two, three or more of the preparation methods described above. Preferably, the preparation methods in the combination are carried out sequentially, so that a product resulting from the preparation method carried out previously is used as a reaction substrate for the preparation method carried out subsequently.

The present disclosure further provides a preparation method M-IIIA-X1, comprising the preparation methods M14, M15, M16, M17 and M10B described above, or being selected from a combination of two, three or more of the preparation methods described above. Preferably, the preparation methods in the combination are carried out sequentially, so that a product resulting from the preparation method carried out previously is used as a reaction substrate for the preparation method carried out subsequently.

The present disclosure further provides a preparation method M-IIIA-X2, comprising preparing the compound of formula IV, the compound of formula V, the compound of formula VI, the compound of formula VII, the compound of formula VIII, and the compound of formula IX by the preparation methods described above, and preparing the compound of formula X by the preparation method M10C.

The present disclosure further provides a preparation method M-IIIA-XXX, comprising preparing the compound of formula XXIII, the compound of formula XXXI, the compound of formula XXIX and the compound of formula XXX by the methods described above.

According to an embodiment of the present disclosure, it will be understood that one of the different preparation methods for preparing the same intermediate may be combined with a preparation method in which the intermediate is used as a reaction substrate to further prepare another compound, and such combination should likewise be understood as the content described herein, and should not be limited to the particular reaction route resulting from the combination of the preparation methods described above. For example, as described above, the preparation method for the compound of formula X includes M10A, M10B, and M10C, any one of which may be combined with the preparation method M19A, or further combined with M20 and/or M1. Similarly, all of the different preparation methods for the compound of formula XXX described above may be combined with the preparation method M1B for the preparation of the compound of formula I.

According to an embodiment of the present disclosure, in any one of the preparation methods or uses described above, it is preferred that the starting material, reaction product or intermediate is not separated or purified using chromatography such as column chromatography.

According to an embodiment of the present disclosure, in any one of the preparation methods or uses described above, it is preferred that the reaction is carried out without using hydrogen as a hydrogenation or reduction reagent, and without using hydrogen by means of catalytic hydrogenation.

According to an embodiment of the present disclosure, in any one of the preparation methods or uses described above, where an organic solvent or a mixed solvent of an organic solvent and water is used, the organic solvent or the mixed solvent is inert to the reaction substrate and the reagent.

### Definitions and Description

Unless otherwise stated, the definitions of groups and terms described in the specification and claims of the present application, including definitions thereof as examples, exemplary definitions, preferred definitions, definitions documented in tables, definitions of specific compounds in the examples, and the like, may be arbitrarily combined and incorporated with each other. The definitions of groups and the structures of the compounds in such combinations and incorporations should fall within the scope of the present specification.

Unless otherwise stated, the numerical ranges described in the specification and claims shall be construed as at least including each specific integer value therein. For example, the numerical range "1-40" shall be construed as including each integer value in the numerical range "1-10", i.e., 1, 2, 3, 4, 5, 6, 7, 8, 9, and 10, and each integer value in the numerical range "11-40", i.e., 11, 12, 13, 14, 15,..., 35, 36, 37, 38, 39, and 40. It should be understood that where one, two or more are used to describe a substituent herein, "more" shall refer to an integer ≥ 3, such as 3, 4, 5, 6, 7, 8, 9, or 10.

The term "halogen" denotes a substituent group selected from fluorine, chlorine, bromine or iodine.

The term "C₁₋₄₀ alkyl" preferably refers to a linear or branched saturated monovalent hydrocarbyl group having 1-40 carbon atoms. For example, "C₁₋₆ alkyl" refers to a linear or branched alkyl group having 1, 2, 3, 4, 5, or 6 carbon atoms. The alkyl is, for example, methyl, ethyl, propyl, butyl, pentyl, hexyl, isopropyl, isobutyl, *sec*-butyl, *tert*-butyl, isopentyl, 2-methylbutyl, 1-methylbutyl, 1-ethylpropyl, 1,2-dimethylpropyl, neopentyl, 1,1-dimethylpropyl, 4-methylpentyl, 3-methylpentyl, 2-methylpentyl, 1-methylpentyl, 2-ethylbutyl, 1-ethylbutyl, 3,3-dimethylbutyl, 2,2-dimethylbutyl, 1,1-dimethylbutyl, 2,3-dimethylbutyl, 1,3-dimethylbutyl, 1,2-dimethylbutyl, etc., or isomers thereof.

The term "C₂₋₄₀ alkenyl" preferably refers to a linear or branched monovalent hydrocarbyl comprising one or more double bonds and having 2-40 carbon atoms, and is preferably "C₂₋₆ alkenyl". "C₂₋₆ alkenyl" preferably refers to a linear or branched monovalent hydrocarbyl group comprising one or more double bonds and having 2, 3, 4, 5, or 6 carbon atoms, in particular, 2 or 3 carbon atoms ("C₂₋₃ alkenyl"); it should be understood that in the case where the alkenyl comprises more than one double bond, the double bonds can be separated from one another or conjugated. The alkenyl is, for example, ethenyl, allyl, (*E*)-2-methylethenyl, (*Z*)-2-methylethenyl, (*E*)-but-2-enyl, (*Z*)-but-2-enyl, (*E*)-but-1-enyl, (*Z*)-but-1-enyl, pent-4-enyl, (*E*)-pent-3-enyl, (*Z*)-pent-3-enyl, (*E*)-pent-2-enyl, (*Z*)-pent-2-enyl, (*E*)-pent-1-enyl, (*Z*)-pent-1-enyl, hex-5-enyl, (*E*)-hex-4-enyl, (*Z*)-hex-4-enyl, (*E*)-hex-3-enyl, (*Z*)-hex-3-enyl, (*E*)-hex-2-enyl, (*Z*)-hex-2-enyl, (*E*)-hex-1-enyl, (*Z*)-hex-1-enyl, isopropenyl, 2-methylprop-2-enyl, 1-methylprop-2-enyl, 2-methylprop-1-enyl, (*E*)-1-methylprop-1-enyl, (*Z*)-1-methylprop-1-enyl, 3-methylbut-3-enyl, 2-methylbut-3-enyl, 1-methylbut-3-enyl, 3-methylbut-2-enyl, (*E*)-2-methylbut-2-enyl, (*Z*)-2-methylbut-2-enyl, (*E*)-1-methylbut-2-enyl, (*Z*)-1-methylbut-2-enyl, (*E*)-3-methylbut-1-enyl, (*Z*)-3-methylbut-1-enyl, (*E*)-2-methylbut-1-enyl, (*Z*)-2-methylbut-1-enyl, (*E*)-1-methylbut-1-enyl, (*Z*)-1-methylbut-1-enyl, 1,1-dimethylprop-2-enyl, 1-ethylprop-1-enyl, 1-propylvinyl, or 1-isopropylvinyl.

The term "C₂-₄₀ alkynyl" refers to a linear or branched monovalent hydrocarbyl group comprising one or more triple bonds and having 2-40 carbon atoms, and is preferably "C₂-C₆ alkynyl". The term "C₂-C₆ alkynyl" refers to a linear or branched monovalent hydrocarbyl group comprising one or more triple bonds and having 2, 3, 4, 5, or 6 carbon atoms, in particular 2 or 3 carbon atoms ("C₂-C₃ alkynyl"). The C₂-C₆ alkynyl is, for example, ethynyl, prop-1-ynyl, prop-2-ynyl, but-1-ynyl, but-2-ynyl, but-3-ynyl, pent-1-ynyl, pent-2-ynyl, pent-3-ynyl, pent-4-ynyl, hex-1-ynyl, hex-2-ynyl, hex-3-ynyl, hex-4-ynyl, hex-5-ynyl, 1-methylprop-2-ynyl, 2-methylbut-3-ynyl, 1-methylbut-3-ynyl, 1-methylbut-2-ynyl, 3-methylbut-1-ynyl, 1-ethylprop-2-ynyl, 3-methylpent-4-ynyl, 2-methylpent-4-ynyl, 1-methylpent-4-ynyl, 2-methylpent-3-ynyl, 1-methylpent-3-ynyl, 4-methylpent-2-ynyl, 1-methylpent-2-ynyl, 4-methylpent-1-ynyl, 3-methylpent-1-ynyl, 2-ethylbut-3-ynyl, 1-ethylbut-3-ynyl, 1-ethylbut-2-ynyl, 1-propylprop-2-ynyl, 1-isopropylprop-2-ynyl, 2,2-dimethylbut-3-ynyl, 1,1-dimethylbut-3-ynyl, 1,1-dimethylbut-2-ynyl or 3,3-dimethylbut-1-ynyl. In particular, the alkynyl is ethynyl, prop-1-ynyl, or prop-2-ynyl.

The term "C₃₋₄₀ cycloalkyl" refers to a saturated monovalent monocyclic or bicyclic hydrocarbon ring or bridged cycloalkane having 3-40 carbon atoms, preferably "C₃₋₁₀ cycloalkyl". The term "C₃₋₁₀ cycloalkyl" refers to a saturated monovalent monocyclic or bicyclic hydrocarbon ring or bridged cycloalkane having 3, 4, 5, 6, 7, 8, 9, or 10 carbon atoms. The C₃₋₁₀ cycloalkyl may be a monocyclic hydrocarbyl such as cyclopropyl, cyclobutyl, cyclopentyl, cyclohexyl, cycloheptyl, cyclooctyl, cyclononyl, or cyclodecyl, or may be a bicyclic hydrocarbyl such as a decahydronaphthalene ring.

The term "3- to 20-membered heterocyclyl" refers to a saturated monovalent monocyclic or bicyclic hydrocarbon ring or bridged cycloalkane, which is a non-aromatic cyclic group with the total number of ring atoms of 3-20 (such as 3, 4, 5, 6, 7, 8, 9, and 10) comprising 1-5 heteroatoms independently selected from N, O, and S, preferably a "3- to 10-membered heterocyclyl". The term "3- to 10-membered heterocyclyl" refers to a saturated monovalent monocyclic or bicyclic hydrocarbon ring or bridged cycloalkane, which comprises 1-5, preferably 1-3 heteroatoms independently selected from N, O, and S, for example, 1, 2, or 3 heteroatoms independently selected from N, O, and S. The heterocyclyl may be connected to the rest of the molecule through any one of the carbon atoms or the nitrogen atom (if present). In particular, the heterocyclyl may include, but is not limited to: 4-membered rings such as azetidinyl and oxetanyl; 5-membered rings such as tetrahydrofuranyl, dioxolyl, pyrrolidinyl, imidazolidinyl, pyrazolidinyl, and pyrrolinyl; 6-membered rings such as tetrahydropyranyl, piperidyl, morpholinyl, dithianyl, thiomorpholinyl, piperazinyl, and trithianyl; or 7-membered rings such as diazepanyl. Optionally, the heterocyclyl may be benzo-fused. The heterocyclyl may be bicyclic, for example, but not limited to, a 5,5-membered ring such as a hexahydrocyclopenta[c]pyrrol-2(1*H*)-yl ring, or a 5,6-membered bicyclic ring such as a hexahydropyrrolo[1,2-a]pyrazin-2(1*H*)-yl ring. The ring containing nitrogen atoms may be partially unsaturated, i.e., it may comprise one or more double bonds, for example, but not limited to, 2,5-dihydro-1*H*-pyrrolyl, 4*H*-[1,3,4]thiadiazinyl, 4,5-dihydrooxazolyl or 4*H*-[1,4]thiazinyl, or it may be benzo-fused, for example, but not limited to, dihydroisoquinolyl. According to the present disclosure, the heterocyclyl is non-aromatic. Where the 3- to 20-membered heterocyclyl is connected to another group to form the compound of the present disclosure, the group may be connected to the carbon atom on the 3- to 20-membered heterocyclyl, or may be connected to the heteroatom on the 3- to 20-membered heterocyclyl. For example, where the 3- to 20-membered heterocyclyl is selected from piperazinyl, the group may be connected to the nitrogen atom on the piperazinyl. Alternatively, where the 3- to 20-membered heterocyclyl is selected from piperidyl, the group may be connected to the nitrogen atom on the piperidyl ring or the carbon atom in the para position.

The term "C₆₋₂₀ aryl" preferably refers to an aromatic or partially aromatic monovalent monocyclic, bicyclic, or tricyclic hydrocarbon ring having 6-20 carbon atoms, preferably "C₆₋₁₄ aryl". The term "C₆₋₁₄ aryl" preferably refers to an aromatic or partially aromatic monovalent monocyclic, bicyclic, or tricyclic hydrocarbon ring having 6, 7, 8, 9, 10, 11, 12, 13, or 14 carbon atoms ("C₆₋₁₄ aryl"), in particular a ring having 6 carbon atoms ("C₆ aryl"), such as phenyl or biphenyl, a ring having 9 carbon atoms ("C₉ aryl"), such as indanyl or indenyl, a ring having 10 carbon atoms ("C₁₀ aryl"), such as tetrahydronaphthyl, dihydronaphthyl or naphthyl, a ring having 13 carbon atoms ("C₁₃ aryl"), such as fluorenyl, or a ring having 14 carbon atoms ("C₁₄ aryl"), such as anthryl. Where the C₆₋₂₀ aryl is substituted, it may be monosubstituted or polysubstituted. In addition, the substitution site is not limited, and may be, for example, ortho-substitution, para-substitution, or meta-substitution.

The term "5- to 20-membered heteroaryl" refers to a monovalent monocyclic, bicyclic, or tricyclic aromatic ring system which has 5-20 ring atoms and comprises 1-5 heteroatoms independently selected from N, O, and S, such as "5- to 14-membered heteroaryl". The term "5-to 14-membered heteroaryl" should be understood to refer to a monovalent monocyclic, bicyclic, or tricyclic aromatic ring system which has 5, 6, 7, 8, 9, 10, 11, 12, 13, or 14 ring atoms, in particular 5, 6, 9, or 10 carbon atoms, contains 1-5, preferably 1-3 heteroatoms independently selected from N, O, and S, and may be benzo-fused in each case. In particular, the heteroaryl is selected from thienyl, furanyl, pyrrolyl, oxazolyl, thiazolyl, imidazolyl, pyrazolyl, isoxazolyl, isothiazolyl, oxadiazolyl, triazolyl, thiadiazolyl, thia-4*H*-pyrazolyl and the like and benzo derivatives thereof, such as benzofuranyl, benzothienyl, benzoxazolyl, benzoisoxazolyl, benzimidazolyl, benzotriazolyl, indazolyl, indolyl, and isoindolyl; or pyridinyl, pyridazinyl, pyrimidinyl, pyrazinyl, triazinyl and the like and benzo derivatives thereof, such as quinolyl, quinazolinyl, and isoquinolyl; or azocinyl, indolizinyl, purinyl and the like and benzo derivatives thereof; or cinnolinyl, phthalazinyl, quinazolinyl, quinoxalinyl, naphthyridinyl, pteridinyl, carbazolyl, acridinyl, phenazinyl, phenothiazinyl, phenoxazinyl, and the like. Where the 5- to 20-membered heteroaryl is connected to another group to form the compound of the present disclosure, the group may be connected to the carbon atom on the 5- to 20-membered heteroaryl ring, or may be connected to the heteroatom on the 5- to 20-membered heteroaryl ring. Where the 5- to 20-membered heteroaryl is substituted, it may be monosubstituted or polysubstituted. In addition, the substitution site is not limited. For example, hydrogen connected to the carbon atom on the heteroaryl ring may be substituted, or hydrogen connected to the heteroatom on the heteroaryl ring may be substituted.

Unless otherwise stated, the heterocyclyl, heteroaryl, or heteroarylene includes all possible isomeric forms thereof, e.g., position isomers thereof. Thus, for some illustrative non-limiting examples, forms that involving substitutions at or bonding to other groups at one, two or more of positions 1, 2, 3, 4, 5, 6, 7, 8, 9, 10, 11, 12, and the like (if present) are included, including pyridin-2-yl, pyridinylene-2-yl, pyridin-3-yl, pyridinylene-3-yl, pyridin-4-yl and pyridinylene-4-yl; thienyl or thienylene, including thien-2-yl, thien-2-ylene, thien-3-yl, and thien-3-ylene; pyrazol-1-yl, pyrazol-3-yl, pyrazol-4-yl and pyrazol-5-yl.

The term "oxo" refers to that the carbon atom, nitrogen atom, or sulfur atom in the substituent is substituted with an oxy group formed after oxidation (=O).

Unless otherwise stated, the definitions of terms used herein are also applicable to groups comprising the terms. For example, the definition of C₁₋₆ alkyl is also applicable to C₁₋₆ alkyloxy, -N(C₁₋₆ alkyl)₂, -NHC₁₋₆ alkyl, -S(O)₂-C₁₋₆ alkyl and the like.

It will be appreciated by those skilled in the art that the compound represented by formula I may be present in the form of various pharmaceutically acceptable salts. If such compounds have basic centers, they can form acid addition salts; if such compounds have acidic centers, they can form base addition salts; if these compounds comprise both acidic centers (e.g., carboxyl) and basic centers (e.g., amino), they can also form internal salts.

The compound of the present disclosure may be present in the form of a solvate (e.g., hydrate), and the compound of the present disclosure contains a polar solvent as a structural element of the crystal lattice of the compound, particularly, for example, water, methanol, or ethanol. The amount of the polar solvent, especially water, may be present in a stoichiometric or non-stoichiometric ratio.

According to the molecular structure, the compound of the present disclosure may be chiral and may therefore be present in various enantiomeric forms. These compounds may therefore be present in a racemic or optically active form. The compounds of the present disclosure or intermediates thereof may be separated into enantiomers by chemical or physical methods well known to those skilled in the art, or used in such form for synthesis. In the case of racemic amines, diastereoisomers are prepared from mixtures by reaction with optically active resolving agents. Examples of suitable resolving agents are optically active acids such as *R*- or *S*-tartaric acid, diacetyltartaric acid, dibenzoyltartaric acid, mandelic acid, malic acid, lactic acid, suitable *N*-protected amino acids (e.g., *N*-benzoylproline or *N*-benzenesulfonylproline), or various optically active camphorsulfonic acids. Enantiomeric resolution by chromatography can be advantageously conducted with the aid of optically active resolving agents, such as dinitrobenzoylphenylglycine, cellulose triacetate or other carbohydrate derivatives or chirally derivatized methacrylate polymers immobilized on silica gel. Suitable eluents for this purpose are mixtures of solvents containing water or alcohol, for example, hexane/isopropanol/acetonitrile.

The term "tautomer" refers to functional isomers resulting from the rapid movement of an atom in a molecule between two positions. The compounds of the present disclosure may exhibit the tautomerism. Tautomeric compounds may be present in two or more interconvertible forms. Prototropic tautomers result from the migration of a covalently bonded hydrogen atom between two atoms. Tautomers are generally present in an equilibrium form. Efforts to separate a single tautomer usually lead to a mixture, the physicochemical properties of which are consistent with the mixture of the compound. The position of the equilibrium depends on the chemical properties of the molecule. For example, in many aliphatic aldehydes and ketones such as acetaldehyde, the keto form predominates; whereas in phenol, the enol form predominates. In the present disclosure, all tautomeric forms of the compound are included.

The corresponding stable isomers can be separated according to known methods, such as extraction, filtration, or column chromatography.

### Beneficial Effects

In the preparation methods of the present disclosure, separation and purification steps of column chromatography such as silica gel column chromatography and the like are not used, and hydrogen is not used as a hydrogenation or reduction reagent, thereby significantly improving the production efficiency and safety.

In addition, the preparation methods of the present disclosure achieve highly efficient utilization of the position isomers IIIA and IIIB, and prevent the yield of the target products from being adversely influenced by discarding the position isomer IIIA. Even more unexpectedly, the total yield of the method in which the position isomer IIIA is used is as high as 9.4%, even significantly higher than a yield of less than 4% which is generated where the position isomer IIIB is used; where the two position isomers are used simultaneously in the preparation of compound I, the yield is increased by more than 3 times relative to that generated where the position isomer IIIB is used alone.

Accordingly, with the preparation methods of the present disclosure, the compound of formula I and the intermediates thereof can be synthesized in high yield, at low cost and in an environmentally friendly manner, such that the compound of formula I can be produced on an industrial scale.

### DETAILED DESCRIPTION

The technical solutions of the present disclosure will be further described in detail with reference to the following specific examples. It should be understood that the following examples are merely exemplary illustrations and explanations of the present disclosure, and should not be construed as limiting the protection scope of the present disclosure. All techniques implemented based on the content of the present disclosure described above are included within the protection scope of the present disclosure.

Unless otherwise stated, the starting materials and reagents used in the following examples are all commercially available products, or can be prepared by using known methods.

### Example 1: Preparation of Compound 3a and Compound 3b

### (1) 1-Amino-3-bromo-5-(4-methoxybenzyloxy)pyridin-1-ium 2,4,6-trimethylbenzenesulfonate (comound 1)

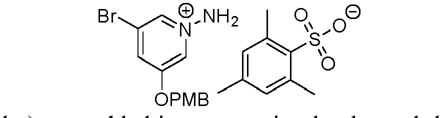

Trifluoroacetic acid (340 kg) was added into a reaction kettle, and the system was purged three times with nitrogen and cooled to 0-10 °C. Under nitrogen atmosphere, *N*-Boc-O-2,4,6-trimethylbenzenesulfonylhydroxylamine (90 kg, 285 mol) was added in batches at a maintained temperature. A large amount of gas was released after each batch was added. After 2 h of reaction, water was added to quench the reaction, and a solid was precipitated. The solid was dissolved in dichloromethane and washed with an aqueous NaHCOs solution until the pH of the organic phase was about 7. The organic phase was separated and then cooled to 0-10 °C under nitrogen atmosphere. With the temperature maintained at 0-10 °C, 3-bromo-5-(4-methoxybenzyloxy)pyridine (64 kg, 217 mol) was added in batches. The mixture was reacted for 1-2 h and then filtered to give a product (89 kg), ¹H-NMR (400 MHz, DMSO-*d*₆): δ. 8.67-8.72 (m, 2H), 8.58 (brs, 2H), 8.34 (d, 1H), 7.43(d, 2H), 7.00 (d, 2H), 6.75 (s, 2H), 5.23 (s, 2H), 3.78 (s, 3H), 2.51 (s, 6H), 2.17 (s, 3H), m/z = 311 [M+1]⁺.

### (2) 6-Bromo-2-fluoro-4-(4-methoxybenzyloxy)pyrazolo[1,5-a]pyridine (compound 3a) and 4-bromo-2-fluoro-6-(4-methoxybenzyloxy)pyrazolo[1,5-a]pyridine (compound 3b)

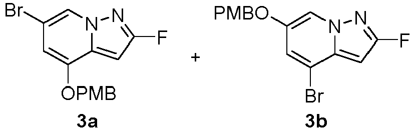

188 kg of tetrahydrofuran was added into a reaction kettle, and then 1-amino-3-bromo-5-(4-methoxybenzyloxy)pyridin-1-ium 2,4,6-trimethylbenzenesulfonate (91 kg, 179 mol) and 2,2-difluorovinyl *p*-toluenesulfonate (51 kg, 218 mol) were added, followed by slow dropwise addition of 1,8-diazabicyclo[5.4.0]undec-7-ene (109 kg, 717 mol). After the dropwise addition was completed, the mixture was reacted at room temperature for 1 h. Dichloromethane (560 kg) was added, and then a saturated ammonium chloride solution (240 kg) was added. The organic phase was washed with water (210 kg), dried over anhydrous sodium sulfate, concentrated under reduced pressure, and recrystallized in acetonitrile to give 6-bromo-2-fluoro-4-(4-methoxybenzyloxy)pyrazolo[1,5-*a*]pyridine (35 kg), ¹H-NMR (400 MHz, DMSO-*d*₆): δ 8.59 (s, 1H), 7.43-7.46 (m, 2H), 7.07 (s, 1H), 6.97-7.00 (m, 2H), 6.39 (d, 1H), 5.22 (s, 2H), 3.78 (s, 3H), m/z = 351 [M+1]⁺; and 4-bromo-2-fluoro-6-(4-methoxybenzyloxy)pyrazolo[1,5-*a*]pyridine (8.2 kg), ¹H-NMR (400 MHz, DMSO-*d*₆): δ 8.50-8.51 (m, 1H), 7.64 (d, 1H), 7.39-7.45 (m, 2H), 6.95-6.99 (m, 2H), 6.33-6.34 (m, 1H), 5.05 (s, 2H), 3.77 (d, 3H), m/z = 351 [M+1]⁺.

### Example 2: Preparation of Compound 11

### (1) 4-Bromo-2-fluoro-6-(4-methoxybenzyloxy)pyrazolo[1,5-a]pyridine-3-carbaldehyde (compound 12)

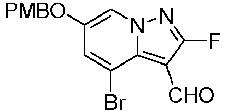

4-Bromo-2-fluoro-6-(4-methoxybenzyloxy)pyrazolo[1,5-*a*]pyridine (7.4 kg, 21 mol) and DMF (21 kg) were added sequentially into a reaction kettle. The system was purged 3 times with nitrogen, and the temperature was controlled at 0-10 °C. Phosphorus oxychloride (8.0 kg, 52 mol) was added dropwise, and the mixture was heated to 20-30 °C for reaction for 17 h to give a product solution, which could be directly used in the next step. ¹H-NMR (400 MHz, DMSO-*d*₆): δ 10.50 (s, 1H), 8.80 (d, 1H), 8.08 (d, 1H), 7.39-7.43 (m, 2H), 6.95-6.99 (m, 2H), 5.12 (s, 2H), 3.77 (s, 3H). m/z = 379 [M+1]⁺.

### (2) 4-Bromo-2-fluoro-6-hydroxypyrazolo[1,5-a]pyridine-3-carbaldehyde (compound 13)

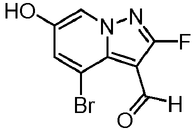

The reaction liquid in the reaction kettle in the previous step was heated to 70 °C. After the starting materials were completely reacted as indicated by sampling for HPLC, the reaction liquid was cooled to 25 °C and then added to 66 kg of water. The mixture was stirred for 2 h with the temperature maintained at 30 °C and filtered. The filter cake was slurried with 17 kg of dichloromethane for 1 h, and the slurry was filtered. The resulting filter cake was dried under vacuum to give a product (4.7 kg), ¹H-NMR (400 MHz, DMSO-*d*₆): δ 10.62 (brs, 1H), 10.46 (d, 1H), 8.32 (t, 1H), 7.80 (s, 1H). m/z = 259 [M+1]⁺.

### (3) 4-Bromo-6-ethoxy-2-fluoropyrazolo[1,5-a]pyridine-3-carbaldehyde (compound 10)

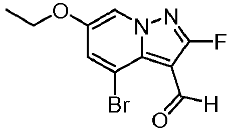

4-Bromo-2-fluoro-6-hydroxypyrazolo[1,5-*a*]pyridine-3-carbaldehyde (4.7 kg, 18 mol), dimethyl sulfoxide (15.5 kg), potassium carbonate (2.8 kg, 20 mol) and iodoethane (3.0 kg, 19 mol) were added sequentially into a reaction kettle. The system was purged three times with nitrogen and heated to 65 °C for reaction. After the starting materials were completely reacted as indicated by sampling for HPLC, the system was cooled to 25 °C. 47 kg of water was added into the reaction kettle, and the mixture was stirred for 1 h at 25-30 °C and then filtered under vacuum to dryness. 47 kg of water was added into the reaction kettle, and the mixture was stirred for 1 h at 25-30 °C and then filtered under vacuum to dryness. Acetonitrile (9.0 kg) was added into a reaction kettle, and the filter cake was added into the reaction kettle. The mixture was stirred for 1-2 h at 25-30 °C, filtered under vacuum to dryness, and dried under vacuum to give a product (4.6 kg). ¹H-NMR (400 MHz, DMSO-*d*₆): δ 10.49 (s, 1H), 8.69 (d, 1H), 8.00 (d, 1H), 4.16 (q, 2H), 1.37 (t, 3H). m/z = 287 [M+1]⁺.

### (4) Synthesis of 4-bromo-6-ethoxy-1H-pyrazolo[3',4':3,4]pyrazolo[1,5-a]pyridine (compound 11)

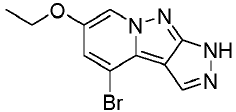

4-Bromo-6-ethoxy-2-fluoropyrazolo[1,5-a]pyridine-3-carbaldehyde (4.6 kg, 16 mol), dimethyl sulfoxide (46 L), and 85% hydrazine hydrate (4.7 kg) were added into a reaction kettle, and the system was heated to 110-115 °C for reaction. After the reaction was completed as indicated by HPLC, the system was cooled to 30-40 °C. Water (138 L) was added, and the mixture was stirred for 1-2 h at a maintained temperature and then filtered. The filter cake was dried under vacuum to give a product (4.0 kg), ¹H-NMR (400 MHz, DMSO-*d*₆): δ 12.81 (brs, 1H), 8.64 (s, 1H), 7.92 (s, 1H), 7.58 (s, 1H), 4.11-4.17 (m, 2H), 1.35-1.38 (t, 3H). m/z = 281 [M+1]⁺.

### Example 3: Preparation of Compound I

### (1) tert-Butyl 3-(5-(6-ethoxy-1H-pyrazolo[3',4':3,4]pyrazolo[1,5-a]pyridin-4-yl)pyridin-2-yl)-3,6-diazabicyclo[3.3.1]heptane-6-carboxylate (compound 19)

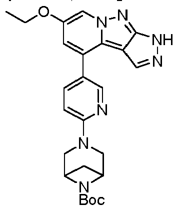

4-Bromo-6-ethoxy-1*H*-pyrazolo[3',4':3,4]pyrazolo[1,5-*a*]pyridine (30 g, 0.11 mol), *tert*-butyl 3-(5-(4,4,5,5-tetramethyl-1,3,2-dioxaborolan-2-yl)pyridin-2-yl)-3,6-diazabicyclo [3.3.3]heptane-6-carboxylate (57 g, 0.14 mol), bis(triphenylphosphine)palladium(II) chloride (1.5 g, 2.2 mmol), lithium carbonate (16 g, 0.22 mol), 300 mL of dioxane, and 150 mL of water were added sequentially into a reaction flask. The system was purged three times with nitrogen, heated to 85 °C for reaction for 20 h, and filtered through celite. The filtrate was poured into 3 L of water, and the mixture was filtered to give a solid. The solid was dissolved in 1.2 L of tetrahydrofuran, and 60 g of SILICYCLE silica gel was added. The mixture was heated to 60 °C, stirred for 12 h, then cooled to room temperature, and filtered through celite to remove the silica gel, and the filtrate was concentrated under reduced pressure to about 100 mL. 300 mL of methyl *tert*-butyl ether was added, and the resulting mixture was concentrated under reduced pressure to about 100 mL. The above procedure was repeated no less than 2 times. The mixture was stirred at room temperature overnight and then filtered. The filter cake was rinsed with a small amount of methyl *tert*-butyl ether, and the solid was dried under vacuum to give a product (42.3 g, HPLC purity: 98.8%). ¹H-NMR (400 MHz, DMSO-*d*₆): δ 12.64 (s, 1H), 8.62 (d, 2H), 8.51 (d, 2H), 8.08 (q, 1H), 7.55 (s, 1H), 7.26 (d, 1H), 6.90 (d, 1H), 4.20-4.26 (m, 2H), 4.15-4.19 (m, 2H), 4.03-4.09 (m, 2H), 2.60 (d, 1H), 1.53 (d, 1H), 1.38-1.42 (m, 3H), 1.30 (s, 9H). m/z = 476 [M+1]⁺.

### (2) 4-(6-(3,6-Diazabicyclo[3.1.1]heptan-3-yl)pyridin-3-yl)-6-ethoxy-1H-pyrazolo[3',4':3,4]pyrazolo [1,5-a]pyridine (compound 20)

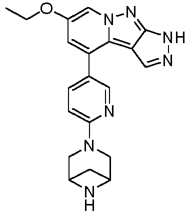

tert-Butyl 3-(5-(6-ethoxy-1*H*-pyrazolo[3',4':3,4]pyrazolo[1,5*-*a]pyridin-4-yl)pyridin-2-yl)-3,6-diazabicyclo[3.3.1]heptane-6-carboxylate (30 g, 0.063 mol), 500 mL of methanol, and concentrated sulfuric acid (25 g, 0.25 mol) were added sequentially into a reaction flask. The system was heated to 60 °C for reaction for 12 h, concentrated under reduced pressure to remove the methanol, and slurried with 500 mL methyl *tert*-butyl ether. The slurry was filtered under vacuum to give a product (43 g, HPLC purity: 99.5%), which was directly used in the next step. ¹H NMR (400 MHz, DMSO-*d*₆): δ 12.67 (s, 1H), 8.62 (d, 1H), 8.50 (d, 1H), 8.07 (q, 1H), 7.59 (s, 1H), 7.23 (d, 1H), 6.84 (d, 1H), 4.15-4.20 (m, 2H), 3.66-3.73 (m, 6H), 3.18 (s, 1H), 2.56-2.57 (m, 1H), 1.49 (d, 1H), 1.35-1.45 (m, 3H). m/z = 376 [M+1]⁺.

### (3) 6-Ethoxy-4-(6-(6-((6-methoxypyridin-3-yl)methyl)-3,6-diazabicyclo[3.3.1]heptan-3-yl)pyridin-3-yl)-1H-pyrazolo[3',4':3,4]pyrazolo[1,5-a]pyridine (compound I)

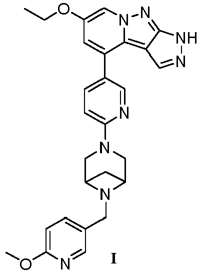

The product of step (2) (30 g), 6-methoxy-3-pyridinecarboxaldehyde (20 g, 0.14 mol), 2-methylpyridine-N-borane (16 g, 0.15 mol), triethylamine (34 mL, 0.24 mol), and 650 mL of *n*-propanol were added sequentially into a reaction flask and reacted at room temperature for 18 h. 300 mL of dichloromethane was added, and the mixture was washed once with a saturated ammonium chloride solution, washed twice with water, and then concentrated under reduced pressure. 300 mL of methanol was added, and the mixture was heated to reflux and then cooled to 40-45 °C. 160 mL of methyl *tert*-butyl ether was slowly added, and the mixture was cooled to 25 °C and stirred for 12 h. Another 160 mL of methyl *tert*-butyl ether was then added, and the mixture was stirred for 1 h and then concentrated under reduced pressure to about 160 mL. 300 mL of methyl *tert*-butyl ether was added, and the mixture was concentrated under reduced pressure again to about 160 mL. The above procedure was repeated no less than 2 times. The mixture was stirred at room temperature overnight and then filtered. The filter cake was rinsed with a small amount of methyl *tert*-butyl ether, and the solid was dried under vacuum to give a product (19 g, HPLC purity: 98.9%). ¹H-NMR (400 MHz, DMSO-*d*₆): δ 12.64 (brs, 1H), 8.64 (s,1H), 8.51 (s,1H), 8.08-8.11 (m, 2H), 7.69 (d, 1H), 7.63 (s, 1H), 7.26 (s, 1H), 6.90 (d, *J* = 8.8Hz, 1H), 6.77 (d, *J* = 8.8Hz, 1H), 4.16-4.21 (m, 2H), 3.83 (s, 3H), 3.76-3.79 (m, 2H), 3.68 (s, 2H), 3.53-3.59 (m, 4H), 2.53-2.56 (m, 1H), 1.56-1.58 (m, 1H), 1.40 (t, 3H). m/z = 497 [M+1]⁺.

### Example 4: Preparation of Compound 11

### (1) 6-Ethoxy-2-fluoro-4-(4-methoxybenzyloxy)pyrazolo[1,5-a]pyridine (compound 14)

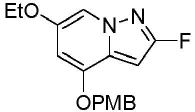

Ethanol (500 mL), 6-bromo-2-fluoro-4-(4-methoxybenzyloxy)pyrazolo[1,5-*a*]pyridine (52.5 g, 150.0 mmol), copper(I) iodide (570 mg, 3.0 mmol), sodium *tert*-butoxide (43.2 g, 450 mmol), and N*1*,*N*2-bis(4-hydroxy-2,6-dimethylphenyl)oxamide (1.0 g, 3.0 mmol) were added sequentially into a reaction flask. Under nitrogen atmosphere, the reaction liquid was heated to 80 °C for reaction for 16 h. After the reaction liquid was cooled, water (150 mL) was added to the reaction liquid under stirring. The mixture was stirred for 30 min and filtered under vacuum to give a solid. The solid was dried under vacuum to give a product (37 g). ¹H-NMR (400 MHz, DMSO-*d*₆): δ 7.91 (s, 1H), 7.41 (d, *J* = 11.2 Hz, 2H), 6.97 (d, *J* = 11.2 Hz, 2H), 6.69 (s, 1H), 6.19 (s, 1H), 5.18 (s, 2H), 3.98-4.03 (m, 2H), 3.76 (s, 3H), 1.32-1.36 (t, 3H). m/z = 317 [M+1]⁺.

### (2) 6-Ethoxy-2-fluoropyrazolo[1,5-a]pyridin-4-ol (compound 15)

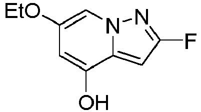

Dichloromethane (450 mL) and 6-ethoxy-2-fluoro-4-((4-methoxybenzyl)oxy)pyrazolo[1,5-*a*]pyridine (37 g, 117.0 mmol) were added sequentially into a reaction flask. Trifluoroacetic acid (45 mL) was added dropwise, and the mixture was reacted for 3 h. The reaction liquid was washed with water (300 mL) and saturated brine (150 mL), and then concentrated to give a crude product solution (about 60 mL). *n*-Heptane (250 mL) was added to the crude product solution described above, and the mixture was stirred for 1 h, filtered under vacuum, and dried under vacuum to give a product (16.5 g). ¹H-NMR (400 MHz, DMSO-*d₆*): δ 10.78 (s, 1H), 7.81 (s, 1H), 6.33 (s, 1H), 6.16 (s, 1H), 3.94-4.00 (m, 2H), 1.35-1.39 (t, 3H). m/z = 197 [M+1]⁺.

### (3) 6-Ethoxy-2-fluoropyrazolo[1,5-a]pyridin-4-yl trifluoromethanesulfonate (compound 16)

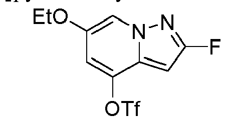

Dichloromethane (200 mL) and 6-ethoxy-2-fluoropyrazolo[1,5-*a*]pyridin-4-ol (14.8 g, 75.5 mmol) were added sequentially into a reaction flask. Triethylamine (11.5 g, 113.3 mmol) and a solution of trifluoromethanesulfonic anhydride (23.4 g, 83.1 mmol) in dichloromethane (50 mL) were then added, and the mixture was reacted at room temperature for 16 h. The reaction liquid was washed with water (200 mL) and saturated brine (100 mL), and then concentrated to give a crude product. DMF (150 mL) was added to the crude product, and after the crude product was completely dissolved, the solution was slowly poured into water (1000 mL) under stirring. After being stirred for 30 min, the mixture was filtered under vacuum, washed with water, and dried by blowing at 60 °C for 16 h to give 6-ethoxy-2-fluoropyrazolo[1,5-*a*]pyridin-4-yl trifluoromethanesulfonate (23.4 g, yield 95%) as a light yellow solid. ¹H-NMR (400 MHz, DMSO-*d*₆): δ 8.56 (s, 1H), 7.60 (s, 1H), 6.48 (s, 1H), 4.08-4.13 (m, 2H), 1.34-1.38 (t, 3H). m/z = 329 [M+1]⁺.

### (4) 4-Bromo-6-ethoxy-2-fluoropyrazolo[1,5-a]pyridine (compound 17)

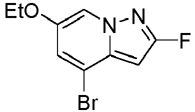

NMP (60 mL), 6-ethoxy-2-fluoropyrazolo[1,5-*a*]pyridin-4-trifluoromethanesulfonate (6.6 g, 20.0 mmol), chloro(pentamethylcyclopentadienyl)bis(triphenylphosphine)ruthenium(II) (800 mg, 1.0 mmol), and LiBr (2.6 g, 30.0 mmol) were added sequentially into a reaction flask. The system was purged 3 times with nitrogen and heated to 90 °C for reaction for 16 h. The reaction liquid was slowly poured into water (800 mL), and the mixture was stirred for 1 h and filtered under vacuum. The solid was washed with water and dried under vacuum to give a product (3.9 g). ¹H-NMR (400 MHz, DMSO-*d*₆): δ 8.40 (s, 1H), 7.56 (s, 1H), 6.32 (s, 1H), 4.03-4.08 (m, 2H), 1.32-1.35 (t, 3H). m/z = 259 [M+1]⁺.

### (5) 4-Bromo-6-ethoxy-2-fluoropyrazolo[1,5-a]pyridine-3-carbaldehyde (compound 10)

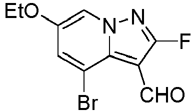

DMF (60 mL), 4-bromo-6-ethoxy-2-fluoropyrazolo[1,5-*a*]pyridine (3.5 g, 13.5 mmol), and POCl₃ (8.3 g, 54 mmol) were added sequentially into a reaction flask, and the mixture was reacted at room temperature for 48 h. The reaction liquid was slowly poured into water (600 mL), and the mixture was stirred for 1 h and filtered under vacuum. The solid was washed with water and dried under vacuum to give a product (4.2 g). ¹H-NMR (400 MHz, DMSO-*d*₆): δ 10.49 (s, 1H), 8.69 (s, 1H), 8.00 (s, 1H), 4.10-4.16 (m, 2H), 1.30-1.34 (t, 3H). m/z = 287 [M+1]⁺.

### (6) 4-Bromo-6-ethoxy-1H-pyrazolo[3',4':3,4]pyrazolo[1,5-a]pyridine (compound 11)

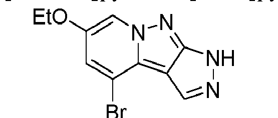

The procedures were the same as those in step (4) of Example 2.

### Example 5: Preparation of Compound 30

### (1) 6-Bromo-1H-pyrazolo[3',4':3,4]pyrazolo[1,5-a]pyridin-4-ol (compound 31)

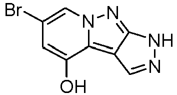

DMSO (200 mL), acethydrazide (8.6 g, 115.8 mmol), and 6-bromo-2-fluoro-4-hydroxypyrazolo[1,5-*a*]pyridine-3-carbaldehyde (20.0 g, 77.2 mmol), and an 80% aqueous hydrazine hydrate solution (24.1 g, 386 mmol) were added sequentially into a reaction flask, and the system was heated to 140 °C for reaction for 6 h. The reaction liquid was cooled, and then slowly poured into water (2000 mL). The mixture was extracted twice with ethyl acetate (1000 mL), washed with water (400 mL), washed with saturated brine (400 mL), and then dried over anhydrous sodium sulfate. The organic phase was concentrated under reduced pressure to give a product (10.5 g). ¹H-NMR (400MHz, DMSO-*d*₆): δ 12.68 (brs, 1H), 11.35 (s, 1H), 8.67 (s, 1H), 7.61 (s, 1H), 6.84 (s, 1H). m/z = 253/255 [M+1]⁺.

### (2) 6-Ethoxy-1H-pyrazolo[3',4':3,4]pyrazolo[1,5-a]pyridin-4-ol (compound 29)

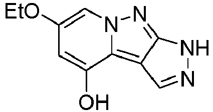

Ethanol (50 mL), 6-bromo-1*H*-pyrazolo[3',4':3,4]pyrazolo[1,5-*a*]pyridin-4-ol (2 g, 8 mmol), copper(I) iodide (160 mg, 8 mmol), cesium carbonate (8 g, 24 mmol), and 8-hydroxyquinoline (240 mg, 1.6 mmol) were added sequentially into a sealed reaction flask. The system was heated to 100 °C for reaction for 4 days. After the reaction was stopped, 200 mL of dioxane was added, and the mixture was heated to reflux and filtered through celite while hot. The filtrate was concentrated under reduced pressure to about 50 mL and then poured into 500 mL of water. The mixture was filtered to give a solid, and the solid was dried under vacuum to give a product (1.5 g). ¹H-NMR (400 MHz, DMSO-*d*₆): δ 12.51 (brs, 1H), 11.02 (brs, 1H), 8.06 (s, 1H), 7.80 (s, 1H), 6.48 (s, 1H), 4.02-4.08 (m, 2H), 1.35 (t, 3H). m/z = 219[M+1]⁺.

### (3) 6-Ethoxy-1H-pyrazolo[3',4':3,4]pyrazolo[1,5-a]pyridin-4-yl trifluoromethanesulfonate (compound 30)

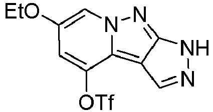

6-Ethoxy-1*H*-pyrazolo[3',4':3,4]pyrazolo[1,5-*a*]pyridin-4-ol (250 mg, 0.68 mmol), diisopropylethylamine (264 mg, 2.0 mmol), and DMF (5 mL) were added into a reaction flask. The system was cooled to 5 °C, and 1,1,1-trifluoro-*N-*phenyl-*N-*((trifluoromethyl)sulfonyl)methanesulfonamide (183 mg, 0.51 mmol) was added. The mixture was reacted at room temperature for 2 h, and then the reaction liquid was poured into 200 mL of water. The mixture was filtered to give a solid, and the solid was dried to give a product (200 mg, HPLC purity: 97.5%). ¹H-NMR (400 MHz, DMSO-*d*₆): δ 12.98 (brs, 1H), 8.77 (s, 1H), 7.85 (s, 1H), 7.59 (s, 1H), 4.15-4.21 (m, 2H), 1.38 (t, 3H). m/z = 351 [M+1]⁺.

### Example 6: Preparation of Compound 30

### (1) 6-Bromo-2-fluoro-4-hydroxypyrazolo[1,5-a]pyridine-3-carbaldehyde (compound 23)

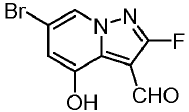

Dichloromethane (15 mL) and 6-bromo-2-fluoro-4-((4-methoxybenzyl)oxy)pyrazolo[1,5-*a*]pyridine-3-carbaldehyde (1.1 g, 3.0 mmol) were added sequentially into a reaction flask. Trifluoroacetic acid (3 mL) was added dropwise, and the mixture was stirred at room temperature for reaction for 3 h and then concentrated under reduced pressure to give a product (0.6 g). ¹H-NMR (400 MHz, DMSO-*d*₆): δ 11.97 (s, 1H), 10.03 (s, 1H), 8.76 (s, 1H), 7.14 (s, 1H). m/z = 259 [M+1]⁺.

### (2) 6-Bromo-1H-pyrazolo[3',4':3,4]pyrazolo[1,5-a]pyridin-4-ol (compound 31)

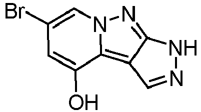

DMSO (200 mL), acethydrazide (8.6 g, 115.8 mmol), and 6-bromo-2-fluoro-4-hydroxypyrazolo[1,5-a]pyridine-3-carbaldehyde (20.0 g, 77.2 mmol), and 80% hydrazine hydrate (24.1 g, 386 mmol) were added sequentially into a reaction flask, and the system was heated to 110 °C for reaction for 12 h. The reaction liquid was cooled and then slowly poured into water (2000 mL). The mixture was filtered, and the solid was dried under vacuum to give a product (10.5 g). ¹H-NMR (400MHz, DMSO-*d*₆): δ 12.68 (brs, 1H), 11.35 (s, 1H), 8.67 (s, 1H), 7.61 (s, 1H), 6.84 (s, 1H). m/z = 253 [M+1]⁺.

### (3) 6-Bromo-4-(4-methoxybenzyloxy)-1H-pyrazolo[3',4':3,4]pyrazolo[1,5-a]pyridine (compound 25)

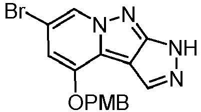

6-Bromo-1H-pyrazolo[3',4':3,4]pyrazolo[1,5-a]pyridin-4-ol (5.0 g, 20.0 mmol), 1-(chloromethyl)-4-methoxybenzene (3.4 g, 22.0 mmol), potassium carbonate (8.3 g, 60.0 mmol), and DMF (50 mL) were added sequentially into a reaction flask. The reaction liquid was heated to 40 °C for reaction for 16 h, then cooled, and slowly poured into water (500 mL) under stirring. The mixture was stirred for 1 h, then filtered under vacuum, washed with water, and dried to give a product (3.7 g). ¹H-NMR (400 MHz, DMSO-*d*₆): δ 12.78 (brs, 1H), 8.81 (s, 1H), 7.83 (s, 1H), 7.48 (d, *J* = 8.4 Hz, 2H), 7.24 (s, 1H), 6.94 (d, *J* = 8.4 Hz, 2H), 5.33 (s, 2H), 3.77 (s, 3H). m/z = 373/375 [M+1]⁺.

### (4) 4-(4-Methoxybenzyloxy)-1H-pyrazolo[3',4':3,4]pyrazolo[1,5-a]pyridin-6-ol (compound 27)

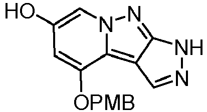

6-Bromo-4-(4-methoxybenzyloxy)-1*H*-pyrazolo[3',4':3,4]pyrazolo[1,5-a]pyridine (3.7 g, 10.0 mmol), bis(pinacolato)diboron (3.8 g, 15.0 mmol), potassium acetate (2.9 g, 30.0 mmol), PdCl₂(dppf) (731 mg, 1.0 mmol), and 1,4-dioxane (70 mL) were added sequentially into a reaction flask. Under nitrogen atmosphere, the reaction liquid was heated to 90 °C for reaction for 16 h. The reaction liquid was then filtered through celite while hot, and the filtrate was concentrated under reduced pressure to give a crude product of 4-(4-methoxybenzyloxy)-6-(4,4,5,5-tetramethyl-1,3,2-dioxaboran-2-yl)-1*H*-pyrazolo[3',4':3,4]py razolo[1,5-*a*]pyridine. THF (70 mL) was added to the crude product, and the mixture was cooled to 5 °C under an ice bath. 30% H₂O₂ (6.5 mL) was then added dropwise, and the mixture was warmed to room temperature for reaction for 1.5 h. Ethyl acetate (150 mL) was added to the

reaction liquid, and the mixture was filtered under vacuum. The filtrate was washed with a 0.1 N aqueous hydrochloric acid solution (50 mL), washed twice with water (40 mL), washed with saturated brine (40 mL), and dried over anhydrous sodium sulfate. The organic phase was concentrated under reduced pressure to give a product (900 mg). ¹H-NMR (400 MHz, DMSO-d₆): δ 12.50 (brs, 1H), 9.83 (s, 1H), 7.88 (s, 1H), 7.69(s, 1H), 7.48(d, *J* = 8.4 Hz, 2H), 6.99 (d, *J* = 8.4 Hz, 2H), 6.70 (s, 1H), 5.28 (s, 2H), 3.79 (s, 3H). m/z = 311 [M+1]⁺.

### (5) 6-Ethoxy-1-ethyl-4-(4-methoxybenzyloxy)-1H-pyrazolo[3',4':3,4]pyrazolo[1,5-a]pyridine comound 28)

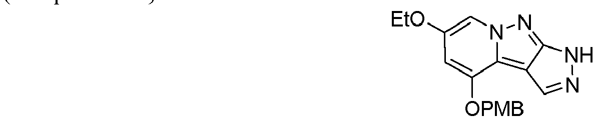

4-(4-Methoxybenzyloxy)-1*H*-pyrazolo[3',4':3,4]pyrazolo[1,5-*a*]pyridin-6-ol (680 mg, 2.2 mmol), K₂CO₃ (911 mg, 6.6 mmol), iodoethane (378 mg, 2.4 mmol), and DMF (15 mL) were added sequentially into a reaction flask. The reaction liquid was heated to 30 °C for reaction for 16 h and then slowly poured into water (150 mL). The mixture was stirred for 0.5 h, filtered under vacuum, washed with water, and dried to give a crude product. The crude product was added to 10 mL of 1,4-dioxane, and the mixture was heated for dissolution. 10 mL of methyl tert-butyl ether was slowly added, and the mixture was cooled to room temperature, and concentrated to 5 mL. Another 20 mL of methyl *t*e*rt*-butyl ether was added, and the mixture was stirred for 12 h, filtered, and dried to give a product (227 mg) as a yellow solid. H¹-NMR (400 MHz, DMSO-*d*₆): δ 12.53 (brs, 1H), 8.16 (s, 1H), 7.73 (s, 1H), 7.46 (d, *J* = 8.4 Hz, 2H), 6.99 (d, *J* = 8.4 Hz, 2H), 6.82 (s, 1H), 5.31 (s, 2H), 4.06-4.12 (m, 2H), 3.77 (s, 3H), 1.37 (t, 3H). m/z = 339 [M+1]⁺.

### (6) 6-Ethoxy-1H-pyrazolo[3',4':3,4]pyrazolo[1,5-a]pyridin-4-ol (compound 29)

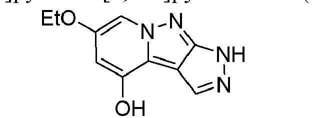

6-Ethoxy-1-ethyl-4-((4-methoxybenzyl)oxy)-1*H*-pyrazolo[3',4':3,4]pyrazolo[1,5-*a*]pyridine (230 mg, 0.68 mmol) and dichloromethane (15 mL) were added sequentially into a reaction flask. Trifluoroacetic acid (0.5 mL) was added, and the reaction liquid was warmed to room temperature, stirred for reaction for 16 h, and then concentrated to give a product (250 mg), which was directly used in the next step. ¹H-NMR (400 MHz, DMSO-*d*₆): δ 12.51 (brs, 1H), 11.02 (brs, 1H), 8.06 (s, 1H), 7.80 (s, 1H), 6.48 (s, 1H), 4.02-4.08 (m, 2H), 1.35 (t, 3H). m/z = 219 [M+1]⁺.

### (7) 6-Ethoxy-1H-pyrazolo[3',4':3,4]pyrazolo[1,5-a]pyridin-4-yl trifluoromethanesulfonate (compound 30)

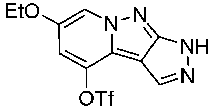

With reference to step (3) of Example 5, compound 30 was prepared.

### Example 7: Preparation of Compound 19

### tert-Butyl 3-(5-(6-ethoxy-1H-pyrazolo[3',4':3,4]pyrazolo[1,5-a]pyridin-4-yl)pyridin-2-yl)-3,6-diazabicyclo[3.3.1]heptane-6-carboxylate (compound 19)

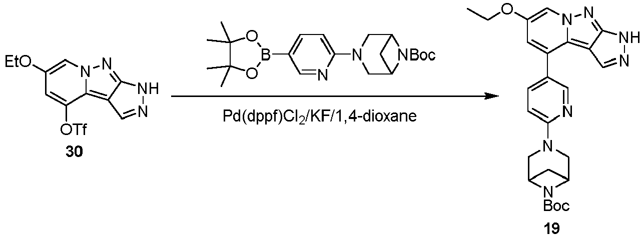

*tert*-Butyl 3-(5-(4,4,5,5-tetramethyl-1,3,2-dioxaborolan-2-yl)pyridin-2-yl)-3,6-diazabicyclo[3.1.1] heptane-6-carboxylate (60 mg, 0.15 mmol), 6-ethoxy-1*H*-pyrazolo[3',4':3,4]pyrazolo[1,5-*a*]pyridin-4-yl trifluoromethanesulfonate (35 mg, 0.05 mmol), PdCl₂ (dppf) (4 mg, 0.005 mmol), potassium fluoride (18 mg, 0.3 mmol), and 1,4-dioxane (0.5 mL) were added sequentially into a reaction flask. Under nitrogen atmosphere, the system was heated to 120 °C for reaction for 16 h. The organic phase was concentrated and then slurried with 50 mL of methyl *tert*-butyl ether. The slurry was filtered to give a product (12 mg). m/z = 476[M+1]⁺.

### Example 8: Preparation of Compound 10

### 4-Bromo-6-ethoxy-2-fluoropyrazolo[1,5-a]pyridine-3-carbaldehyde (compound 10)

### (1) 6-Bromo-2-fluoro-4-((4-methoxybenzyl)oxy)pyrazolo[1,5-a]pyridine-3-carbaldehyde (compound 4)

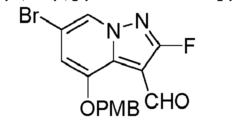

DMF (35 mL) and 6-bromo-2-fluoro-4-((4-methoxybenzyl)oxy)pyrazolo[1,5-*a*]pyridine (3.5 g, 10.0 mmol) were added sequentially into a reaction flask, and the system was cooled to 5 °C. POCl₃ (6.1 g, 40.0 mmol) was added dropwise, and then the mixture was stirred at room temperature for reaction for 48 h. The reaction liquid was slowly poured into water (350 mL), and the mixture was stirred for 1 h and filtered under vacuum. The solid was dried under vacuum to give a product (3.6 g). ¹H-NMR (400 MHz, DMSO-*d*₆): δ 10.01 (s, 1H), 8.88 (s, 1H), 7.59 (s, 1H), 7.47 (d, *J* = 8.8 Hz, 2H), 7.01 (d, *J* = 8.8 Hz, 2H), 5.34 (s, 2H), 3.78 (s, 3H). m/z = 379 [M+1]⁺.

### (2) 2-Fluoro-4-((4-methoxybenzyl)oxy)-6-(4,4,5,5-tetramethyl-1,3,2-dioxaborolan-2-yl)pyrazolo [1,5-a]pyridine-3-carbaldehyde (compound 5)

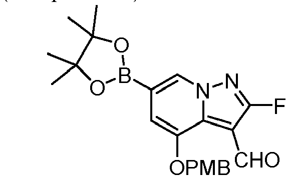

6-Bromo-2-fluoro-4-((4-methoxybenzyl)oxy)pyrazolo[1,5-*a*]pyridine-3-carbaldehyde (7.6 g, 20.0 mmol), bis(pinacolato)diboron (5.6 g, 22.0 mmol), potassium acetate (5.9 g, 60.0 mmol), PdCl₂(dppf) (1.5 g, 2.0 mmol), and 1,4-dioxane (100 mL) were added sequentially into a reaction flask. The system was purged 3 times with nitrogen and heated to 85 °C for reaction for 16 h. The reaction liquid was filtered through celite, and the filtrate was concentrated under reduced pressure to give a product (8.6 g), which was directly used in the next step. m/z = 465 [M+1]⁺.

### (3) 2-Fluoro-6-hydroxy-4-((4-methoxybenzyl)oxy)pyrazolo[1,5-a]pyridine-3-carbaldehyde (compound 6)

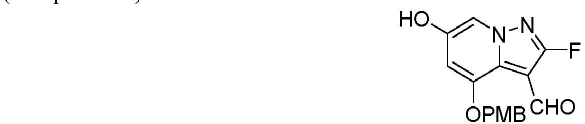

2-Fluoro-4-((4-methoxybenzyl)oxy)-6-(4,4,5,5-tetramethyl-1,3,2-dioxaboran-2-yl)pyrazolo[1,5-*a*]pyridine-3-carbaldehyde (4.3 g, 10.1 mmol) and 1,4-dioxane (110 mL) were added sequentially into a reaction flask. The system was cooled to 5 °C under an ice bath, and 30% H₂O₂ (4 mL) was added dropwise. The reaction liquid was warmed to room temperature for reaction for 2 h. 200 mL of dichloromethane was added, and the mixture was washed sequentially with 50 mL of water, a NaHSO₃ solution (50 mL), and saturated brine (50 mL), and then dried over anhydrous sodium sulfate. The organic phase was concentrated to a volume of 10 mL, and 100 mL of methyl *tert*-butyl ether was added. The mixture was filtered to give a product (2.1 g). ¹H-NMR (400 MHz, DMSO-*d*₆): δ 10.27 (s, 1H), 9.97 (s, 1H), 7.84 (s, 1H), 7.47 (d, *J* = 8.4 Hz, 2H), 7.06 (s, 1H), 7.00 (d, *J* = 8.4 Hz, 2H), 5.28 (s, 2H), 3.78 (s, 3H). m/z = 317 [M+1]⁺.

### (4) 6-Ethoxy-2-fluoro-4-((4-methoxybenzyl)oxy)pyrazolo[1,5-a]pyridine-3-carbaldehyde (compound 7)

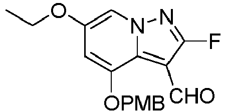

2-Fluoro-6-hydroxy-4-((4-methoxybenzyl)oxy)pyrazolo[1,5-*a*]pyridine-3-carbaldehyde (2.0 g, 6.3 mmol), K₂CO₃ (1.8 g, 12.6 mmol), iodoethane (1.2 g, 7.6 mmol), and DMF (19 mL) were added sequentially into a reaction flask. The reaction liquid was heated to 40 °C for reaction for 3 h and then poured into 400 mL of water. The mixture was extracted 3 times with 100 mL of dichloromethane, and the filtrate was concentrated to a volume of 10 mL. Methyl *tert*-butyl ether (100 mL) was added, and the mixture was concentrated under reduced pressure to about 10 mL. Another 100 mL of methyl *tert*-butyl ether was added, and the mixture was concentrated under reduced pressure again to about 10 mL. The above procedure was repeated twice to give a product (1.25 g). ¹H-NMR (400 MHz, DMSO-*d*₆): δ 10.07 (s, 1H), 8.20 (s, 1H), 7.47 (d, *J* = 8.4 Hz, 2H), 7.18 (s, 1H), 7.00 (d, *J* = 8.4 Hz, 2H), 5.32 (s, 2H), 4.07-4.12 (m, 2H), 3.78 (s, 3H), 1.32-1.39 (t, 3H). m/z = 345 [M+1]⁺.

### (5) 6-Ethoxy-2-fluoro-4-hydroxypyrazolo[1,5-a]pyridine-3-carbaldehyde (compound 8)

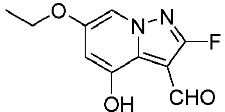

6-Ethoxy-2-fluoro-4-((4-methoxybenzyl)oxy)pyrazolo[1,5-*a*]pyridine-3-carbaldehyde (450 mg, 1.3 mmol) and 30 mL of dichloromethane were added sequentially into a reaction flask. Trifluoroacetic acid (2 mL) was added dropwise, and the mixture was reacted at room temperature for 2 h and then concentrated under reduced pressure to a volume of 10 mL. 100 mL of methyl *tert*-butyl ether was added, and the mixture was concentrated under reduced pressure to about 10 mL. Another 100 mL of methyl *tert*-butyl ether was added, and the mixture was concentrated under reduced pressure again to about 10 mL. The above procedure was repeated twice to give a product (305 mg). ¹H-NMR (400 MHz, DMSO-*d*₆): δ 11.59 (s, 1H), 9.85 (s, 1H), 8.15 (s, 1H), 6.83 (s, 1H), 4.04-4.09 (m, 2H), 1.30-1.36 (t, 3H). m/z = 225 [M+1]⁺.

### (6) 6-Ethoxy-2-fluoro-3-formylpyrazolo[1,5-a]pyridin-4-yl trifluoromethanesulfonate (compound 9)

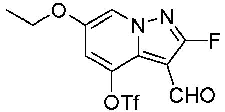

6-Ethoxy-2-fluoro-4-hydroxypyrazolo[1,5-*a*]pyridine-3-carbaldehyde (315 mg, 1.4 mmol), diisopropylethylamine (362 mg, 2.8 mmol), and DMF (5 mL) were added to a reaction flask. The system was cooled to 5 °C, and 1,1,1-trifluoro-*N*-phenyl-*N*-((trifluoromethyl)sulfonyl) methanesulfonamide (660 mg, 1.7 mmol) was added. The reaction liquid was warmed to room temperature for reaction for 2 h and then poured into 200 mL of water. The mixture was extracted 3 times with 100 mL of dichloromethane and dried over anhydrous sodium sulfate. The organic phase was concentrated to give a product (430 mg), which was directly used in the next step. ¹H-NMR (400 MHz, DMSO-*d*₆): δ 9.89 (s, 1H), 8.85 (s, 1H), 7.89 (s, 1H), 4.17-4.22 (m, 2H), 1.32-1.38 (t, 3H). m/z = 357 [M+1]⁺.

### (7) 4-Bromo-6-ethoxy-2-fluoropyrazolo[1,5-a]pyridine-3-carbaldehyde (compound 10)

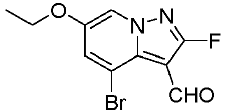

NMP (0.5 mL), 6-ethoxy-2-fluoro-3-formylpyrazolo[1,5-*a*]pyridin-4-trifluoromethanesulfonate (5 mg, 0.015 mmol), tris(acetonitrile)pentamethylcyclopentadienylruthenium(II) trifluoromethanesulfonate (1.0 mg, 0.002 mmol), and LiBr (4 mg, 0.045 mmol) were added sequentially into a reaction flask. The system was purged 3 times with nitrogen and heated to 100 °C for reaction for 2 h. The reaction liquid was poured into 100 mL of water, and the mixture was extracted 3 times with 100 mL of dichloromethane and dried over anhydrous sodium sulfate. The organic phase was concentrated and separated by column chromatography to give a product (2 mg). ¹H-NMR (400 MHz, DMSO-*d*₆): δ 10.49 (s, 1H), 8.69 (s, 1H), 8.00 (s, 1H), 4.10-4.16 (m, 2H), 1.30-1.34 (t, 3H). m/z = 287 [M+1]⁺.

The exemplary embodiments of the present disclosure have been described above. It should be understood that the protection scope of the present application is not limited to the illustrative embodiments described above. Any modification, equivalent replacement, alteration and the like made by those skilled in the art without departing from the spirit and principle of the present disclosure shall fall within the protection scope of the claims of the present application.

## Claims

1. A preparation method M1A for a compound of formula I, wherein the preparation method comprises reacting a compound of formula XX with R^{k}-C(O)-H to give the compound of formula I: 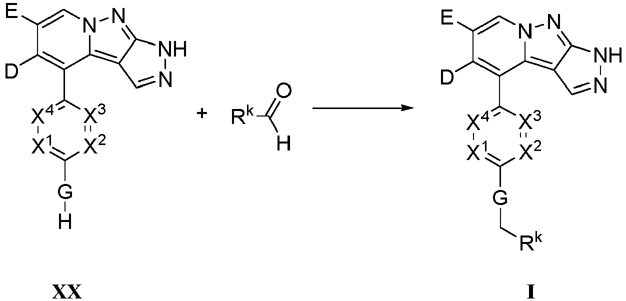 wherein,
X¹, X², X³, and X⁴ are identical or different, and are each independently selected from CR¹ or N;
each R¹ is identical or different, and is independently selected from H, halogen, CN, OH, and the following groups unsubstituted or optionally substituted with one, two or more R^{a}: C₁₋₄₀ alkyl, C₃₋₄₀ cycloalkyl, C₁₋₄₀ alkyloxy, C₃₋₄₀ cycloalkyloxy, NR²R³, -C(O)R⁴, -OCR⁵, -S(O)₂R⁶, and OS(O)₂R⁷;
D and E are identical or different, and are each independently selected from H, halogen, CN, OH, -O-R²¹, and the following groups unsubstituted or optionally substituted with one, two or more R^{c}: C₁₋₄₀ alkyl, C₃₋₄₀ cycloalkyl, C₆₋₂₀ aryl, 5- to 20-membered heteroaryl, 3- to 20-membered heterocyclyl, and NR²R³, provided that at least one of D and E is selected from -O-R²¹;
R²¹ is selected from the following groups unsubstituted or optionally substituted with one, two or more R^{d}: C₁₋₄₀ alkyl, C₃₋₄₀ cycloalkyl, C₆₋₂₀ aryl, 5- to 20-membered heteroaryl, and 3- to 20-membered heterocyclyl;
G is selected from the following groups unsubstituted or optionally substituted with one, two or more R^{e}: C₃₋₄₀ cycloalkyl, C₃₋₄₀ cycloalkenyl, C₆₋₂₀ aryl, 5- to 20-membered heteroaryl, 3- to 20-membered heterocyclyl, C₃₋₄₀ cycloalkyloxy, C₃₋₄₀ cycloalkenyloxy, C₆₋₂₀ aryloxy, 5- to 20-membered heteroaryloxy, and 3- to 20-membered heterocyclyloxy;
R^{k} is selected from the following groups unsubstituted or optionally substituted with one, two or more R^{g}: C₁₋₄₀ alkyl, C₃₋₄₀ cycloalkyl, C₃₋₄₀ cycloalkenyl, C₆₋₂₀ aryl, 5- to 20-membered heteroaryl, and 3- to 20-membered heterocyclyl;
each R² is identical or different, and is independently selected from H, C₁₋₄₀ alkyl, C₃₋₄₀ cycloalkyl, C₆₋₂₀ aryl, 5- to 20-membered heteroaryl, 3- to 20-membered heterocyclyl, -C(O)R⁴, and -S(O)₂R⁶;
each R³ is identical or different, and is independently selected from H, C₁₋₄₀ alkyl, C₃₋₄₀ cycloalkyl, C₆₋₂₀ aryl, 5- to 20-membered heteroaryl, 3- to 20-membered heterocyclyl, -C(O)R⁴, and -S(O)₂R⁶;
or, R² and R³, together with the N atom connected thereto, form 5- to 20-membered heteroaryl or 3- to 20-membered heterocyclyl;
each R⁴ is identical or different, and is independently selected from H, C₁₋₄₀ alkyl, C₃₋₄₀ cycloalkyl, C₆₋₂₀ aryl, 5- to 20-membered heteroaryl, 3- to 20-membered heterocyclyl, C₁₋₄₀ alkyloxy, C₃₋₄₀ cycloalkyloxy, C₆₋₂₀ aryloxy, 5- to 20-membered heteroaryloxy, 3- to 20-membered heterocyclyloxy, and NR²R³;
each R⁵ is identical or different, and is independently selected from H, C₁₋₄₀ alkyl, C₃₋₄₀ cycloalkyl, C₆₋₂₀ aryl, 5- to 20-membered heteroaryl, 3- to 20-membered heterocyclyl, C₁₋₄₀ alkylcarbonyl, C₃₋₄₀ cycloalkylcarbonyl, C₆₋₂₀ arylcarbonyl, 5- to 20-membered heteroarylcarbonyl, and 3- to 20-membered heterocyclylcarbonyl;
each R⁶ is identical or different, and is independently selected from H, C₁₋₄₀ alkyl, C₂₋₄₀ alkenyl, C₂₋₄₀ alkynyl, C₃₋₄₀ cycloalkyl, C₆₋₂₀ aryl, 5- to 20-membered heteroaryl, 3- to 20-membered heterocyclyl, C₁₋₄₀ alkyloxy, C₃₋₄₀ cycloalkyloxy, C₆₋₂₀ aryloxy, 5- to 20-membered heteroaryloxy, 3- to 20-membered heterocyclyloxy, and NR²R³;
each R⁷ is identical or different, and is independently selected from H, C₁₋₄₀ alkyl, C₃₋₄₀ cycloalkyl, C₆₋₂₀ aryl, 5- to 20-membered heteroaryl, and 3- to 20-membered heterocyclyl;
each R^{a}, R^{c}, R^{d} and R^{e} are identical or different, and are independently selected from halogen, CN, OH, SH, oxo (=O), NO₂, and the following groups unsubstituted or optionally substituted with one, two or more R^{g}: C₁₋₄₀ alkyl, C₂₋₄₀ alkenyl, C₂₋₄₀ alkynyl, C₃₋₄₀ cycloalkyl, C₆₋₂₀ aryl, 5-to 20-membered heteroaryl, 3- to 20-membered heterocyclyl, C₁₋₄₀ alkyloxy, C₃₋₄₀ cycloalkyloxy, C₆₋₂₀ aryloxy, 5- to 20-membered heteroaryloxy, and 3- to 20-membered heterocyclyloxy;
each R^{g} is identical or different, and is independently selected from halogen, CN, OH, SH, oxo (=O), NO₂, and the following groups unsubstituted or optionally substituted with one, two or more R^{h}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, C₁₋₄₀ alkyloxy, C₂₋₄₀ alkenyloxy, C₂₋₄₀ alkynyloxy, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, and 3- to 20-membered heterocyclyloxy;
each R^{h} is identical or different, and is independently selected from halogen, CN, OH, SH, oxo (=O), NO₂, and the following groups unsubstituted or optionally substituted with one, two or more R^{g}: C₁₋₄₀ alkyl, C₂₋₄₀ alkenyl, C₂₋₄₀ alkynyl, C₃₋₄₀ cycloalkyl, C₃₋₄₀ cycloalkenyl, C₃₋₄₀ cycloalkynyl, C₆₋₂₀ aryl, 5- to 20-membered heteroaryl, 3- to 20-membered heterocyclyl, C₁₋₄₀ alkyloxy, C₂₋₄₀ alkenyloxy, C₂₋₄₀ alkynyloxy, C₃₋₄₀ cycloalkyloxy, C₃₋₄₀ cycloalkenyloxy, C₃₋₄₀ cycloalkynyloxy, C₆₋₂₀ aryloxy, 5- to 20-membered heteroaryloxy, and 3- to 20-membered heterocyclyloxy;
or, where the C₃₋₄₀ cycloalkyl, C₃₋₄₀ cycloalkenyl, C₃₋₄₀ cycloalkynyl or 3- to 20-membered heterocyclyl described above is substituted with two or more substituents at different positions, any two of the substituents may also, together with the atom connected thereto, form a bridged ring, wherein the bridge atoms other than the bridgehead atoms in the bridged ring may comprise 1, 2, 3, 4, or 5 divalent groups selected from CH₂, O, and NH;
or, where one atom (such as carbon atom) is substituted with two or more substituents, two of the substituents may also, together with the shared atom connected thereto, form a cyclic group such as C₃₋₄₀ cycloalkyl, C₃₋₄₀ cycloalkenyl, C₃₋₄₀ cycloalkynyl, 3- to 20-membered heterocyclyl, or the like.

2. The preparation method M1A as claimed in claim 1, wherein,
X¹, X², X³, and X⁴ are identical or different, and are each independently selected from CR¹ or N;
for example, at least one of X¹, X², X³, and X⁴ is N, e.g., one, two or three of X¹, X², X³, and X⁴ are N;
each R¹ is identical or different, and is independently selected from H, halogen, CN, OH, and the following groups unsubstituted or optionally substituted with one, two or more R^{a}: C₁₋₆ alkyl, C₃₋₁₀ cycloalkyl, C₁₋₆ alkyloxy, and C₃₋₁₀ cycloalkyloxy, for example, selected from the following group unsubstituted or optionally substituted with 1, 2 or 3 R^{a}: C₁₋₆ alkyl, C₃₋₆ cycloalkyl, C₁₋₆ alkoxy, or C₃₋₆ cycloalkyloxy;
D and E are identical or different, and are each independently selected from H, halogen, CN, NH₂, C₁₋₆ alkyl, or -O-R²¹, provided that at least one of D and E is selected from -O-R²¹;
preferably, at least one of D and E is selected from the following group: 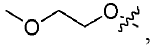 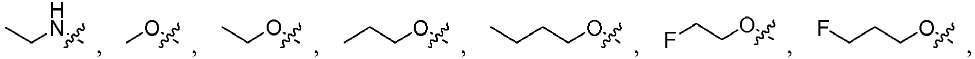 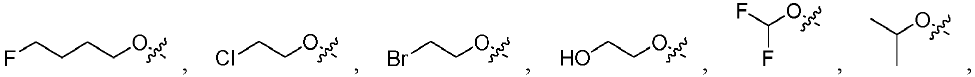 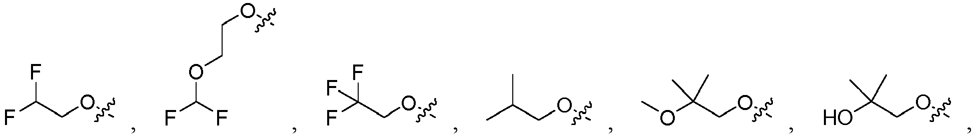 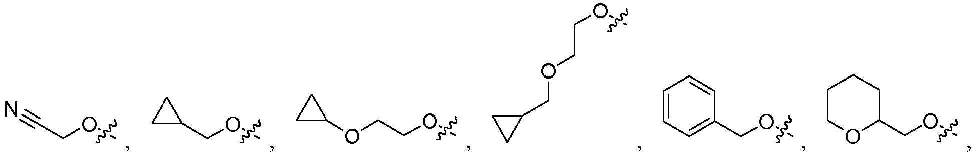 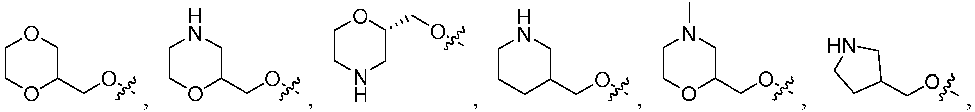 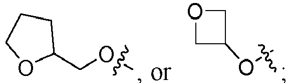
preferably, R² is selected from C₁₋₆ alkyl unsubstituted or optionally substituted with one, two or more R^{d};
preferably, each R^{a}, R^{c} and R^{d} are identical or different, and are independently selected from halogen, CN, OH, and the following groups unsubstituted or optionally substituted with one, two or more R^{g}: C₁₋₆ alkyl, C₁₋₆ alkyloxy, C₃₋₁₀ cycloalkyl, and C₃₋₁₀ cycloalkyloxy;
preferably, each R^{g} is identical or different, and is independently selected from halogen or C₃₋₁₀ cycloalkyl;
preferably, G is selected from C₃₋₁₀ cycloalkyl, C₆₋₁₄ aryl, 5- to 14-membered heteroaryl, and 3-to 10-membered heterocyclyl, for example, 6- to 7-membered heterocyclyl having a monocyclic, bicyclic or bridged ring structure containing 1, 2 or 3 heteroatoms independently selected from N, O, and S;
preferably, R^{k} is selected from the following groups unsubstituted or optionally substituted with one, two or more R^{g}: C₃₋₁₀ cycloalkyl, C₆₋₁₄ aryl, 5- to 14-membered heteroaryl, and 3- to 10-membered heterocyclyl, wherein the heteroaryl may be pyridinyl, for example, selected from pyridin-2-yl, pyridin-3-yl, pyridin-4-yl, pyridin-5-yl, and pyridin-6-yl; the aryl may be phenyl; preferably, X¹, X², X³, and X⁴ are identical or different, and are each independently selected from CH or N; for example, at least one of X¹, X², X³, and X⁴ is N, e.g., one, two or three of X¹, X², X³, and X⁴ are N;
preferably, R¹ is H;
preferably, E is H;
preferably, D is selected from the following groups: halogen, BnO-, H, -CN, -NH₂, -OCH₃, and the following groups: 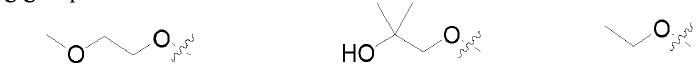 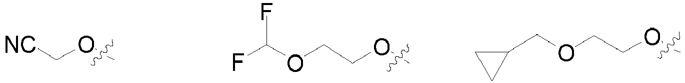 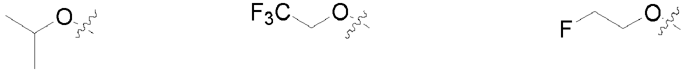 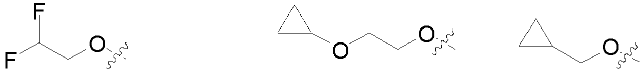 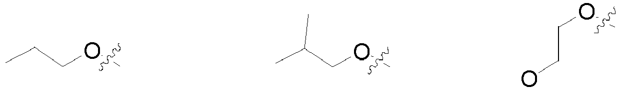 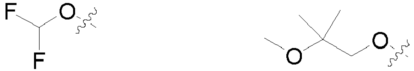 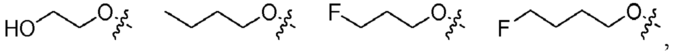 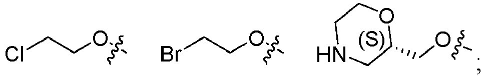
preferably, G is selected from 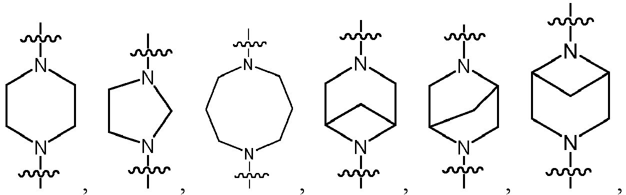 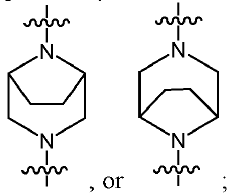
preferably, R^{k} is selected from pyridinyl or phenyl unsubstituted or optionally substituted with one, two or more R^{g}, wherein where one, two or more R^{g} substituents are present, the R^{g} may be a substituent at position 1, 2, 3, 4, 5 or 6 of the pyridinyl or phenyl, as long as it does not affect the connection of R^{k} to group G via methylene;
preferably, R^{k} in the compound of formula I may, together with methylene, form a group selected from the following: 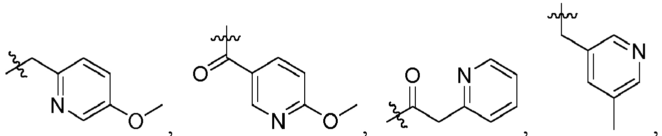 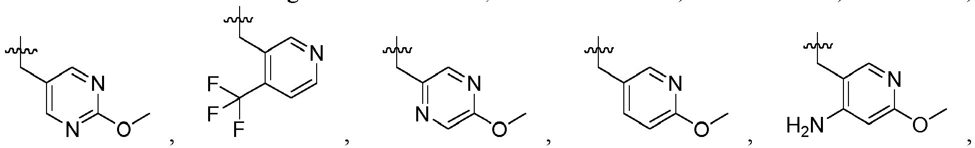 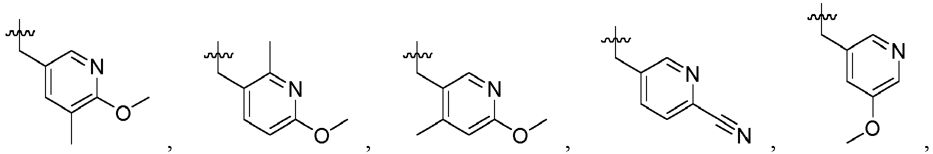 or 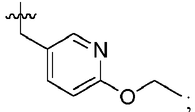
or R^{k} in the compound of formula I may be selected from group 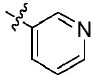 substituted with 1, 2, 3 or 4 groups selected from C₁₋₆ alkyl and C₁₋₆ alkyloxy.

3. The preparation method M1A as claimed in claim 1, wherein the compound of formula I is selected from the following compounds:

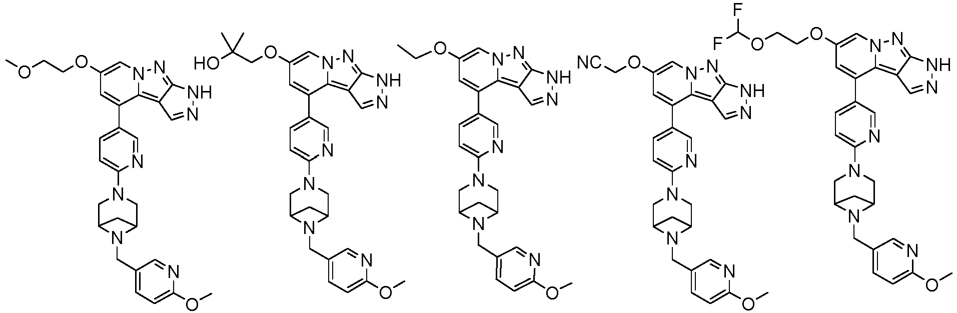

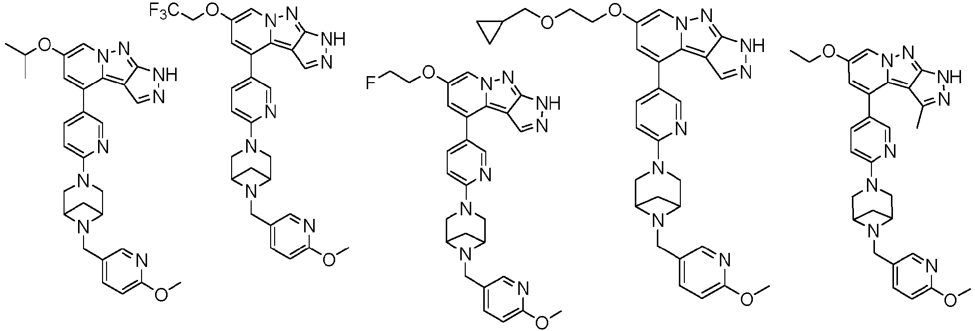

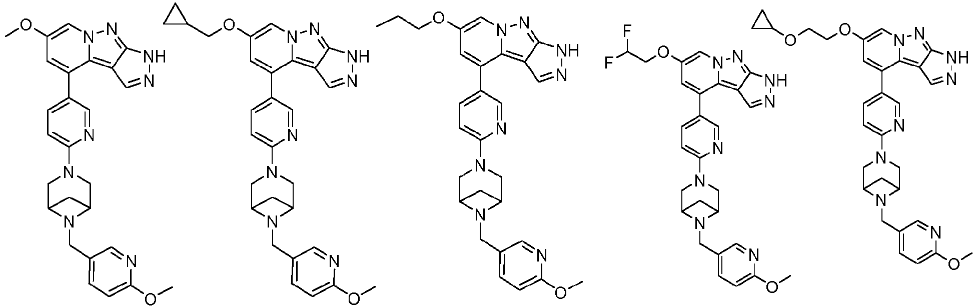

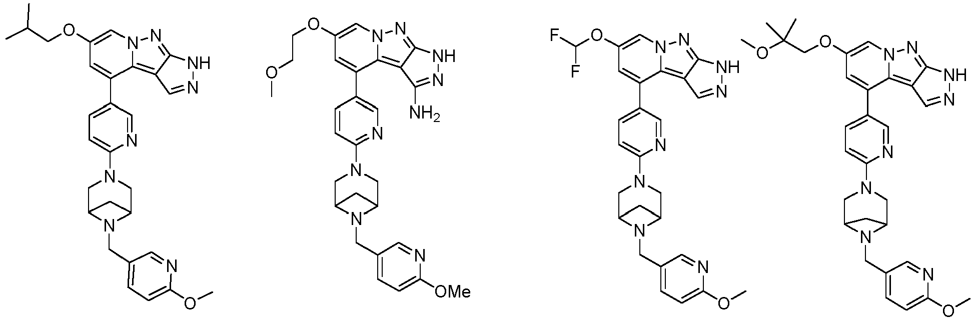

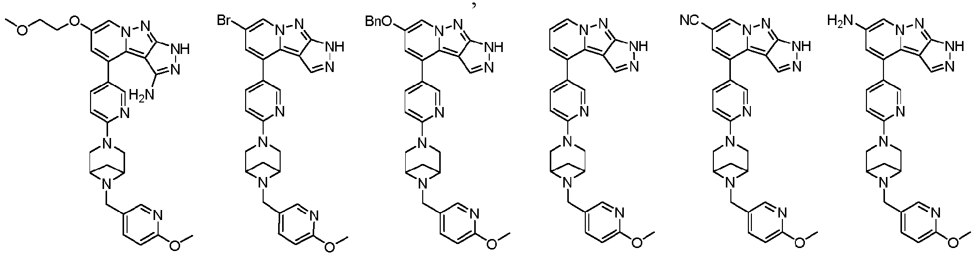

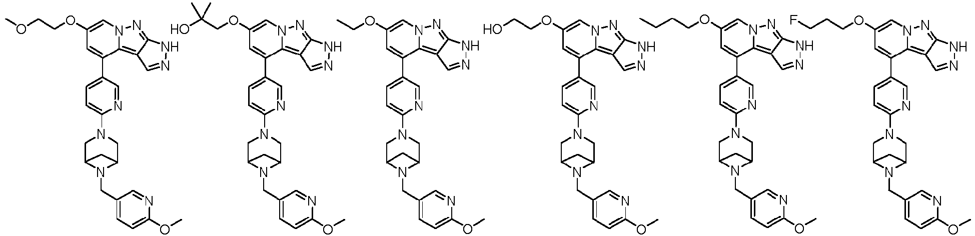

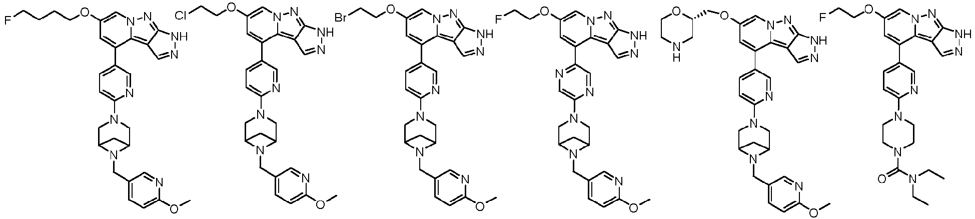

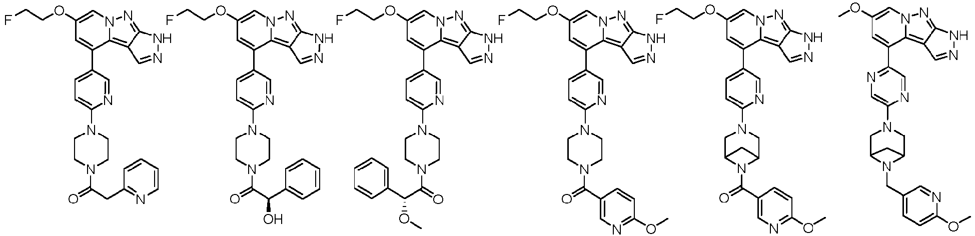

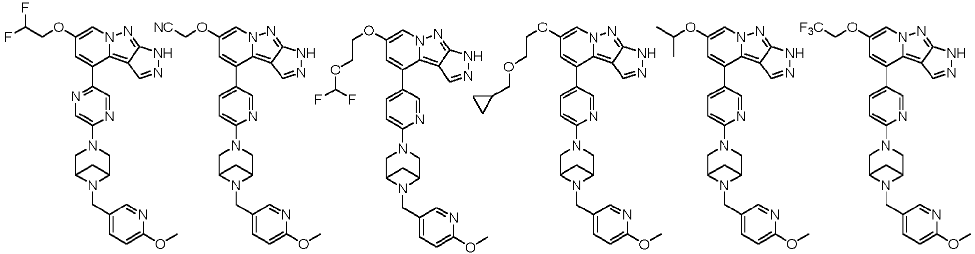

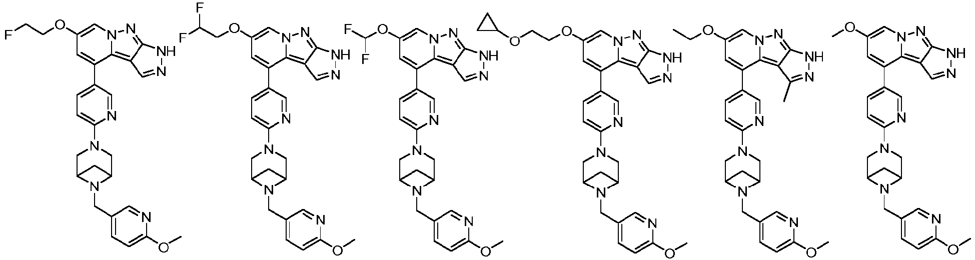

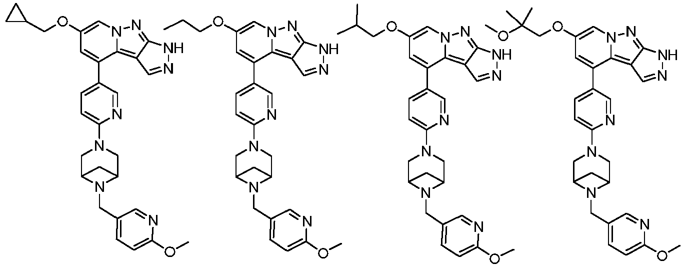

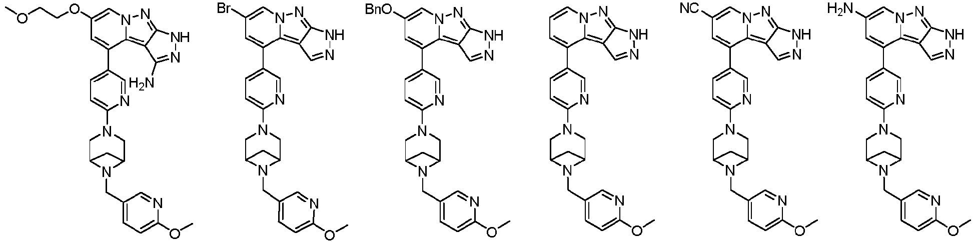

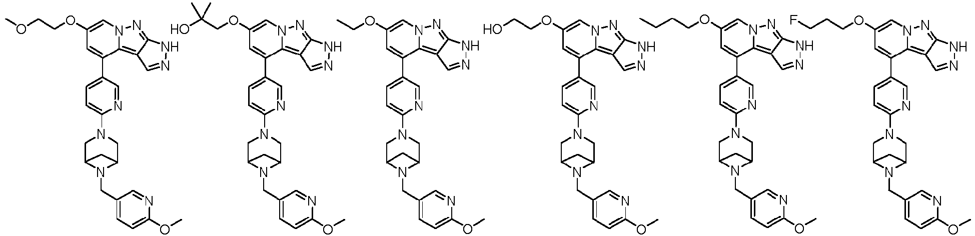

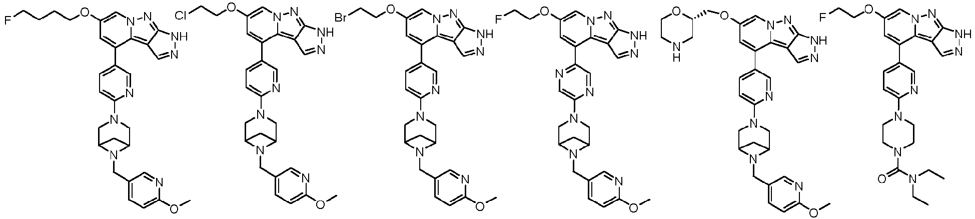

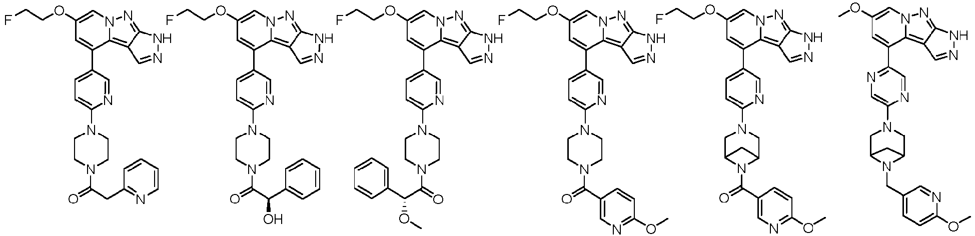

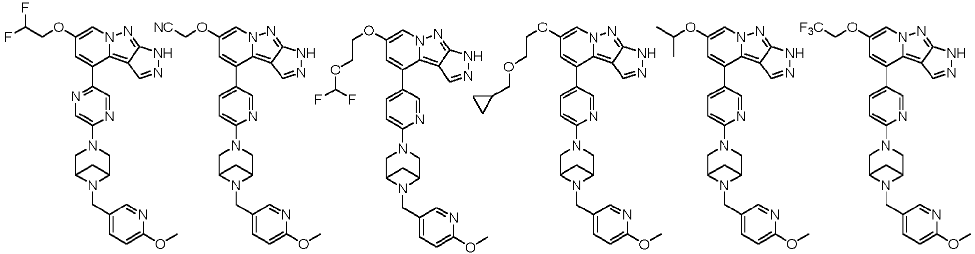

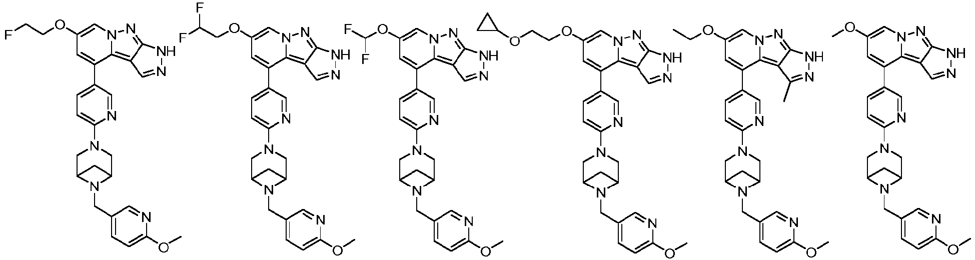

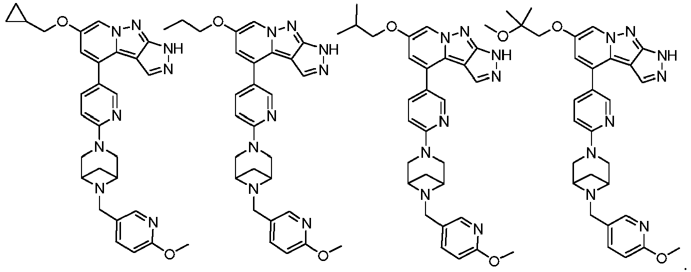

4. The preparation method M1A as claimed in any one of claims 1 to 3, wherein,
the reaction of the compound of formula XX with R^{k}-C(O)-H may be carried out in the presence of borane, pyridine borane, 2-picoline borane (Pic-BH₃), sodium borohydride, sodium triacetoxyborohydride, or sodium cyanoborohydride, preferably in the presence of 2-picoline borane and a base;
the molar ratio of the compound of formula XX to R^{k}-C(O)-H may be 1:1 to 1:5, preferably 1:1.1 to 1:1.3;
the molar ratio of the compound of formula XX to borane or 2-picoline borane may be 1:1 to 1:5, preferably 1:1.1 to 1:1.3;
the molar ratio of the compound of formula XX to the base may be 1:1 to 1:5, preferably 1:1.5 to 1:3.5, such as 1:1.8 to 1:2.2;
the reaction in the preparation method for the compound of formula I may be carried out at a temperature of 15-50 °C, for example, 20-30 °C, such as room temperature.

5. The preparation method M1A as claimed in any one of claims 1 to 4, wherein,
the preparation method M1A comprises subjecting compound 20 to the following reaction to prepare compound 1:

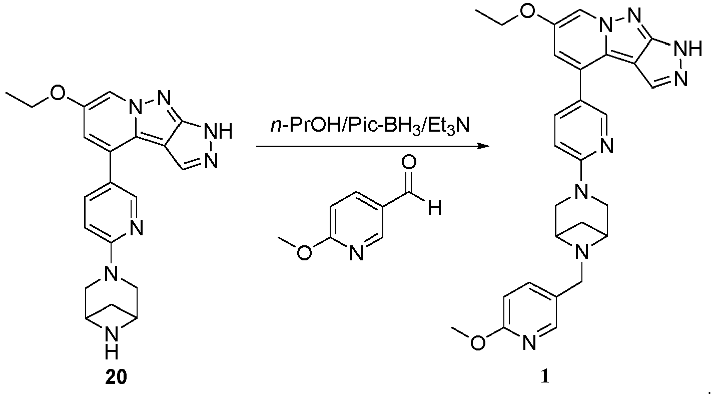

6. A preparation method M20 for the compound of formula XX, comprising reacting a compound of formula XIX under a condition where PG¹⁹ is removed to give the compound of formula XX: 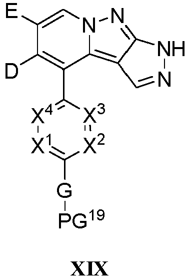 wherein,
G is selected from the following groups unsubstituted or optionally substituted with one, two or more R^{e}: 5- to 20-membered heteroaryl, 3- to 20-membered heterocyclyl, 5- to 20-membered heteroaryloxy, or 3- to 20-membered heterocyclyloxy, wherein G has at least 1 N atom, and the N atom is connected to PG¹⁹;
X¹, X², X³, X⁴, D, E, and R^{e} are independently defined as in claims 1 to 4;
PG¹⁹ is an amino protecting group, for example, PG¹⁹ may be selected from *tert*-butoxycarbonyl (Boc), cyclobutoxycarbonyl, benzyloxycarbonyl (CBz), *p*-methoxybenzylcarbonyl (Moz), 2-biphenyl-2-propoxycarbonyl (BPoc), 2,2,2-trichloroethoxycarbonyl (Troc), phthalimidyl, *p*-toluenesulfonyl, trifluoroacetyl, (9*H*-fluoren-9-ylmethoxy)carbonyl (Fmoc), benzyl, 4-methoxybenzyl, diphenylmethyl, 2-(trimethylsilyl)ethoxycarbonyl (Teoc), adamantyloxycarbonyl (Adoc), formyl, acetyl, and the like;
the condition where PG¹⁹ of the compound of formula XIX is removed may be, for example, a condition where PG¹⁹ group of the compound of formula XIX is removed in the presence of an acid, wherein the acid may be selected from one, two or more of inorganic acids or organic acids such as hydrochloric acid, sulfuric acid, formic acid, acetic acid, and the like;
preferably, in the preparation method for the compound of formula XX, the molar ratio of the compound of formula XIX to the acid may be 1:1 to 1:5, such as 1:3 to 1:4;
the reaction of the compound of formula XIX for removing PG¹⁹ may be carried out in the presence of an organic solvent, wherein the organic solvent may be, for example, an alcohol solvent, such as one, two or more selected from methanol, ethanol, *n*-propanol, isopropanol, *n*-butanol, sec-butanol, and *tert*-butanol;
preferably, the reaction in the preparation method for the compound of formula I may be carried out at a temperature of 40-70 °C, for example, 50-60 °C;
the compound of formula XX is defined as in claims 1 to 5.

7. The preparation method M20 as claimed in claim 6, wherein the preparation method comprises subjecting compound 19 to the following reaction to prepare compound 20:

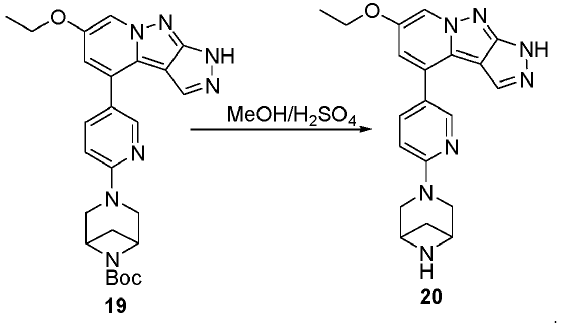

8. A preparation method M19A for the compound of formula XIX, comprising reacting a compound of formula XI with a compound of formula XVIII to give the compound of formula XIX: 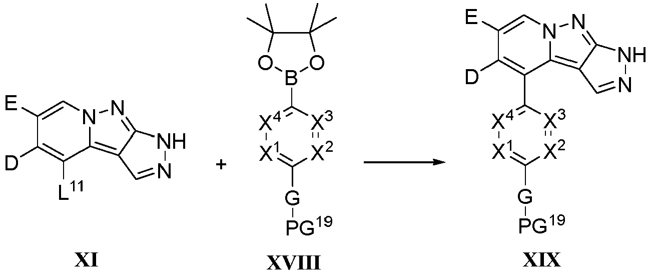
wherein X¹, X², X³, X⁴, D, E, and G are independently defined as in claim 6 or 7;
L¹¹ is selected from leaving groups, such as the following groups unsubstituted or substituted with 1, 2, 3, 4, 5 or 6 halogens: C₁₋₆ alkylsulfonyloxy and C₁₋₆ alkylbenzenesulfonyloxy (e.g., methanesulfonyloxy (MsO-), trifluoromethanesulfonyloxy (TfO-), and *p*-toluenesulfonyloxy (TsO-)), and halogen (e.g., F, Cl, Br, or I);
preferably, the reaction may be carried out at a temperature of not less than 50 °C, for example, not less than 80 °C, such as 85 °C;
preferably, the preparation method may be carried out in the presence of a catalyst, wherein the catalyst may be a palladium catalyst, such as Pd(dba)₂, PdCl₂, Pd(OAc)₂, Pd(dppf)Cl₂, Pd₂(dba)₃, PdCl₂(CH₃CN)₂, Pd(PPh₃)₄, PdCl₂(PPh₃)₂, Pd(t-Bu)₃, PdCl₂(PPh₃)₂•CH₂Cl₂, Pd(OAc)/PPh₃, PdCl₂[(Pet₃)]₂, Pd(DIPHOS)₂, PdCl₂(Bipy), [PdCl(Ph₂PCH₂PPh₂)]₂, PdCl₂[P(o-Tol)₃]₂, Pd₂(dba)₃/P(o-Tol)₃, Pd₂(dba)/P(furanyl)₃, PdCl₂[P(furanyl)₃]₂, PdCl₂(PMePh₂)₂, PdCl₂[P(4-F-Ph)₃]₂, PdCl₂[P(C₆F₆)₃]₂, PdCl₂[P(2-COOH-Ph)(Ph)₂]₂, and PdCl₂[P(4-COOH-Ph)(Ph)₂]₂;
preferably, the preparation method may be carried out in the presence of a base;
preferably, the molar ratio of the compound of formula XI to the compound of formula XVIII may be 1:1 to 1:2, for example, 1:1.1 to 1:1.3, such as 1:1.2 to 1:1.3;
preferably, the molar ratio of the compound of formula XI to the base may be 1:1 to 1:5, for example, 1:1.5 to 1:3.5, such as 1:1.8 to 1:2.2;
preferably, the molar ratio of the compound of formula XI to the catalyst may be 1:0.001 to 1:0.05, such as 1:0.02 to 1:0.03.

9. The preparation method M19A as claimed in claim 8, comprising subjecting compound 11 and compound 18 to the following reaction to prepare compound 19:

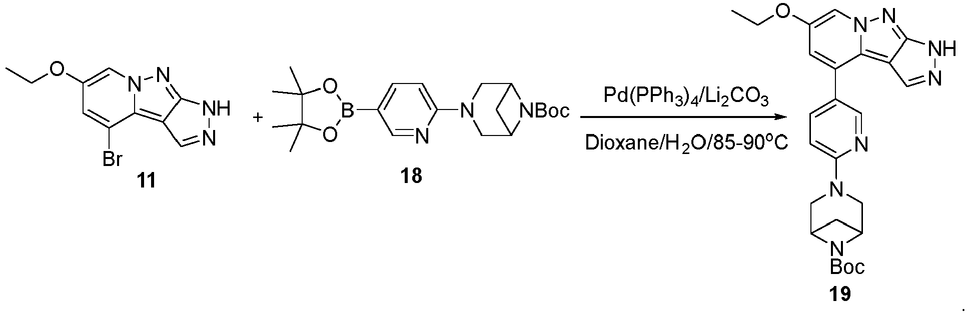

10. A preparation method M11 for the compound of formula XI, comprising reacting a compound of formula X with hydrazine (e.g., hydrazine hydrate) to give the compound of formula XI: 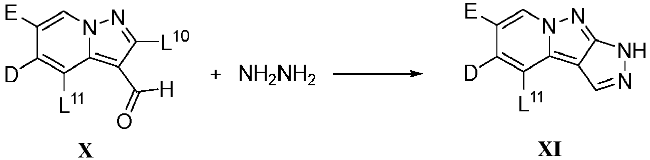 wherein,
D, E, and L¹¹ are independently defined as in claim 8 or 9;
L¹⁰ is selected from a leaving group, for example, halogen, such as F, Cl, Br, or I;
preferably, the reaction may be carried out at a temperature of not less than 100 °C, such as 110-115 °C;
preferably, the molar ratio of the compound of formula X to hydrazine may be 1:1 to 1:15, for example, 1:2 to 1:10, such as 1:6 to 1:8.

11. The preparation method M11 as claimed in claim 10, wherein the preparation method comprises subjecting compound 10 to the following reaction to prepare compound 11:

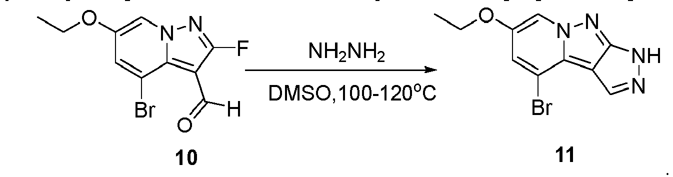

12. A preparation method M10A for the compound of formula X, comprising reacting a compound of formula XIII with a compound of R²¹-L¹³ to give the compound of formula X: 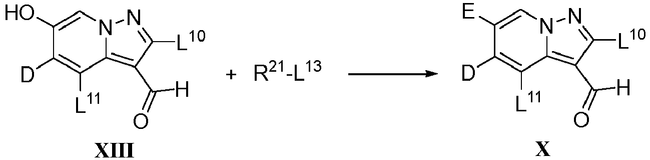 wherein,
D, L¹⁰, and L¹¹ are independently defined as in claim 10 or 11;
E is selected from -O-R²¹,
R²¹ is defined as in any one of claims 1 to 5;
L¹³ is selected from leaving groups, for example, halogens, such as the following groups unsubstituted or substituted with 1, 2, 3, 4, 5 or 6 halogens: C₁₋₆ alkylsulfonyloxy and C₁₋₆ alkylbenzenesulfonyloxy (e.g., methanesulfonyloxy (MsO-), trifluoromethanesulfonyloxy (TfO-), and *p*-toluenesulfonyloxy (TsO-), and F, Cl, Br or I;
preferably, the molar ratio of the compound of formula XIII to the compound of R²¹-L¹³ may be 1:1 to 1:2, for example, 1:1 to 1:1.2, such as 1:1 to 1:1.1;
preferably, the reaction may be carried out at a temperature of not less than 50 °C, for example, 60-70 °C.

13. The preparation method M10A as claimed in claim 11 or 12, wherein the preparation method comprises subjecting compound 13 to the following reaction to prepare compound 10:

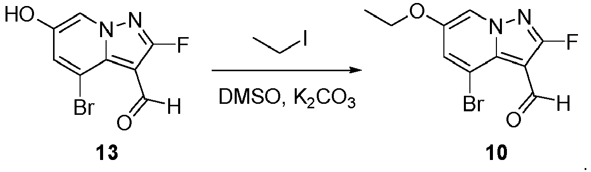

14. A preparation method M13 for the compound of formula XIII, comprising reacting a compound of formula IIIB in the presence of phosphorus oxychloride (POCl₃), wherein the compound of formula IIIB has the following structure: 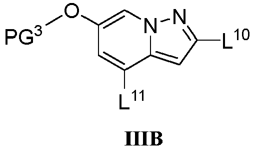 wherein,
L¹⁰ and L¹¹ are independently defined as in claim 12 or 13;
PG³ is selected from hydroxyl protecting groups such as the following groups unsubstituted or optionally substituted with 1, 2, 3, 4 or 5 substituents selected from C₁₋₆ alkyl, C₁₋₆ alkyloxy and halogen: benzyl, C₁₋₆ alkyl, tri(C₁₋₆ alkyl)silyl, *tert*-butyldimethylsilyl, *tert*-butyldiphenylsilyl, allyl, triphenylmethyl, C₁₋₆ alkyloxymethyl, benzyloxymethyl, C₁₋₆ alkylcarbonyl, C₁₋₆ haloalkylcarbonyl, and benzoyl, for example, selected from benzyl, 4-methoxybenzyl, 4-methylbenzyl, 4-chlorobenzyl, 4-bromobenzyl, or 2,3,4-trimethoxybenzyl.

15. The preparation method M13 as claimed in claim 14, wherein the preparation method comprises reacting compound 3b in the presence of phosphorus oxychloride (POCl₃), wherein compound 3b has the following structure:

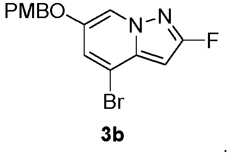

16. The preparation method M13 as claimed in claim 14 or 15, wherein the compound of formula IIIB may be first reacted with phosphorus oxychloride to give a compound of formula XII, and then the compound of formula XII is further reacted to give the compound of formula XIII, wherein the compound of formula XII has the following structure: 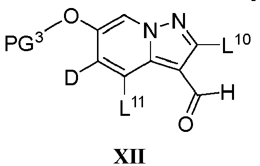
wherein L¹⁰, L¹¹, and PG³ are independently defined as in claim 14 or 15;
preferably, the molar ratio of the compound of formula IIIB to phosphorus oxychloride may be 1:2 to 1:4, for example, 1:2 to 1:3, such as 1:2.4 to 1:2.5;
preferably, the preparation method for the compound of formula XIII may be carried out by a one-pot process;
preferably, a reaction solvent for the reaction of the compound of formula IIIB with phosphorus oxychloride to give the compound of formula XII may be an organic solvent, such as *N*,*N*-dimethylformamide;
preferably, the reaction of the compound of formula IIIB with phosphorus oxychloride to give the compound of formula XII may be carried out at a temperature of -5 to 80 °C, for example, 0-70 °C.

17. The preparation method M13 as claimed in any one of claims 14 to 16, comprising preparing compound 12 from compound 3b and preparing compound 13 from compound 12 by the following reaction:

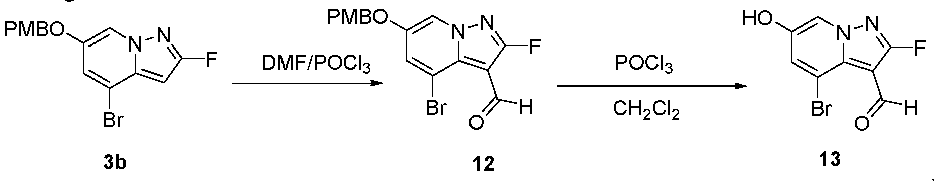

18. A preparation method M10B for the compound of formula X, comprising reacting a compound of formula XVII with phosphorus oxychloride to give the compound of formula X: 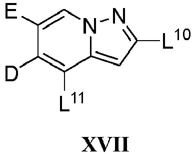
wherein D, E, L¹⁰, and L¹¹ are independently defined as in claim 10 or 11;
preferably, the molar ratio of the compound of formula XVII to phosphorus oxychloride may be 1:2 to 1:8, for example, 1:3 to 1:6, such as 1:4 to 1:5.

19. The preparation method M10B as claimed in claim 18, comprising subjecting compound 17 to the following reaction to prepare compound 10:

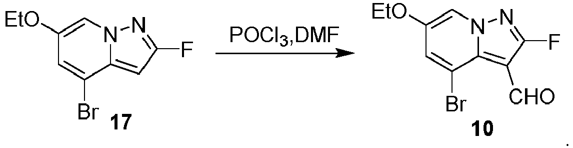

20. A preparation method M17 for the compound of formula XVII, comprising reacting a compound of formula XVI with an alkali metal halide or an organic halide salt to give the compound of formula XVII: 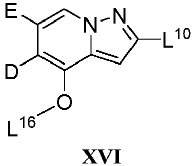
wherein D, E, and L¹⁰ are independently defined as in claim 10 or 11;
L¹⁶ is the following group unsubstituted or substituted with 1, 2, 3, 4, 5 or 6 halogens: C₁₋₆ alkylsulfonyl or C₁₋₆ alkylbenzenesulfonyl, such as methanesulfonyl (Ms-), trifluoromethanesulfonyl (Tf-) or *p*-toluenesulfonyl (Ts-);
the alkali metal halide or the organic halide salt may be selected from, for example, at least one of LiBr, NaBr, KBr, CsBr, and Bu₄NBr;
the molar ratio of the compound of formula XVI to the alkali metal halide or the organic halide salt may be 1:1 to 1:5, for example, 1:1 to 1:2;
the preparation method for the compound of formula XVII may be carried out in the presence of a catalyst, wherein the catalyst is, for example, chloro(pentamethylcyclopentadienyl)bis(triphenylphosphine)ruthenium(II), tris(acetonitrile)pentamethylcyclopentadienylruthenium(II) trifluoromethanesulfonate, pentamethylcyclopentadienyltris(acetonitrile)ruthenium(II) hexafluorophosphate, chloro(pentamethylcyclopentadienyl)ruthenium(II) (dimer), chloro(pentamethylcyclopentadienyl)ruthenium(I) (tetramer), tris(acetonitrile)tetramethylcyclopentadienylruthenium(II) trifluoromethanesulfonate, or dichloro(1,5-cyclooctadien)ruthenium(II);
according to an embodiment of the present disclosure, in the preparation method for the compound of formula XVII, the molar ratio of the compound of formula XVI to the catalyst may be 1:0.01 to 1:0.1;
according to an embodiment of the present disclosure, the reaction in the preparation method for the compound of formula XVII may be carried out at a temperature of not less than 50 °C, for example, not less than 80 °C, such as 90-100 °C.

21. The preparation method M17 as claimed in claim 20, comprising subjecting compound 16 to the following reaction to prepare compound 17:

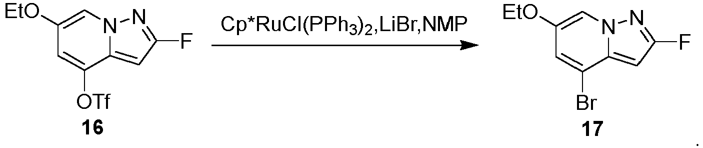

22. A preparation method M16 for the compound of formula XVI, comprising reacting a compound of formula XV with a sulfonylation reagent to give the compound of formula XVI: 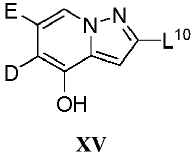
wherein D, E, and L¹⁰ are independently defined as in claim 10 or 11;
the sulfonylation reagent may be selected from one of C₁₋₆ alkylsulfonyl chloride unsubstituted or substituted with 1, 2, 3, 4, 5 or 6 halogens, C₁₋₆ alkylsulfonic anhydride unsubstituted or substituted with 1, 2, 3, 4, 5 or 6 halogens, and C₁₋₆ alkylbenzenesulfonyl chloride unsubstituted or substituted with 1, 2, 3, 4, 5 or 6 halogens, such as methanesulfonyl chloride, methanesulfonic anhydride, trifluoromethanesulfonyl chloride, trifluoromethanesulfonic anhydride, *p*-toluenesulfonyl chloride, *p*-toluenesulfonic anhydride, and 1,1,1-trifluoro-*N*-phenyl-*N*-((trifluoromethyl)sulfonyl)methanesulfonamide (PhN(Tf)₂); preferably, the molar ratio of the compound of formula XV to the sulfonylation reagent may be 1:1 to 1:5, for example, 1:1 to 1:1.2, such as 1:1.1 to 1:1.15.

23. The preparation method M16 as claimed in claim 22, comprising subjecting compound 15 to the following reaction to prepare compound 16:

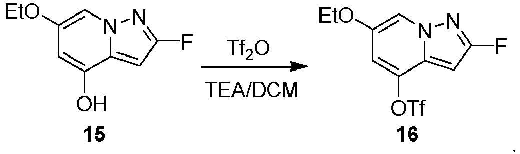

24. A preparation method M15 for the compound of formula XV, comprising reacting a compound of formula XIV with an acid to give the compound of formula XV: 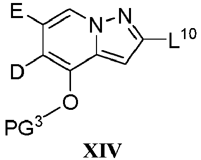
wherein D and E are independently defined as in claims 1 to 5;
L¹⁰ and PG³ are independently defined as in any one of claims 14 to 17;
preferably, the acid may be selected from at least one of organic acids or inorganic acids such as hydrochloric acid, hydrobromic acid, hydroiodic acid, phosphoric acid, sulfuric acid, methanesulfonic acid, trifluoromethanesulfonic acid, trimethylsilyl trifluoromethanesulfonate, trifluoroacetic acid, phosphorus oxychloride, succinic acid, and ascorbic acid;
preferably, the molar ratio of the compound of formula XIV to trifluoroacetic acid may be 1:1 to 1:10, for example, 1:4 to 1:6, such as 1:5 to 1:5.5.

25. The preparation method M15 as claimed in claim 24, comprising subjecting compound 14 to the following reaction to prepare compound 15:

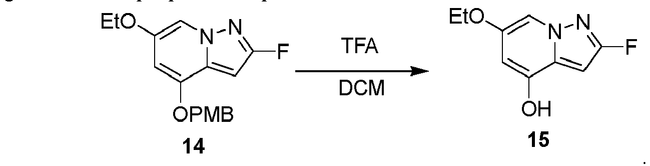

26. A preparation method M14 for the compound of formula XIV, comprising reacting a compound of formula IIIA with ethanol to give the compound of formula XIV: 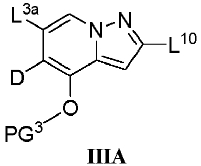
wherein D is defined as in claims 1 to 5;
L¹⁰ and PG³ are independently defined as in any one of claims 14 to 17;
L^{3a} is selected from a leaving group, for example, halogen, such as chlorine, bromine, or iodine; preferably, the preparation method for the compound of formula XIV may be carried out in the presence of a copper(I) catalyst or a copper(I) catalyst with a ligand; the copper catalyst may be selected from at least one of copper(I) iodide (CuI), copper(I) bromide (CuBr), and copper(II) acetylacetonate (Cu(acac)₂);
preferably, the ligand may be selected from at least one of *N*1,*N*2-bis(4-hydroxy-2,6-dimethylphenyl)oxamide, 8-hydroxyquinoline, *N*,*N'*-bis(2,4,6-trimethoxyphenyl)oxamide, *N*-benzyl-*N*-(2-methylnaphthalen-1-yl)oxamide, and *N*1,*N*2-bis(4-hydroxy-2,6-dimethylphenyl)oxamide;
preferably, the molar volume ratio of the compound of formula IIIA to ethanol may be 1 mol:(2-5) L, such as 1 mol:(3-4) L;
preferably, the molar ratio of the compound of formula IIIA to the copper(I) catalyst may be 1:0.01 to 1:0.2;
preferably, the molar ratio of the compound of formula IIIA to the base may be 1:1 to 1:10, such as 1:2 to 1:4;
preferably, the molar ratio of the compound of formula IIIA to the ligand may be 1:0.01 to 1:0.2, such as 1:0.02;
preferably, the reaction may be carried out at a temperature of not less than 50 °C, for example, not less than 70 °C, such as 80 to 100 °C.

27. The preparation method M14 as claimed in claim 26, comprising subjecting compound 3a to the following reaction to prepare compound 14:

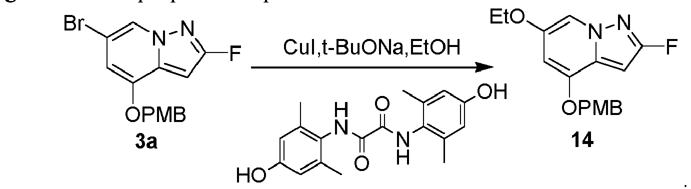

28. A preparation method M10C for the compound of formula X, comprising reacting a compound of formula IX with an alkali metal halide or an organic halide salt to give the compound of formula X: 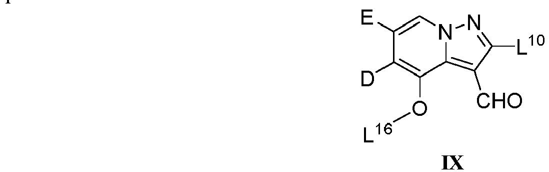
wherein D, E, L¹⁰, and L¹⁶ are independently defined as described above;
preferably, the alkali metal halide or the organic halide salt may be selected from, for example, at least one of LiBr, NaBr, KBr, CsBr, and Bu₄NBr;
the molar ratio of the compound of formula IX to the alkali metal halide or the organic halide salt may be 1:1 to 1:5, for example, 1:1 to 1:2;
the preparation method for the compound of formula X may be carried out in the presence of a catalyst, wherein the catalyst is, for example, chloro(pentamethylcyclopentadienyl)bis(triphenylphosphine)ruthenium(II), tris(acetonitrile)pentamethylcyclopentadienylruthenium(II) trifluoromethanesulfonate, pentamethylcyclopentadienyltris(acetonitrile)ruthenium(II) hexafluorophosphate, chloro(pentamethylcyclopentadienyl)ruthenium(II) (dimer), chloro(pentamethylcyclopentadienyl)ruthenium(I) (tetramer), tris(acetonitrile)tetramethylcyclopentadienylruthenium(II) trifluoromethanesulfonate, or dichloro(1,5-cyclooctadien)ruthenium(II);
the molar ratio of the compound of formula IX to the catalyst may be 1:0.01 to 1:0.1, such as 0.05;
according to an embodiment of the present disclosure, the reaction in the preparation method for the compound of formula X may be carried out at a temperature of not less than 50 °C, for example, not less than 80 °C, such as 90-100 °C.

29. The preparation method M10C as claimed in claim 28, comprising subjecting compound 9 to the following reaction to prepare compound 10:

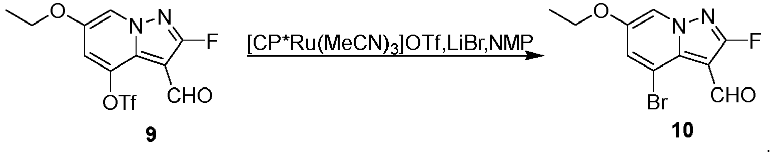

30. A preparation method for a compound of formula IV, comprising reacting the compound of formula IIIA with phosphorus oxychloride to give the compound of formula IV shown below: 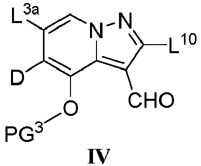
wherein D, L¹⁰, PG³, L^{3a}, and the compound of formula IIIA are independently defined as in claim 26 or 27;
the molar ratio of the compound of formula IIIA to phosphorus oxychloride may be 1:2 to 1:8, for example, 1:3 to 1:6, such as 1:4 to 1:5.

31. A preparation method for a compound of formula XXIII, comprising reacting the compound of formula IV with trifluoroacetic acid to give the compound of formula XXIII shown below: 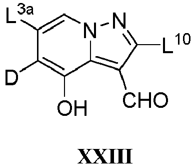 wherein D, L¹⁰, and L^{3a} are independently defined as in claim 26 or 27.

32. A preparation method for a compound of formula XXXI, comprising reacting the compound of formula XXIII with hydrazine (e.g., hydrazine hydrate) to give the compound of formula XXXI shown below: 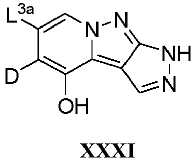 wherein D and L^{3a} are independently defined as in claim 26 or 27.

33. A preparation method for a compound of formula XXIX, comprising derivatizing group L^{3a} of the compound of formula XXXI into group E defined as in any one of claims 1 to 5, for example,
converting L^{3a} into a hydroxyl-derived intermediate and then further derivatizing the hydroxyl-derived intermediate to group E, so as to give the compound of formula XXIX;
or, with reference to the preparation method M14 as claimed in claim 26 or 27, derivatizing group L^{3a} of the compound of formula XXXI into group E to give the compound of formula XXIX:

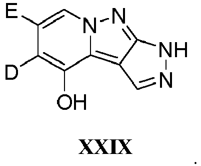

34. A preparation method for a compound of formula XXX, comprising reacting the compound of formula XXIX defined as in claim 33 with the sulfonylation reagent defined as in claim 22 or 23 to give the compound of formula XXX in which the hydrogen atom on hydroxyl is substituted with the following group unsubstituted or substituted with 1, 2, 3, 4, 5 or 6 halogens: C₁₋₆ alkylsulfonyl or C₁₋₆ alkylbenzenesulfonyl, such as methanesulfonyl (Ms-), trifluoromethanesulfonyl (Tf-) or *p*-toluenesulfonyl (Ts-): 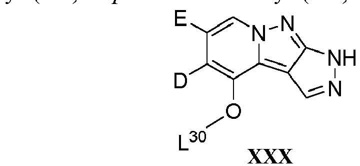
wherein D and E are independently defined as described above;
L³⁰ is the following group unsubstituted or substituted with 1, 2, 3, 4, 5 or 6 halogens: C₁₋₆ alkylsulfonyl or C₁₋₆ alkylbenzenesulfonyl, such as methanesulfonyl (Ms-), trifluoromethanesulfonyl (Tf-) or *p*-toluenesulfonyl (Ts-).

35. A preparation method, comprising subjecting compound 3a to the following reaction to prepare compound 4, compound 5, compound 6, compound 7, compound 8, and/or compound 9:

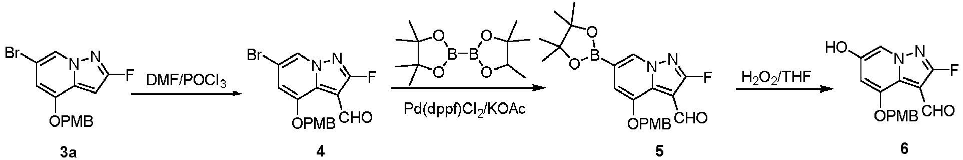

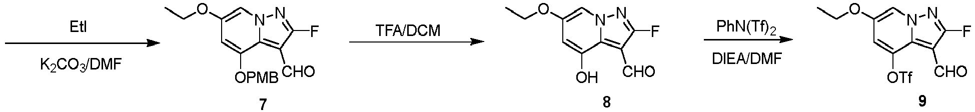

36. A preparation method, comprising subjecting compound 3a to the following reaction to prepare compound 4, compound 23, compound 31, compound 29, and/or compound 30:

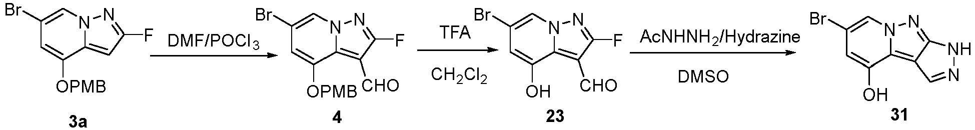

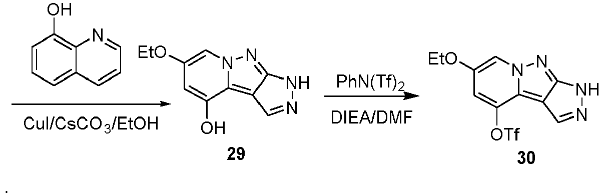

37. A preparation method, comprising subjecting compound 4 to the following reaction to prepare compound 23, compound 31, compound 25, compound 26, compound 27, compound 28, compound 29, and/or compound 30:

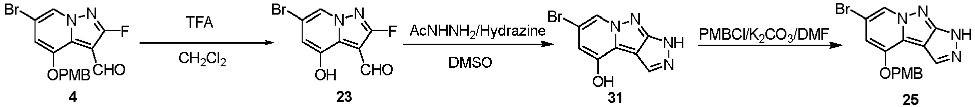

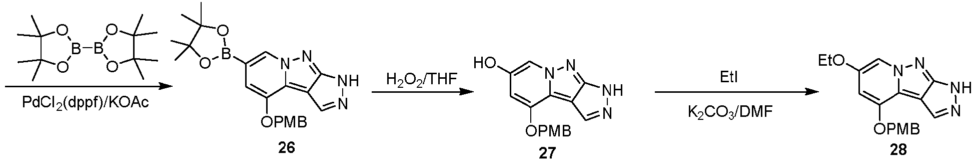

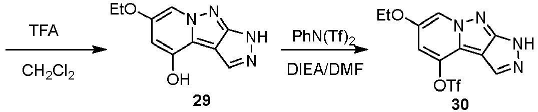

38. A preparation method M19B for the compound of formula XIX, comprising reacting the compound of formula XXX defined as in claim 34 with the compound of formula XVIII defined as in claim 8 or 9 to give the compound of formula XIX, wherein,
the compound of formula XVIII is defined as in claim 34, and the compound of formula XIX is defined as in claim 6 or 7;
preferably, the reaction may be carried out in the presence of a catalyst, wherein the catalyst may be a palladium catalyst, such as Pd(dba)₂, PdCl₂, Pd(OAc)₂, Pd(dppf)Cl₂, Pd₂(dba)₃, PdCl₂(CH₃CN)₂, Pd(PPh₃)₄, PdCl₂(PPh₃)₂, Pd(t-Bu)₃, PdCl₂(PPh₃)₂•CH₂Cl₂, Pd(OAc)/PPh₃, PdCl₂[(Pet₃)]₂, Pd(DIPHOS)₂, PdCl₂(Bipy), [PdCl(Ph₂PCH₂PPh₂)]₂, PdCl₂[P(o-Tol)₃]₂, Pd₂(dba)₃/P(o-Tol)₃, Pd₂(dba)/P(furanyl)₃, PdCl₂[P(furanyl)₃]₂, PdCl₂(PMePh₂)₂, PdCl₂[P(4-F-Ph)₃]₂, PdCl₂[P(C₆F₆)₃]₂, PdCl₂[P(2-COOH-Ph)(Ph)₂]₂, and PdCl₂[P(4-COOH-Ph)(Ph)₂]₂;
preferably, the reaction may be carried out at a temperature of not less than 80 °C, for example, not less than 100 °C, such as 120 °C;
preferably, the molar ratio of the compound of formula XXX to the compound of formula XVIII may be 1:1 to 1:5, for example, 1:2 to 1:4;
preferably, the molar ratio of the compound of formula XXX to a base may be 1:1 to 1:10, for example, 1:4 to 1:8, such as 1:5 to 1:6;
preferably, the molar ratio of the compound of formula XXX to the catalyst may be 1:0.01 to 1:0.2, such as 1:0.05 to 1:0.1.

39. A preparation method M1B for the compound of formula I, comprising reacting the compound of formula XXX defined as in claim 34 with a compound of formula XXXX to give the compound of formula I defined as in any one of claims 1 to 5: 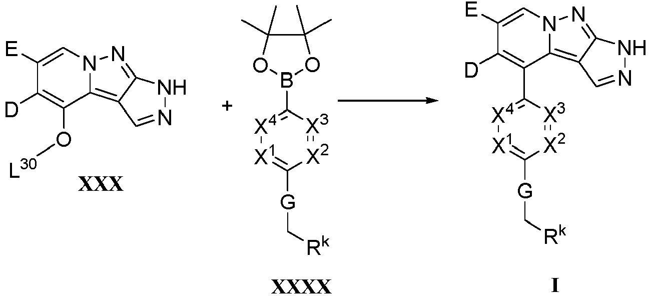
wherein, X¹, X², X³, X⁴, G, and R^{k} in the compound of formula XXXX are each independently defined as in any one of claims 1 to 5;
preferably, the reaction may be carried out in the presence of a catalyst, wherein the catalyst may be a palladium catalyst, such as Pd(dba)₂, PdCl₂, Pd(OAc)₂, Pd(dppf)Cl₂, Pd₂(dba)₃, PdCl₂(CH₃CN)₂, Pd(PPh₃)₄, PdCl₂(PPh₃)₂, Pd(t-Bu)₃, PdCl₂(PPh₃)₂•CH₂Cl₂, Pd(OAc)/PPh₃, PdCl₂[(Pet₃)]₂, Pd(DIPHOS)₂, PdCl₂(Bipy), [PdCl(Ph₂PCH₂PPh₂)]₂, PdCl₂[P(o-Tol)₃]₂, Pd₂(dba)₃/P(o-Tol)₃, Pd₂(dba)/P(furanyl)₃, PdCl₂[P(furanyl)₃]₂, PdCl₂(PMePh₂)₂, PdCl₂[P(4-F-Ph)₃]₂, PdCl₂[P(C₆F₆)₃]₂, PdCl₂[P(2-COOH-Ph)(Ph)₂]₂, and PdCl₂[P(4-COOH-Ph)(Ph)₂]₂.

40. The preparation method M1B as claimed in claim 39, comprising subjecting compound 30 and compound 40 to the following reaction to prepare compound 1:

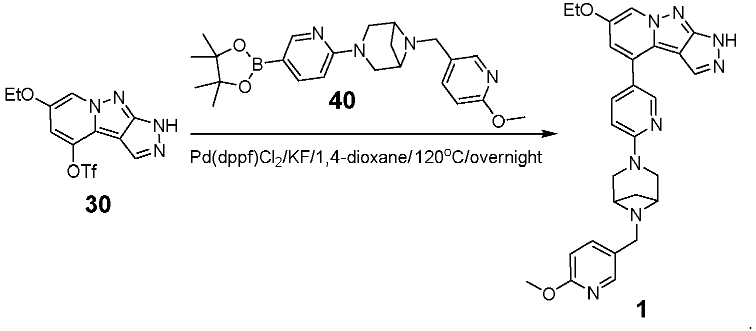

41. The preparation method as claimed in any one of claims 1 to 40, wherein,
the reaction may be carried out in the presence of a base, wherein the base may be an organic base or an inorganic base, for example,
an organic base selected from the following: tertiary amines, substituted or unsubstituted pyridines, and substituted or unsubstituted triethylamine, trimethylamine, *N*,*N-*diisopropylethylamine, tri-*n*-propylamine, tri-*n*-butylamine, tri-*n*-hexylamine, tricyclohexylamine, *N*-methylcyclohexylamine, *N-*methylpyrrolidine, *N-*methylpyrrolidone, *N*-methylpiperidine, *N*-ethylpiperidine, *N*,*N*-dimethylaniline, *N*-methylmorpholine, pyridine, 2,3-or 4-methylpyridine, 2-methyl-5-ethylpyridine, 2,6-dimethylpyridine, 2,4,6-trimethylpyridine, 4-dimethylaminopyridine, quinoline, methylquinoline, *N*,*N*,*N*,*N-*tetramethylethylenediamine, *N*,*N*-dimethyl-1,4-diazacyclohexane, *NN-*diethyl-1,4-diazacyclohexane, 1,8-bis(dimethylamino)naphthalene, diazabicyclooctane (DABCO), diazabicyclononane (DBN), diazabicycloundecane (DBU), butylimidazole, methylimidazole, sodium *tert*-butoxide and potassium *tert*-butoxide; or
an inorganic base selected from the following: hydrides, hydroxides, amides, alkoxides, acetates, fluorides, phosphates, carbonates and bicarbonates of alkali metals or alkaline earth metals, such as sodium amide, sodium hydride, lithium diisopropylamide, sodium methoxide, potassium tert-butoxide, sodium hydroxide, potassium hydroxide, sodium acetate, sodium phosphate, potassium phosphate, potassium fluoride, cesium fluoride, sodium carbonate, potassium carbonate, potassium bicarbonate, sodium bicarbonate, and cesium carbonate;
the reaction may be carried out in the presence of an organic solvent or a mixed solvent of an organic solvent and water, wherein the organic solvent may be selected from one or a mixture of two or more of the following: ethers (e.g., ethyl propyl ether, methyl *tert*-butyl ether, *n*-butyl ether, anisole, phenetole, cyclohexyl methyl ether, dimethyl ether, diethyl ether, diphenyl ether, dipropyl ether, diisopropyl ether, di-*n*-butyl ether, diisobutyl ether, diisoamyl ether, ethylene glycol dimethyl ether, isopropyl ethyl ether, diethylene glycol dimethyl ether, triethylene glycol dimethyl ether, tetrahydrofuran, 2-methyltetrahydrofuran, 1,4-dioxane and ethylene oxide, and/or propylene oxide polyethers); sulfoxides (e.g., tetrahydrodioxythiophene and dimethyl sulfoxide, tetramethyl sulfoxide, dipropyl sulfoxide, benzyl methyl sulfoxide, diisobutyl sulfoxide, dibutyl sulfoxide, or diisoamyl sulfoxide); sulfones (e.g., dimethyl sulfone, diethyl sulfone, dipropyl sulfone, dibutyl sulfone, diphenyl sulfone, dihexyl sulfone, methyl ethyl sulfone, ethyl propyl sulfone, ethyl isobutyl sulfone, and cyclopentyl sulfone); aliphatic, cycloaliphatic or aromatic hydrocarbons (e.g., pentane, hexane, heptane, octane, nonane, cyclohexane, methylcyclohexane, petroleum ether, naphtha, octane, benzene, toluene, or xylene); haloalkanes (e.g., dichloromethane, chloroform, carbon tetrachloride, dichloroethane, or trichloroethane); halogenated aromatic compounds (e.g., chlorobenzene or dichlorobenzene); amides (e.g., hexamethylphosphoramide, formamide, *N*,*N*-dimethylacetamide, *N*-methylformamide, *N*,*N-*dimethylformamide, *N*,*N-*dipropylformamide, *N*,*N-*dibutylformamide, *N-*methylpyrrolidine, *N*-methylcaprolactam, 1,3-dimethyl-3,4,5, 6-tetrahydro-2(1*H*)-pyrimidine, octylpyrrolidine, octylcaprolactam, 1,3 -dimethyl-2-imidazolinedione, *N*-formylpiperidine, or *N*,*N'*-1,4-diformylpiperazine); nitriles (e.g., acetonitrile, propionitrile, *n*-butyronitrile, isobutyronitrile, or benzonitrile); alcohols (e.g., methanol, ethanol, *n*-propanol, isopropanol, *n*-butanol, *sec*-butanol, or *tert*-butanol); and ketones (e.g., acetone or *N-*methylpyrrolidone); preferably, where an organic solvent or a mixed solvent of an organic solvent and water is used, the organic solvent or the mixed solvent is inert to the reaction substrate and the reagent.

42. A preparation method selected from one of the following preparation methods:
a preparation method M-IIIB-I, comprising the preparation methods M13, M10A, M11, M19A, M20 and M1A described above, or being selected from a combination of two, three or more of the preparation methods described above;
a preparation method M-IIIA-X1, comprising the preparation methods M14, M15, M16, M17 and M10B described above, or being selected from a combination of two, three or more of the preparation methods described above;
a preparation method M-IIIA-X2, comprising preparing the compound of formula IV, the compound of formula V, the compound of formula VI, the compound of formula VII, the compound of formula VIII, and the compound of formula IX by the preparation methods described above, and preparing the compound of formula X by the preparation method M10C; and
a preparation method M-IIIA-XXX, comprising preparing the compound of formula XXIII, the compound of formula XXXI, the compound of formula XXIX and the compound of formula XXX by the methods described above,
wherein the preparation methods are defined as in claims 1 to 41;
preferably, the preparation methods in the combination are carried out sequentially, so that a product resulting from the preparation method carried out previously is used as a reaction substrate for the preparation method carried out subsequently.

43. The preparation method as claimed in any one of claims 1 to 42, wherein the starting material, reaction product or intermediate is not separated or purified using chromatography such as column chromatography; and/or
the reaction is carried out without using hydrogen as a hydrogenation or reduction reagent, and without using hydrogen by means of catalytic hydrogenation.

44. A compound selected from one, two or more of the following compounds:
compound IV, compound VIII, compound IX, compound X, compound XI, compound XII, compound XIII, compound XIV, compound XV, compound XVI, compound XVII, compound XIX, compound XX, compound XXIII, compound XXIX, compound XXX and compound XXXI; and
compound 4, compound 8, compound 9, compound 10, compound 11, compound 12, compound 13, compound 14, compound 15, compound 16, compound 17, compound 19, compound 20, compound 23, compound 29, compound 30 and compound 31;
wherein the compounds described above are defined as in any one of claims 1 to 40.

45. Use of one, two or more of the compounds as claimed in claim 44 in the preparation of the compound of formula I, wherein the compound of formula I is defined as in any one of claims 1 to 5.
